# EUROPEAN PATENT APPLICATION

(11) **EP 4 063 484 A1**
(43) Date of publication of application: **28.09.2022**
(21) Application number: 20904625.9
(22) Date of filing: 09.12.2020
(51) Int. Cl.: C12N 1/15, C12N 15/80

(54) **RECOMBINANT FILAMENTOUS FUNGUS FOR PRODUCING ETHANOL, AND CONSTRUCTION AND APPLICATION THEREOF**

(30) Priority: 24.12.2019 CN 201911349044; 27.08.2020 CN 202010874879; 17.11.2020 CN 202011283377
(71) Applicant: Tianjin Institute of Industrial Biotechnology, Chinese Academy of Sciences, Tianjin 300308 (CN)
(72) Inventor: TIAN, Chaoguang, Tianjin 300308 (CN); ZHANG, Yongli, Tianjin 300308 (CN); LI, Jingen, Tianjin 300308 (CN); LI, Jinyang, Tianjin 300308 (CN); SUN, Tao, Tianjin 300308 (CN); LIU, Qian, Tianjin 300308 (CN); SUN, Wenliang, Tianjin 300308 (CN)
(74) Representative: Ipside
(86) International application number: PCT/CN2020/134922
(87) International publication number: WO 2021/129396

(57) **Abstract**

The invention discloses a construction method of genetic engineering fungi of filamentous fungi. Through the genetic engineering method, the filamentous fungi overexpress the positive regulation genes of ethanol synthesis, and / or down regulate the negative regulation genes of endogenous ethanol synthesis to obtain genetic engineering strains.Or overexpression of acetaldehyde dehydrogenase and ethanol dehydrogenase containing mitochondrial localization signal sequence, or overexpression of pyruvate decarboxylase and ethanol dehydrogenase containing mitochondrial localization signal sequence, or overexpression of acetaldehyde dehydrogenase, ethanol dehydrogenase and pyruvate decarboxylase containing mitochondrial localization signal sequence in filamentous fungal cells. Compared with the original strain, the ethanol synthesis ability of the obtained genetically engineered strainsare improved.

## Description

### Technical Field

The invention belongs to the field of genetic engineering and biotechnology. Specifically, the invention relates to a method for constructing a recombinant engineering strain for producing ethanol and the obtained recombinant engineering strain. The invention also relates to a method for producing ethanol by the recombinant engineering fungus.

### Background of the Invention

With the rapid development of the world economy, human beings demand for energy is increasing. On the one hand, the reserves of fossil energy such as petroleum are limited, and a lot of pollution will be produced in the combustion process. Energy and environmental issues have become a major challenge for human development. Transportation industry is the main sector of energy consumption and an important way of greenhouse gas emission, so there is an urgent need to develop clean and renewable alternative fuels. Among them, fuel ethanol has the characteristics of rich raw materials, clean and renewable and good integration with the existing fuel system, and has become the most important alternative energy at present (Zabed, H., Sahu, J.N., Suely, A., Boyce, A.N. and Faruq, G. (2017) Bioethanol production from renewable sources: current perspectives and technological progress. Renewable and Sustainable Energy Reviews, 71, 475-501). In the production of fuel ethanol, the raw materials used in the production of the first generation of fuel ethanol are mainly sugarcane and corn. With the rapid growth of fuel ethanol consumption, the disadvantages of the first generation of fuel ethanol "competing with people for food and land" are becoming increasingly prominent. The second generation fuel ethanol with non grain lignocellulose as raw materials can solve this problem well, and is in line with the strategic policy of sustainable development of China's energy economy. China is rich in lignocellulose resources, and the total output of crop straw produced each year is about 900 million tons (Tian F, Li F, Yuan J, Xu K, Wang K, Wang C, Shu M, Li Y, Tong Y, Cui Z. Current situation of cellulose ethanol industry and technical difficulties in key processes [J]. Modern Chemical Industry, 2019,48 (09): 2051-2056). Most of them are treated by incineration or rough return to the field, which not only causes a huge waste of resources, but also causes serious environmental pollution. Making full use of this huge amount of biomass resources to produce fuel ethanol will provide an important guarantee for China's energy security, optimize the energy structure, improve the ecological environment and promote the economic development of rural areas.

At present, the main fermentation microorganisms used in the production of fuel ethanol are *S. cerevisiae* and *Zymomonas mobilis* (Sarris, D. and Papanikolaou, S. (2016) Biotechnological production of ethanol: Biochemistry, processes and technologies. Engineering in Life Sciences, 16, 307-329), which showed excellent production performance in the first generation of grain ethanol production. However, in the production of the second generation fuel ethanol, *S. cerevisiae* and *Zymomonas mobilis* can not meet the production requirements due to the lack of cellulase secretion and pentose metabolism (especially xylose and arabinose). The main components of lignocellulose are lignin, cellulose and hemicellulose. Cellulose is D-glucose β- 1,4 glycosidic bond linked chain polymer compound; hemicellulose is a heterogeneous polymer composed of several different types of monosaccharides, including pentoses such as xylose, arabinose and hexoses such as mannose and galactose. The saccharification products of lignocellulose are mainly about 70% cellobiose and glucose and about 30% xylose (Carroll A, Somerville C. Cellulosic biofuels. Annu Rev Plant Biol. 2009; 60:165-82). In order to improve the fermentation efficiency of xylose, scientists have made many efforts. However, the productivity and yield of ethanol produced by xylose fermentation of *S. cerevisiae* and *Zymomonas mobilis* engineering strains are still lower compared with that of glucose fermentation (Sharma NK, Behera S, Arora R, Kumar S, Sani RK. Xylose transport in yeast for lignocellulosic ethanol production: Current status. J Biosci Bioeng. 2018; 125 (3): 259-67). In addition, due to the existence of glucose inhibition effect, natural *S. cerevisiae* can not use xylose in the presence of glucose, which also hinders the effective utilization of mixed sugar in cellulose hydrolysates.

Cellulase from fungi can hydrolyze cellulose to produce cellobiose, while glucosidase can further hydrolyze cellobiose to glucose. Therefore, the production of biofuels from biomass requires microorganisms that can effectively use cellobiose and glucose. Because glucose inhibits the activity of cellulase and the utilization of other sugars in the fermentation process, the direct utilization of cellobiose by microorganisms is a better method. The degradation of cellobiose to glucose occurs in the body, so it can effectively reduce the inhibitory effect of carbon metabolism in the process of mixed sugar fermentation. However, natural *S. cerevisiae* cannot use disaccharide fermentation. Only the introduction of exogenous cellobiose utilization pathway can make use of cellobiose, which mainly includes cellobiose transporters from *Neurospora* for cellobiose induction (Kim H, Lee WH, Galazka JM, Cate JH, Jin YS. Analysis of cellodextrin transporters from M. thermophila in S. cerevisiae for cellobiose fermentation. Appl Microbiol Biotechnol. 2014;98(3):1087-94) and hydrolysis system or cellobiose phosphorylation system replaced by marine bacterium *Saccharophagus degradans* (Sadie CJ, Rose SH, den Haan R, van Zyl WH. Co-expression of a cellobiose phosphorylase and lactose permease enables intracellular cellobiose utilisation by S. cerevisiae. Appl Microbiol Biotechnol. 2011;90(4):1373-80). Although *S. cerevisiae* has been further modified, its ability to use cellobiose is still greatly limited.

Cellulose degrading thermophilic fungi are excellent producers of high temperature resistant cellulases. They naturally have the ability to secrete a large number of biomass degrading enzymes, and can make full use of a variety of sugars such as cellobiose, glucose and xylose produced by degradation (Karnaouri A, Topakas E, Antonopoulou I, Christakopoulos P. Genomic insights into the fungal lignocellulolytic system of M. thermophila. Front Microbiol. 2014; 5:281). At the same time, the optimum growth temperature of these microorganisms is high, which can save the amount of cooling water in the fermentation process and reduce the production cost. It has been proved that it can be used for large-scale industrial fermentation (Visser H, Joosten V, Punt PJ, Gusakov AV, Olson PT, Joosten R, Bartels J, Visser J. Development of a mature fungal technology and production platform for industrial enzymes based on a M. thermophila isolate, previously known as Chrysosporium lucknowense C1. Ind Biotechnol. 2011; 7:214-23). However, the ethanol production of wild-type filamentous fungi (including *M. thermophila*) is low, so they can not be directly used in the actual production of ethanol. Therefore, genetic transformation of cellulose degrading fungi to realize the direct fermentation of biomass raw materials to ethanol is an urgent problem to be overcome in the production of second-generation fuel ethanol, and it also has great application potential.

In order to realize the one-step fermentation of filamentous fungi from cellulose to ethanol, the characteristics of low ethanol yield and slow metabolic rate of filamentous fungi must be overcome. The level of ethanol production and the speed of metabolic rate largely depend on the strength of glycolysis pathway, and the most important regulatory enzyme in glycolysis pathway is phosphofructokinase. Therefore, regulating the activity of phosphofructokinase is one of the keys to accelerate the glycolysis rate of filamentous fungi and improve ethanol production. At the same time, in order to realize the efficient production of cellulosic ethanol by filamentous fungi, it is necessary to improve the ethanol synthesis capacity of filamentous fungi. However, unlike *Saccharomyces cerevisiae,* which can anaerobic ferment ethanol, most filamentous fungi are aerobic fungi, and a large amount of carbon sources will enter mitochondria and eventually convert into CO₂ through tricarboxylic acid cycle and respiration, which makes it difficult to greatly improve the yield of ethanol. Therefore, if we want to use filamentous fungi to synthesize ethanol efficiently with cellulose as substrates in one step, we must overcome the above bottleneck.

### Summary of the Invention

At present, the fermentation production of ethanol mainly takes grain crops such as corn and sugarcane and non grain crops such as cassava as raw materials. Cellulose degrading filamentous fungi represented by *M*. *thermophila* can directly use lignocellulose to produce ethanol, which greatly reduces the pretreatment cost and simplifies the production process. However, the ethanol production of wild-type filamentous fungi (including *M. thermophila*) is low, which can not meet the production demand. Moreover, the genetic operation of filamentous fungi is complex, inefficient and long cycle, which also restricts the process of using filamentous fungi for ethanol production through genetic operation. In recent years, the inventors have successively established the genetic operating system of *Rhizopus thermophila* (Xu, J., Li, J., Lin, L., Liu, Q., Sun, W., Huang, B. and Tian, C. (2015) Development of genetic tools for M. thermophila. BMC Biotechnol, 15, 35) and gene editing system (Liu, Q., Gao, R., Li, J., Lin, L., Zhao, J., Sun, W. and Tian, C. (2017) Development of a genome-editing CRISPR/Cas9 system in thermophilic fungal M. species and its application to hyper-cellulase production strain engineering. Biotechnol Biofuels, 10, 1), greatly accelerated the genetic transformation process of *M. thermophila.* On the basis of the above, the inventors also systematically studied the mechanism of ethanol production by filamentous fungi (especially *M. thermophila*), and significantly improved the efficiency of ethanol production by *M*. *thermophila* (the carbon sources used include glucose, xylose, fructose, cellobiose, cellulose and xylan), providing a new strategy for the production of second-generation fuel ethanol. The strategies of genetic transformation include: overexpression of glucose and cellobiose transporter to improve the absorption rate of cellulose degradation products by *M*. *thermophila*; overexpression of key genes in ethanol synthesis pathway to improve the efficiency of ethanol synthesis; knock out the main branching pathways; increase the level of cytoplasmic NADH; accelerate the rate of glycolysis; knock out the endogenous ethanol consumption pathway; and overexpression of ethanol synthesis gene containing mitochondrial localization signal sequence for ethanol synthesis.

The invention first provides a construction method of filamentous fungi recombinant engineering fungi, which is characterized in that the key genes of ethanol synthesis pathway are overexpressed in the filamentous fungi, and / or the expression of endogenous ethanol synthesis competitive pathway genes is down regulated, and / or the ethanol synthesis gene containing mitochondrial localization signal sequence is overexpressed in the filamentous fungi for ethanol synthesis through genetic engineering, compared with the original strain, the ethanol synthesis ability of the genetically engineered strain is improved.

Preferably, the filamentous fungi are cellulose degrading filamentous fungi. Specifically, the filamentous fungi are selected from *Neurospora, Aspergillus, Trichoderma, Penicillium, Myceliophthora, Sporotrichum, Fusarium, Rhizopus, Mucor* and *Paecilomyces.*

In a more preferred manner, the *Myceliophthora* is selected from the group consisting of *M. thermophila* and *M. heterothalica.*

On the other hand, the positive regulation gene of ethanol synthesis is selected from the genes that strengthen the pathway of ethanol synthesis, improve the ability of sugar transport, accelerate the rate of glycolysis and improve the level of cytoplasmic NADH; the negative regulation gene of ethanol synthesis is selected from the genes in the branch pathway of ethanol synthesis, energy metabolism, the shuttle of cytoplasmic reducing force to mitochondria and the endogenous ethanol metabolism pathway.

In some examples, the shuttle of cytoplasmic reducing force to mitochondria is reduced or blocked, and / or the energy metabolism pathway is modified, and / or the ethanol synthesis pathway is strengthened, and / or the sugar molecule transport is strengthened, and / or the ethanol synthesis by-product pathway is weakened, and / or the glycolysis rate is accelerated.

In another aspect, the overexpression of the positive regulation gene of ethanol synthesis is realized by introducing exogenous and / or endogenous positive regulation genes of ethanol synthesis, wherein the positive regulation gene of ethanol synthesis is selected from one of ethanol dehydrogenase, glucose transporter, cellobiose transporter, or pyruvate decarboxylase, or a variety of combinations thereof. More specifically, overexpression of exogenous ethanol dehydrogenase to improve ethanol production capacity, or overexpression of exogenous glucose transporter to improve ethanol production capacity, or overexpression of exogenous cellobiose transporter to improve ethanol production capacity, and overexpression of exogenous pyruvate decarboxylase to improve ethanol production capacity.

Preferably, the introduction is to transfer the expression vector carrying the positive regulation genes of exogenous or endogenous ethanol synthesis into the host cell, and the preferred promoters are *tef, gpdA, trpC, cbh1* and *glaA* promoters.

More preferably, the introduced positive regulatory gene of exogenous ethanol synthesis comes from yeast, preferably from *S. cerevisiae* and *Zymomonas mobilis.*

In some examples, the ethanol dehydrogenase coding gene *Scadh1* and / or pyruvate decarboxylase gene *Scpdc1* and / or glucose transporter coding gene *glt-1* and / or cellobiose transporter coding gene *cdt-1* / *cdt-2* are overexpressed in the filamentous fungi.

In some specific examples, the overexpression of the positive regulation gene of ethanol synthesis in the filamentous fungus is selected from exogenous ethanol dehydrogenase, and the preferred ethanol dehydrogenase is ethanol dehydrogenase *Scadh1,* and / or ZmADHI from *Zymomonas mobilis,* the preferred pyruvate decarboxylases are ScPDC1 and ScPDC5 from *Saccharomyces cerevisiae,* and / or ZmPDC from *Zymomonas mobilis.*

In another example, the down-regulated gene expression is to inactivate the related genes or reduce the expression or activity by gene knockout or small RNA interfering or changing the promoter or gene mutation. Preferably, the gene editing is a genome editing method based on CRISPR / Cas9.

In some specific examples, the endogenous ethanol synthesis negative regulation gene is selected from the genes in the branch pathway of ethanol synthesis: *ldh1, Idh2, mpd*; the genes in the shuttle pathway of cytoplasmic reducing power to mitochondria: *mdh, gpd, nde1, nde2;* the genes in the electron transport chain (respiratory chain): *cox*; and the genes of ethanol metabolism: *Mtadh*, *Mtaldh.* More specifically, it is selected from one of lactate dehydrogenase, mannitol-1-phosphate dehydrogenase, cytoplasmic malate dehydrogenase, glycerol-3-phosphate dehydrogenase, cytochrome C oxidase, outer NADH dehydrogenase, ethanol dehydrogenase, acetaldehyde dehydrogenase, or a variety of combinations thereof. More specifically, the expression of lactate dehydrogenase gene, a negative regulator of endogenous ethanol synthesis, was decreased or lost in the strain, so as to improve the ability of ethanol production; either reduce or lose the expression of endogenous mannitol-1-phosphate dehydrogenase gene to improve the ability of ethanol production, or reduce or lose the expression of endogenous cytoplasmic malate dehydrogenase gene to improve the ability of ethanol production, or reduce or lose the expression of endogenous glycerol-3-phosphate dehydrogenase gene to improve the ability of ethanol production, or reduce or lose the expression of endogenous ethanol dehydrogenase (Mycth_55576) gene, or reduce or lose the expression of one or more combination genes in endogenous acetaldehyde dehydrogenase, or reduce the expression of endogenous cytochrome C oxidase in the strain.

In some examples, the endogenous ethanol synthesis negative regulatory gene is selected from the lactate dehydrogenase gene and / or 1-phosphate mannitol dehydrogenase.

In other examples, the endogenous ethanol synthesis negative regulatory gene is a cytochrome C oxidase coding gene and / or an external NADH dehydrogenase gene.

In some examples, the filamentous fungi overexpress the ethanol dehydrogenase of *Saccharomyces cerevisiae,* the cellobiose transporter coding gene *cdt-1* / *cdt-2* from *Neurospora crassa,* and down regulate the expression of endogenous lactate dehydrogenase genes *Idh-1* and / or *Idh-2.*

In some examples, the ethanol dehydrogenase of *Saccharomyces cerevisiae* is overexpressed in the filamentous fungus and the expression of the outer NADH dehydrogenase gene *nde* is down regulated, wherein one or both of the outer NADH dehydrogenase genes *nde1*and *nde2* are down regulated.

In some examples, ethanol dehydrogenase of *Saccharomyces cerevisiae* is overexpressed in the filamentous fungus and the expression of cytochrome C oxidase gene is down regulated.

In some examples, ethanol dehydrogenase of *Saccharomyces cerevisiae* is overexpressed in the filamentous fungus, the expression of cytoplasmic malate dehydrogenase gene *mdh* is down regulated, and the expression of glycerol-3-phosphate dehydrogenase gene *gpd* is optionally further down regulated.

In some specific examples, the ethanol dehydrogenase gene *Scadh1* and pyruvate decarboxylase gene *Scpdc1* of *Saccharomyces cerevisiae* are overexpressed in the filamentous fungus, optionally further overexpressing the glucose transporter coding gene *glt-1,* and further regulating the expression of lactate dehydrogenase gene and mannitol 1-phosphate dehydrogenase gene. Or further down regulate the cytoplasmic malate dehydrogenase gene *mdh,* and / or down regulate the ethanol dehydrogenase gene *Mtadh* and / or acetaldehyde dehydrogenase gene *Mtaldh.*

In some specific examples, the ethanol dehydrogenase gene *Scadh1* and pyruvate decarboxylase gene *Scpdc1* of *Saccharomyces cerevisiae* are overexpressed in the filamentous fungi, optionally further overexpressing the glucose transporter coding gene *glt-1,* and further regulating the expression of endogenous lactate dehydrogenase gene *Idh1* / *Idh2* and 1-phosphate mannitol dehydrogenase gene *mpd.*

In some examples, the *Saccharomyces cerevisiae* ethanol dehydrogenase gene *Scadh1* and the pyruvate decarboxylase gene *Scpdc1* are overexpressed in the filamentous fungus.

In another examples, the endogenous phosphofructokinase 2 gene is knocked out in the filamentous fungus, and the mutant phosphofructokinase 2 gene is preferably further expressed, wherein the mutant phosphofructokinase 2 refers to the retention of kinase activity and the loss or reduction of phosphokinase activity after mutation; the phosphofructose kinase 2 refers to the gene encoding the synthesis and degradation of fructose-2,6-diphosphate or its homologous gene; the mutated phosphofructose kinase 2 refers to gene ID (herein, gene ID refers to NCBI https://www.ncbi.nlm.nih.gov/ gene number; the same below): 11510101 is a mutant corresponding to one or two or three sites in H233, E306 and H371 in the amino acid sequence to reduce or lose phosphatase activity. In this examples, in order to promote the glucose metabolism rate and improve the ethanol yield, the invention mutates the phosphofructokinase 2 gene to obtain a mutant gene that can promote the glucose metabolism rate and improve the ethanol yield. At the same time, in order to further verify whether knockout of phosphofructokinase 2 and overexpression of mutant phosphofructokinase 2 can also promote glucose metabolism rate and improve ethanol production, the invention has carried out experimental verification on phosphofructokinase 2 knockout strain and strain overexpressing the gene mutant.

In other examples, overexpression of ethanol synthesis gene containing mitochondrial localization signal sequence in the filamentous fungus is used to synthesize ethanol by overexpression of acetaldehyde dehydrogenase and ethanol dehydrogenase genes containing mitochondrial localization signal sequence, or overexpression of pyruvate decarboxylase and ethanol dehydrogenase genes containing mitochondrial localization signal sequence, or overexpression of acetaldehyde dehydrogenase, ethanol dehydrogenase and pyruvate decarboxylase genes containing mitochondrial localization signal sequence. Preferably, the pyruvate decarboxylase and ethanol dehydrogenase are derived from *Saccharomyces cerevisiae* or *Zymomonas mobilis*; the acetaldehyde dehydrogenase comes from *Thermoanaerobacterium saccharolyticum.* In a preferred examples, the mitochondrial localization signal sequence refers to the N-terminal of the amino acid sequence used to guide the transmembrane transfer of protein to mitochondria. Preferably, the localization signal sequence derived from the mitochondrial matrix protein of *M*. *thermophila* is selected. In these examples, the invention hopes to use the abundant pyruvate, acetyl CoA and NADH in mitochondria to directly synthesize ethanol in mitochondria. The verification shows that the above strategy overcomes the defect that filamentous fungi make a large number of carbon sources enter mitochondria due to aerobic respiration, and finally convert into carbon dioxide after passing through the tricarboxylic acid cycle, which makes it difficult to improve ethanol production, and realizes the effective improvement of ethanol production.

The invention also provides a genetically engineered fungus obtained by the construction method as described above.

In the examples of the invention, compared with the original strain, the ethanol production capacity is enhanced or increased by at least 10%; preferably at least 20%, 30%, 40%, 50%.

The invention further provides the use of the recombinant fungus with improved ethanol production capacity obtained by the above construction method in the production of ethanol.

Among them, it uses monosaccharide or / and glycan, or substances containing monosaccharide or / and glycan as the substrates.

Preferably, the monosaccharide is glucose, xylose, arabinose or a combination thereof; the glycan includes cellobiose, xylobiose, sucrose, maltose, xylooligosaccharide, cellooligosaccharide, cellulose, crystalline cellulose, hemicellulose, starch, plant woody biomass or a combination thereof. More preferably, the plant woody biomass is selected from crop straw, forestry waste, energy plants or some or all of their decomposition products. Further preferably, the crop straw is selected from corn straw, wheat straw, rice straw, sorghum straw, soybean straw, cotton straw, bagasse and corncob; the forestry waste is selected from branches and leaves and sawdust; the energy plant is selected from sweet sorghum, switchgrass, miscanthus, reed or a combination thereof.

In some examples, the filamentous fungi are selected from *Neurospora, Aspergillus, Trichoderma, Penicillium, Myceliophthora, Sporotrichum, Fusarium, Rhizopus, Mucor* and *Paecilomyces.* Preferably, the *Myceliophthora* is selected from the group consisting of *Myceliophthora thermophila* and *Myceliophthora heterothalica.* More preferably, *Myceliophthora thermophila.*

In the preferred mode, the recombinant strain is *Myceliophthora thermophila,* and the fermentation temperature is 40-60 °C, preferably 45-52 °C, more preferably 48-50 °C.

The invention also provides a method for producing ethanol. In the culture medium containing monosaccharide or / and glycan, the genetic engineering fungi obtained by the above construction method are cultured to collect ethanol from the culture.

Preferably, the monosaccharide is glucose, xylose, arabinose or a combination thereof; the glycan includes cellobiose, xylobiose, sucrose, maltose, xylooligosaccharide, cellooligosaccharide, cellulose, crystalline cellulose, hemicellulose, starch, plant biomass or a combination thereof. Further, the plant biomass is selected from crop straw, forestry waste, energy plants or some or all of their decomposition products, more preferably, the crop straw is selected from corn straw, wheat straw, rice straw, sorghum straw, soybean straw, cotton straw, bagasse and corncob. The forestry waste is selected from branches and leaves and sawdust; the energy plant is selected from sweet sorghum, switchgrass, miscanthus, reed or a combination thereof.

In some examples, the filamentous fungi are selected from *Neurospora, Aspergillus, Trichoderma, Penicillium, Myceliophthora, Sporotrichum, Fusarium, Rhizopus, Mucor* and *Paecilomyces.* Preferably, the Myceliophthora is selected from *M. thermophila* and *M*. *heterothalica*; more preferably, *M. thermophila.* Preferably, the filamentous fungi are *Myceliophthora* or *Trichoderma.* More preferably, the Myceliophthora is selected from *M*. *thermophila* and *M. heterothalica.* The most preferred *Myceliophthora* fungus is selected from *M. thermophila,* and more preferably, the fermentation temperature is 40-60 °C, preferably 45-52 °C, more preferably 48-50 °C. The specific steps include culturing the recombinant fungi in a substrate; collect the culture medium and extract ethanol.

For the first time, the invention carries out genetic transformation on filamentous fungi, especially the strains of *Myceliophthora,* so as to enhance the ethanol production capacity of the strains. In particular, it has the ability to effectively use the fermentation substrates such as glycans or plant biomass that can not be used by ordinary strains to synthesize ethanol. In particular, using *M. thermophila,* through genetic transformation, ethanol can be synthesized in high yield under high temperature that ordinary strains cannot tolerate. The use of *M*. *thermophila* can realize the one-step conversion of biomass raw materials to ethanol and reduce the production cost of cellulosic ethanol. The invention verifies the above effect through experiments and has outstanding application potential and wide practical application prospects.

### Brief description of the Drawings

Fig. 1 shows the ethanol yield of wild-type strain, engineering strain E1 under the conditions of glucose and cellulose;
Fig. 2 shows the ethanol yield of engineering fungi E1 and E2 under the conditions of glucose and cellulose;
Fig. 3 shows the ethanol yield of engineering fungi E2 and E3 under the conditions of glucose and cellulose;
Fig. 4 shows the ethanol yield of wild-type strains, engineering strains JY144 and JY518 under the conditions of cellobiose and cellulose;
Fig. 5 shows the ethanol yield of engineering fungi E3 and E4 under the conditions of glucose and cellulose;
Fig. 6 shows the ethanol yield of engineering fungi E3 and E5 under the conditions of glucose and cellulose;
Fig. 7 shows the ethanol yield of engineering fungi E1 and E7 under the conditions of glucose and cellulose;
Fig. 8 shows the ethanol yield of engineering fungi E1 and E6 under the conditions of glucose and cellulose;
Fig. 9 shows the ethanol yield of engineering fungi E7 and E8 under the conditions of glucose and cellulose;
Fig. 10 shows the ethanol yield of engineering fungi E1, E10, E11 and E12 under the conditions of glucose and cellulose;
Fig. 11 shows the ethanol yield of engineering fungi E5, E14 and E15 under the conditions of glucose and cellulose.
Fig. 12 shows the ethanol yield of wild-type strain WT and engineering strains E17, E18 and E19 under the conditions of glucose and cellulose;
Fig. 13 shows the ethanol yield of wild-type strain WT and engineering strains E17, E18 and E19 under the conditions of cellobiose, sucrose and xylan;
Fig. 14 shows the content of residual glucose in the culture medium of wild-type strain WT and engineering strains E17, E18 and E19 after 3, 5 and 7 days of culture;
Fig. 15 shows the ethanol yield of wild-type strain, strain A1 under the conditions of glucose and cellulose;
Fig. 16 shows the ethanol yield of wild-type strain, strain A2 under the conditions of glucose and cellulose.

In the figures, Glucose represents glucose, Cellobiose represents cellobiose, Avicel represents cellulose, and WT represents the wild-type strain ATCC42464 of *M*. *thermophila.*

### Detailed examples

In order to further elaborate the technical means adopted by the invention and its effects, the technical schemes of the invention are further described below in combination with the preferred examples of the invention. It should be understood that these examples are only used to illustrate the invention and not to limit the scope of the invention. Those skilled in the art should understand that the details and forms of the technical schemes of the invention can be modified or replaced without departing from the spirit and scope of the invention, but these modifications or replacements fall within the protection scope of the invention.

Unless otherwise specified, the methods used in the following examples are conventional methods, such as the conditions described in <Molecular cloning: laboratory manual>(New York: Cold SpringHarbor Laboratory Press, 1989) written by Sambrook et al, or according to the conditions recommended by the manufacturer. The reagents or instruments used without the manufacturer indicated are conventional products that can be purchased through formal channels.

The percentage concentration in the example of the invention is mass percentage concentration unless otherwise specified.

### Example 1

### 1. Construction of Scadh1 overexpression vector (pAN52-Scadh1)

The expression vector was constructed with pAN52-TB-Intron (Environ Microbiol. 015.17(4):1444-62) as the skeleton. The PelF fragment (sequence shown in SEQ ID No.1) of the promoter of elF-5A gene (Mycoth_2297659, gene ID: 11511639) was inserted into the linearized vector pAN52-TB-Intron digested by BglII and Spe I to obtain the recombinant plasmid pAN52-PelF-TtrpC-neo. Then, the fragment of *Scadh1* (gene ID: 854068) encoding ethanol dehydrogenase from *S. cerevisiae* S288c was amplified with primers elF-ScADH1-F/R, and recombined into the linearized vector pAN52-PelF-TtrpC-neo digested by EcoRI and BamHI with Gibson assembly kit of NEB, to obtain the expression vector pAN52-PelF-Scadh1 of *Scadh1* encoding ethanol dehydrogenase regulated by constitutive strong promoter PelF.

The PCR reaction system is: 2 × Phanta ^{®} Max Buffer 25 µL, 10 mM dNTPs 1 µL, upstream / downstream primers 1.5 µL of each, template DNA 1 µL, Phanta ^{®} Max Super-Fidelity DNA Polymerase 1 µL, double distilled water 19µL.

The PCR reaction conditions were as follows: first, 95 °C for 60s; then 98 °C 15s, 58 °C 15s, 72 °C 2min, 34 cycles; finally, 72 °C for 5min and 4 °C for 10min.

The primers used for vector construction in this example are as follows:

| **SEQ ID NO.** | **Primer** | **Sequence** (**5'-3'**) |
|---|---|---|
| 3 | elF-5A-F | tctgcagatctttaattaactcgagCCACATCATGTAAACAGAG |
| 4 | elF-5A-R | ggatagacatCTTGTTGTTGTTGTTGTG |
| 5 | elF-ScADH1-F | caacaacaagATGTCTATCCCAGAAACTC |
| 6 | elF-ScADH1-R | gatttcagtaacgttaagtggatccTTATTTAGAAGTGTCAACAACG |

### 2. The expression vector (pAN52-Scadh1) was introduced into M. thermophila

*M. thermophila* ATCC 42464 was cultured in MM inclined medium [50 × Vogel's salt 20mL, sucrose 20g, agar 15g, histidine (50mg / mL) 20mL, fix the volume to 1L, autoclave. The formula of 50 × Vogel's salt (1L) is: 150g trisodium citrate (1 / 2H₂O), 250g anhydrous KH₂PO₄, 100g anhydrous NH₄NO₃, 10g MgSO₄ · 7H₂O, 5g CaCl₂ · 2H₂O, 5mL Microelement solution, 2.5mL biotin (0.1 mg / mL), and fix the volume to 1L. Microelement solution formula (100mL): 5g C₆H₈O · 7H₂O, 5g ZnSO₄ · 7H₂O, 1g Fe (NH₄)₂(SO₄) · 6H₂O, 0.25g CuSO₄ · 5H₂O, 0.05g MnSO₄ · H₂O, 0.05g H₃BO₃, 0.05g NaMoO₄·2H₂O, dissolve in water, fix the volume to 100mL] and incubate at 45 °C for 10 days.

### 2.2 Protoplast transformation of M. thermophila

1) Mycelium preparation The mature spores of *M. thermophila* were collected with sterilized water containing 0.05% Tween 80, filtered by mirror paper, coated on MM plate covered with cellophane and cultured at 37 °C for 16h.
2) Protoplast preparation Put cellophane with hyphae into 30mL lysate (formula: 0.15g lysase, aseptic operation, add 30mL solution A, filter and sterilize; formula of solution A: 1.036g potassium dihydrogen phosphate, 21.864g sorbitol, dissolve in 90mL deionized water, adjust the pH to 5.6 with potassium hydroxide, fix the volume to 100mL, high temperature sterilization), lyse at 28 °C for 2h, and shake gently every 15min.
   Then, after filtering with mirror wiping paper, centrifuge at 4 °C, 2000rpm for 10min, discard the supernatant, add 4mL solution B (0.735g calcium chloride, 18.22g sorbitol, 1mLTris · HCl (1M, pH 7.5), dissolve in 90mL deionized water, adjust the pH to 7.6 with hydrochloric acid, fix the volume to 100mL, and sterilize at high temperature), and centrifuge at 4 °C, 2000rpm for 10min; discard the supernatant and add a certain volume of solution B according to 200 µL / 5µg plasmid.
3) Protoplast transformation Precooled 15mL centrifuge tube, successively add 50 µL precooled PEG (12.5g PEG6000, 0.368g calcium chloride, 500 µL Tris · HCl (1M pH 7.5)), 10 µL plasmid pAN52-Scadh1, 200 µL protoplast linearized with BglII. Place it on ice for 20min, add 2mL precooled PEG, at room temperature for 5min, add 4mL solution B, and mix gently. Add 3mL of the above solution into 12mL of melted MM medium containing corresponding antibiotics, place it in the plate, culture at 45 °C, and after 2d-4d,pick out a single mycelium under the stereomicroscope, and culture on the corresponding resistant plate.

### 2.3 verification of transformant of M. thermophila

(1) Genome extraction Genomic DNA is extracted from the transformants selected in the above transformation process by phenol chloroform method, including the following operations:
   1) Add 200mg zirconium beads and 1mL lysis buffer (formula: 0.2MTris · HCl (pH 7.5), 0.5M NaCl, 10mM EDTA, 1% SDS (w / V)) into the 2.0mL distilled DNA extraction tube, and pick out the filaments of *M*. *thermophila* growing in the plate into the DNA extraction tube.
   2) Place all DNA extraction tubes on the grinding aid, oscillate at the maximum speed for 30s, and repeat twice.
   3) 65 °C water bath for 30 min, take out and vortex the tubes every few min during water bathing.
   4) After water bathing, take out and add 80 µL 1M Tris · HCl (pH 7.5) to each tube for neutralization.
   5) Add 400 µL phenol: chloroform (1:1), mix up and centrifuged at 13000 rpm for 5 min.
   6) Take 300 µL supernatant into a new 1.5mL EP tube and add 600 µL 95% ethanol (DNA).
   7) After incubation on ice for one hour, centrifuged at 4 °C, 13000 rpm for 10 min, get white DNA precipitated to the bottom of EP tube.
   8) Wash the precipitates with 400 µL75% ethanol (DNA grade), centrifuge at 4 °C, 13000 rpm for 10 min, and gently take out the supernatant.
   9) Place the EP tube in a vacuum concentrator and vacuum dry the ethanol.
   10) Add 50 µLddH₂Oto dissolve the DNA, and determine the DNA concentration with nanodrop. After measuring the concentration, place the extracted DNA in a refrigerator at -20°C for further PCR verification.

### 2.4 PCR validation for M. thermophila transformants

The genomic DNA was used as the templates, and elF-5A-F and elF-ScADH1-R were used as the primers for PCR validation of the transformants. The PCR products were subjected to 1% agarose gel electrophoresis (110V voltage, 30 min). The gene amplification bands were observed under the gel imaging system. It was shown that the target band of ∼2486bp was obtained by PCR amplification under the guidance of primers elF-5A-F and elF-ScADH1-R. This band showed that the pAN52-PelF-Scadh1 linearized by BglII was integrated into the genome of wild-type *M. thermophila* ATCC42464.

### 3. Determination of ethanol production capacity of M. thermophila transformants

Inoculate all the transformants verified above into 100mL of 250mL triangular flask, take glucose as carbon source medium (the medium formula is: 75 g glucose, 10 g yeast extract, 0.15 g KH₂PO₄, 0.15 g K₂HPO₄, 0.15 g MgS0₄ · 7H₂O, 0.1 g CaCl₂ · 2H₂O, 1 mL Microelement, 1 mL 0.1 mg / mL biotin solution, fix the volume to 1L, high pressure steam sterilization), cellulose (Avicel) as carbon source medium, and the formula is: 75 g Avicel, 10 g yeast extract, 0.15 g KH₂PO₄, 0.15 g K₂HPO₄, 0.15 g MgS0₄ · 7H₂O, 0.1 g CaCl2 · 2H₂O, 1 mL Microelement, 1 mL 0.1 mg / mL biotin solution, fix the volume to 1L, high pressure steam sterilization. The transformed sporozoites collected with distilled water were filtered by two layers of distilled mirror paper, and the number of spores was calculated. The inoculation concentration was 2.5 × 10⁵ / mL, the medium volume was 100mL / bottle, cultured at 45 °C for 7 days, and the shaker speed was 150 rpm. The inoculation concentration was 2.5 × 10⁵ spores / mL, cultured at 45 °C, 150 rpm, and sampled on the 7th day to determine the ethanol content.
1) Sample handling:
   Take 1 mL fermentation broth into a 1.5mL centrifuge tube, centrifuge at 12000rpm for 10min, and take the supernatant to determine the ethanol content.
2) Determination of ethanol content

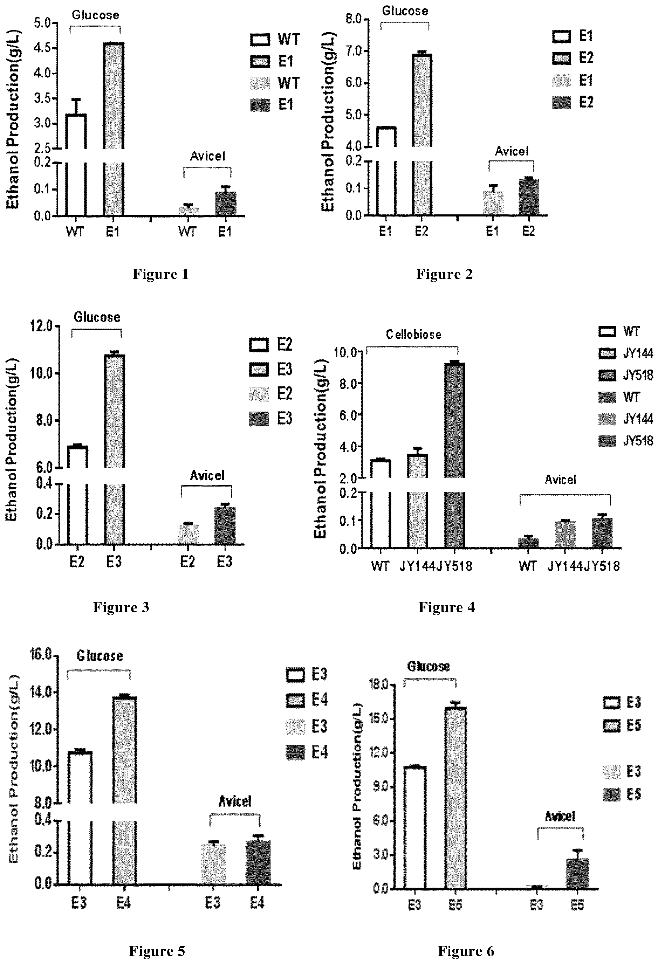

The ethanol content of the treated samples was determined by high performance liquid chromatography, in which the detector was differential detector, 5 mM H₂SO₄ was mobile phase, and the flow rate was 0.5mL/min. The obtained strain overexpressing pAN52-PelF-Scadh1 in wild-type strain ATCC42464 was named strain E1. The results showed that the overexpression of *Scadh1* gene in wild-type strain ATCC42464 could significantly promote the production of ethanol. When glucose was used as carbon source for fermentation for 7 days, the ethanol yield of strain E1 was 4.60 g / L (as shown in Figure 1), which was 45.1% higher than that of wild-type strain (*M. thermophila*ATCC42464, 3.17 g / L). When fermenting with cellulose (Avivel) as carbon source for 7 days, the ethanol yield of strain E1 was 87 mg / L, which was significantly higher than that of wild-type strain (*M*. *thermophilaATCC42464,* 30.5 mg / L), which was increased by 2.8 times. The results showed that overexpression of ethanol dehydrogenase *Scadh1* could significantly increase the ethanol production of *M*. *thermophila* when using glucose and cellulose (Avicel) as carbon sources.

### Example 2

### 1. Construction of pdc1 overexpression vector (pAN52-pdc1)

Using the genomic DNA of S. *cerevisiae* S288C (taxID: 559292) as the template, *pdc1* gene (gene ID: 850733) was amplified, and the 1.175kb fragment upstream of the translation extension factor coding reading frame (Mycth_2298136) (named Ptef promoter) was used as the promoter (the nucleic acid sequence is shown in SEQ ID No.2), The two fragments were recombined into the linearized vector pAN52-TB-Intron digested by Xho I and BamHI by Gibson assembly of NEB, and the recombinant expression plasmid pAN52-tef-pdc1 of *pdc1* gene was obtained.

The PCR reaction system and reaction conditions in this example are the same as those described in the vector construction in Example 1.

The primers used for vector construction in this example are as follows:

| **SEQ ID NO.** | **Primer** | **Sequence** (**5'-3'**) |
|---|---|---|
| 19 | pdc1-Gib-Ptef-F | TACCGTCAAAATGTCTGAAATTACTTTGGG |
| 25 | pdc1-Gib-Ptef-R | GATTTCAGTAACGTTAAGTGGATCCTTATTGCTTAGCGTTGGTAG |
| 30 | Ptef-Gib-pdc 1-F | TCTGCAGATCTTTAATTAACTCGAGCACCCGCCATGATTCCGTAG |
| 31 | Ptef-Gib-pdc1-R | TTTCAGACATTTTGACGGTATTTGTGTTCTGAAGAAC |

The transformation method and verification method of subsequent target gene overexpression vector into *M. thermophila* are the same as those described in Example 1.

### 2. Determination of ethanol production capacity of M. thermophila transformants

The starting strain of this transformation is strain E1. The strain overexpressing pyruvate decarboxylase gene *pdc1* obtained by transformation is named strain E2. Strain E2 and strain E1 were inoculated in 75 g / L glucose and 75 g / L cellulose (Avicel) medium respectively (see example 1 for the formula). The transformed sporozoites collected with distilled water were filtered by two layers of distilled mirror paper, and the number of spores was calculated. The inoculation concentration was 2.5 × 10⁵/ mL, the volume of culture medium was 100mL / bottle, cultured under 45 °C light for 7 days, and the rotating speed of shaker was 150rpm.

The supernatant was centrifuged to determine the content of ethanol. The results are shown in Figure 2: when glucose was used as carbon source, the ethanol yield of strain E2 was 6.87 g / L, which was 49.3% higher than that of the original strain E1 (4.60 g / L). When fermenting with cellulose (Avicel) as carbon source for 7 days, the ethanol yield of E1 strain was 87 mg / L and that of E2 strain was 129 mg / L, which was 48.3% higher than that of E1. It shows that overexpression of pyruvate decarboxylase gene *pdc1* can increase the ethanol production of *M*. *thermophila* under the conditions of glucose and cellulose (Avicel).

### Example 3

### 1. Construction of glt-1 expression vector of glucose transporter gene

The glucose transporter coding gene *glt-1* (NCU01633, GeneID:3872148) was amplified from the genome of *Neurospora crassa.* The 1372bp fragment upstream of the glycosylaldehyde-3-phosphate dehydrogenase coding gene *gpdA* of *M. thermophila* (Mycth_2311855) (as shown in SEQ ID No.15) was used as the promoter, and the Tcbh1 fragment downstream of the cellobiose hydrolase coding gene *cbh-1* of *M*. *thermophila* (Mycth_109566) was used as the Terminator (as shown in SEQ ID No.7), Glyphosate resistance gene (*bar*) was used as screening marker. The above fragments were recombined into the linearized vector pCAMBIA-0380 digested by BglII by Gibson assembly of NEB, so as to obtain the *glt-1* overexpression vector p0380-PgpdA-1633-bar.

The PCR reaction system and reaction conditions in this example are the same as those described in Example 1.

The primers used for vector construction in this example are as follows:

| **SEQ ID NO.** | **Primer** | **Sequence** (**5'-3'**) |
|---|---|---|
| 36 | P-bar-F | AAGAGGAGTCCACCATGGTACTCGACAGAAGATGATATTG |
| 37 | P-bar-R | AACGTTAAGTTCAGATCTCGGTGACGGG |
| 39 | TtrpC-bar-F | CGAGATCTGAACTTAACGTTACTGAAATCATC |
| 41 | TtrpC-bar-R | GCTAGCGTTAACACTAGTCACCTCTAAACAAGTGTACC |
| 51 | Tcbhl-F | AGCCATGGAGAGGTTTAGCACGAACCTCTCTGAAGGAG |
| 52 | Tcbhl-R | GTATGATGGGTCAGTTCAG |
| 54 | 1633-F | ATGGGTCTCTTCTCGAAA |
| 55 | 1633-R | CTAAACCTCTCCATGGCT |
| 56 | PgpdA-F | AAGAGGAGTCCACCATGGTACTTGCATCGTCCCAAAGC |
| 57 | PgpdA-R | TTTCGAGAAGAGACCCATTTTGATTTCTGTGATGTGGG |

### 2. Analysis of ethanol production by recombinant transformant of M. thermophila

The constructed gene expression vector p0380-PgpdA-1633-bar was integrated into the genome of the starting strain, *M. thermophila* E2 strain (named E3 strain), and the final concentration of 100 µg / mL glyphosate was used as the screening antibiotic. See Step 2 of Example 1 for the method. The obtained transformants were verified by using primers PgpdA-F and 1633-R, the PCR system and method are shown in step 1 of example 1.

Inoculate all the verified transformants into 100mL medium with glucose as carbon source in 250mL triangular flask (see Step 3 of example 1 for the formula), and the inoculation concentration is 2.5 × 10⁵ spores / mL, 45 °C, 150 rpm culture. After the sample is treated by the method described in step 3.1 of example 1, the ethanol content in the fermentation broth is determined. The method is the same as that in step 3.2 of example 1. Results as shown in Figure 3, overexpression of glucose transporter in *M. thermophila* significantly promoted the production of ethanol under glucose conditions. On the 7th day, the ethanol production of strain E3 reached 10.74g/L, which was 56.3% higher than that of control strain E2 (6.87g / L).

When cellulose (Avicel) was used as carbon source for 7 days, the ethanol yield of strain E3 was 0.238g/L, which was significantly higher than that of strain E2 (0.129 g / L), an increase of 84.5%. It shows that overexpression of glucose transporter / hexose transporter gene *glt-1* can significantly increase the ethanol production of *M*. *thermophila* under the conditions of glucose and cellulose (Avicel).

### Example 4

In this example, firstly, JY144 strain was obtained by overexpressing the ethanol dehydrogenase gene *Scadh1* in the wild-type strain ATCC42464. Then, the genome editing technology based on CRISPR / Cas9 (Qian Liu, et al. Development of a genome-editing CRISPR/Cas9 system in thermophilic fungal Myceliophthora species and its application to hyper-cellulase production strain engineering. Biotechnol Biofuels 2017, 10:1) is adopted, the cellobiose transporter coding genes *cdt-1* and *cdt-2* were fixed-point integrated into the *ldh-1* and *ldh-2* loci of the genome of strain JY144 respectively. The obtained strain was namedJY518to achieve the effect of overexpression of the target gene and knockout of the metabolic branch at the same time. The specific process is as follows:

### 1. Construction of Scadh1 overexpression vector (pAN52-Scadh1)

The expression vector was constructed with pAN52-TB-Intron (Environ Microbiol. 015.17(4):1444-62) as the skeleton. The P *tef* fragment of *tef gene* promoter (SEQ ID No.1) was inserted into the linearized vector pAN52-TB-Intron digested by BglII and Spe I to obtain the recombinant plasmid pAN52-MtTef-TtrpC-neo. Then, the fragment of *Scadh1* (Gene ID: 854068) encoding ethanol dehydrogenase from *S. cerevisiae* S288C was amplified with primersTef-ScADH1-F/R, and recombined into the linearized vector pAN52-MtTef-TtrpC-neo digested by EcoRI and BamHI with Gibson assembly kit of NEB to obtain the expression vector pAN52-MtTef-Scadh1. The transformation and identification methods of *M. thermophila* are the same as those in step 2 of Example 1.

### 2. Construction of sgRNA expression frame vector

The protopacer (eg. The target site) of the target genes *ldh-1* (Mycth_38939) and *ldh-2* (Mycth_110317) was designed by software sgRNACas9 tool. The sequence sgRNA promoter, protopacer and sgRNA were connected by fusion PCR, and the sgRNA expression frame vector was constructed by gene overlap extension (SOE). The PCR reaction system and reaction conditions were the same as those in Example 1.

sgRNA expression plasmids U6p-ldh1-sgRNA and U6p-ldh2-sgRNA were obtained by SOE-PCR amplification, and their sequences were shown in SEQ ID No.12 and SEQ ID No.9, respectively.

### 3. Construction of donor DNA vector

Using the 1372bp fragment upstream of glycosylaldehyde-3-phosphate dehydrogenase coding gene *gpdA* (Mycth_2311855) of *M. thermophila* as the promoter of *cdt-1* (SEQ ID No.15), and the genome of *M*. *thermophila* as the template, and mediated by primers, the cellobiose transporter coding gene *cdt-1* of *Neurospora crassa* (NCU00801,GeneID:3879950) and about 900bp upstream / downstream homologous fragment of *ldh-1* gene were amplified by PCR. Then, the Gibson assembly of NEB was used to recombine them into the linearized vector pAN52-TB-Intron digested by Spe I and EcoRV, and the obtained vector was sequenced. Donor DNA fragment donor-*cdt1* that the gene *cdt-1* regulated by promoter PgpdA was obtained, and its sequence was shown in SEQ ID No.16

Using the genome of *Neurospora crassa* as a template, and mediated by primers, the cellobiose transporter coding gene *cdt-2* (NCU08114, GeneID: 3880022) of *Neurospora crassa* was amplified by PCR. The 730bp fragment Ppdc upstream of pyruvate decarboxylase encoding reading frame *pdc* (Mycth_112121) was used as the promoter of *cdt-2* (as shown in SEQ ID No.17), glyphosate resistance gene *Bar* was used as a screening marker, and about 1000bp homologous fragment upstream / downstream of *ldh-2* gene, the Gibson assembly of NEB was used to recombine them into the linearized vector pAN52 -TB-Intron digested by Spe I and EcoRV, and the obtained vector was sequenced. The donor DNA fragment donor-cdt2 of cdt-1 gene which expression was under the regulation of promoter PgpdA was obtained, and the sequence is shown in SEQ ID No.20.

The PCR reaction system and reaction conditions in this example are the same as those described in the construction of vector in Example 1.

The primers used for vector construction in this example are as follows:

| **SEQ ID NO.** | **Primer** | **Sequence (5'-3')** |
|---|---|---|
| 43 | Tef-F | GCAGATCTCATGTACCTTGACGTCCTCCGAG |
| 44 | Tef-R | AAACACTAGTTCTGAAGAACGAAACTGGCGACT |
| 45 | Tef-ScADH1-F | TTTAAACGATATCGAATTCGAGCATACAATCAACTATCTC |
| 46 | Tef-ScADH1-R | GATTTCAGTAACGTTAAGTGCTTTAAAATTTGTATACAC |
| 58 | cdt1-F | TCACAGAAATCAAAACTAGAATGTCGTCTCACGGCTC |
| 59 | cdt1-R | TTAAGTGGATCCGAATTCGATATCCTAAGCAACGATAGCTTCGGA C |
| 60 | 38939-up-F | AAGAGGAGTCCACCATGGTACAGGATATTGATAACACC |
| 61 | 38939-up-R | CGATCTCTAGAAGTCCTCGTCCCGAGCCAAACACTTGG |
| 62 | 38939-down-F | CAGGTACACTTGTTTAGAGGCCGCTGGACAGGTTCGTG |
| 63 | 38939-down-R | GCTAGCGTTAACACTAGTCAGCCCATCTGTTGTTAACC |
| 64 | pAN52-F | ACGAGGACTTCTAGAGATCGTTGCATCGTCCCAAAGC |
| 65 | TtrpC-bar-R2 | CCTCTAAACAAGTGTACCTG |
| 66 | Ppdc-F2 | TCAAGTCCAGCATAGCGAC |
| 67 | TtrpC-bar-R2 | CCTCTAAACAAGTGTACCTG |
| 68 | 110317-up-F | AAGAGGAGTCCACCATGGTACGTCAGGTGTCTGTTCTC |
| 69 | 110317-up-R | GTCGCTATGCTGGACTTGAGTACGCAGTCGTCACGCC |
| 70 | 110317-down-F | CAGGTACACTTGTTTAGAGGTATTCCGGGACGTAGTGC |
| 71 | 110317-down-R | GCTAGCGTTAACACTAGTCACTCCAACTCGCTGAGGAG |

### 2. Determination of the ability of the M. thermophila transformants to produce ethanol from cellobiose

Firstly, the vector pAN52-MtTef-Scadh1 was transferred into the genome of wild-type ATCC42464 of *M*. *thermophila,* and the obtained strain overexpressing *Scadh1* gene was named as JY144 strain. Then, taking JY144 strain as the starting strain, *cdt-1* and *cdt-2* expression vectors, Cas9 fragment, U6p-ldh1-sgRNA, U6p-ldh2-sgRNA were transformed into JY144 strain, and the final concentration of glyphosate was 100µg / mL as the screening antibiotic. The method is shown in step 2 of Example 1. The transformants were simultaneously verified by primer pairs 38939-up-F/38939-down-R and 11 0317-up-F/11 0317-down-R.

Inoculate the verified correct transformants into the medium with cellobiose and cellulose (Avicel) as carbon sources (see step 3 of Example 1 for the formula, only replace glucose with cellobiose / cellulose), and the inoculation concentration is 2.5 × 10⁵ spores / mL, 45 °C, 150 rpm culture. After the sample is treated by the method described in step 3.1 of Example 1, the ethanol content in the fermentation broth is determined. The method is the same as that described in Example 1.

Results as shown in Figure 4, under the condition of cellobiose as carbon source, the ethanol yield of JY144 strain was 3.45g/L, which was higher than that of wild-type strain (3.08g/L). Based on JY144 strain, *cdt-1* and *cdt-2* were overexpressed and *ldh-1* and *ldh-2* genes were inactivated at the same time. The obtained strain was named JY518. When cellobiose was used as carbon source, the ethanol yield of JY518 on the 7th day was 9.20g/L, which was 1.67 times higher than that of the original strain JY144, which was 3.45g/L.

When cellulose (Avicel) was used as carbon source for fermentation for 7 days, the ethanol yield of JY518 strain was 0.104g/L, which was higher than that of the starting strain JY144 (0.0914g / L), increased by 13.8%, while the ethanol yield of wild-type strain (ATCC42464) was 0.0305g/L. The results showed that overexpression of cellobiose transporters *cdt-1* and *cdt-2* could increase the ethanol production of *M*. *thermophila* under cellobiose and cellulose conditions.

### Example 5

This example mainly aims at the negative regulatory genes lactate dehydrogenase *Ldh-2* (Mycth_110317) and mannitol 1-phosphate dehydrogenase *Mpd* (Mycth_2310298), and adopts the genome editing technology based on CRISPR / Cas9 (Biotechnol Biofuels 2017, 10:1) inactivate the target gene to knock out the metabolic branch and promote ethanol synthesis.

### 1. Vector construction

### (1) Construction of sgRNA expression frame

The protospacer (eg. The target site) of target genes *ldh-2* (Mycth_110317) and *mpd* (Mycth_2310298) was designed by the softwares gRNACas9 tool. The sgRNA promoter, protopacer and sgRNA were connected by fusion PCR, and the sgRNA expression frame vector was constructed by gene overlap extension (SOE).

The PCR reaction system and reaction conditions are as shown in Example 1.

sgRNA expression plasmids U6p-ldh2-sgRNA and U6p-mpd-sgRNA were obtained by SOE-PCR amplification, and their sequences are shown in SEQ ID No.9 and SEQ ID No.26, respectively.

### (2) Construction of donor DNA vector

In the invention, the donor DNA fragments are respectively connected to the plasmid PPk2BarGFP linearized by restriction endonuclease Xbal and EcoRV by Gibson assembly method from the homologous fragment of about 600bp upstream / downstream of the target gene and the PtrpC-bar fragment of glyphosate resistance gene expression frame, and finally construct the donor DNA fragments donor-ldh2 and donor-mpd. Their nucleic acid sequences are shown in SEQ ID No.27 and SEQ ID No.28, respectively.

The PCR reaction system and reaction conditions in this example are the same as those described in Example 1.

The primers used for vector construction in this example are as follows:

| **SEQ ID NO.** | **Primer** | **Sequence (5'-3')** |
|---|---|---|
| 76 | 110317UP-F | AGTTTATGTGGAAAATGGCA |
| 77 | 110317UP-R | CTCGTAGTCGCCGACGGACTATCCGTTGGGCGGAGCAT |
| 78 | 110317Down-F | CCAACGGATAGTCCGTCGGCGACTACGAGGACTGCG |
| 79 | 110317Down-R | TACGTGCCGACCCTCGAGAC |
| 80 | Cas9-F | TCCTCCGAGGTTCGACATCAGGGTT |
| 81 | Cas9-R | CTCTAAACAAGTGTACCTGTGCAT |
| 82 | 110317sgRNA-b | ACCTATCAAGGAGCAGCGAGGAAAGAAAGAAAAGAAGAGG |
| 83 | 110317sg RNA-c | TGCTCCTTGATAGGTTAGGTTTTAGAGCTAGAAATAGCAA |
| 84 | 110317doner-bar-F | acggatagtcATATTGAAGGAGCACTTTTTG |
| 85 | 110317doner-bar-R | gtcgccgacgGATCACTAGTACAGGATTC |
| 86 | KO 11 0317YZ- F | CGGATTCAAGATGCCTCAGCCA |
| 87 | KO 11 0317YZ- R | GACCGGGTTGGTGGCCACGA |
| 88 | 2310298-up-F | CCTGACTGTATAAGAGAAATG |
| 89 | 2310298-up-R | AGCGACCGGAGTAAGCTCCCGCCGTGGCAATTTCCCGAAC |
| 90 | 2310298-Down-F | AAATTGCCACGGCGGGAGCTTACTCCGGTCGCTGTCA |
| 91 | 2310298-Down-R | CCAATGAAGCGTTCCTTACGGCT |
| 92 | sgRNA-a | AGGATCGGTGGAGTGAAGTTCG |
| 93 | sgRNA-b | ACGGAGCAGGTAACCAAGTCGAGGAAAGAAAGAAAAGAAG |
| 94 | 2310298sgRNA-c | GACTTGGTTACCTGCTCCGTGTTTTAGAGCTAGAAATAGC |
| 95 | 2310298sgRNA-d | AAAAAAAGCACCGACTCGGTGCC |
| 96 | K02310298YZ-F | TGACTGACCGTGATGATTTCCAG |
| 97 | K02310298YZ-R | AGCCTAACGTCGAGACCGGCGT |

### 2. Determination of ethanol production capacity of M. thermophila transformants

Cas9 expression frame, sgRNA expression plasmids U6p-ldh2-sgRNA (SEQ ID No.9) and U6p-mpd-sgRNA (SEQ ID No.26), and donor DNA fragments donor-ldh2(SEQ ID No.27) and donor-mpd (SEQ ID No.28) were mixed in equal proportion and co-transformed into protoplast cells of *M*. *thermophila* strain E3. Cas9 protein was mediated by gRNA, the target site is identified and cut by pairing the target sequence with the DNA strand of *ldh-2* (Mycth_1 10317) and *mpd* (Mycth_2310298) on the host cell genome, and then the donor DNA fragment is homologously recombined with the sequences on both sides of the target site, so as to achieve the purpose of editing the genome. The transformants are screened by adding glyphosate to the plate. The transformation method and verification method of introducing the target gene fragment into *M. thermophila* are the same as those described in Example 1.

The transformant (named E4 strain) with simultaneous knockout of lactate dehydrogenase gene *ldh-2* and 1-phosphate mannitol dehydrogenase gene mpdwas obtained. Strain E4 and strain E3 were inoculated in 75 g / L glucose and 75 g / L cellulose (Avicel) medium, respectively (see example 1 for the formula). The transformed sporozoites collected with distilled water were filtered by two layers of distilled mirror paper, and the number of spores was calculated. The inoculation concentration was 2.5 × 10⁵ spores/ mL, the medium volume was 100mL / bottle, cultured at 45 °C for 7 days, and the shaker speed was 150 rpm.

The supernatant was centrifuged to determine the content of ethanol. The results are shown in Figure 5: the ethanol yield of strain E4 increased from 10.74g/L of E3 to 13.70g/L, an increase of 27.6%, indicating that the knockout of *ldh-2* and *mpd* genes is helpful to improve the ethanol yield of *M*. *thermophila* under glucose conditions.

When cellulose was used as carbon source for fermentation for 7 days, the ethanol yield of strain E4 was 0.265 g / L, which was higher than that of starting strain E3 (0.238 g / L), increased by 11.3%. The results showed that knockout of lactate dehydrogenase gene and 1-phosphate mannitol dehydrogenase gene could increase the ethanol production of *M*. *thermophilaon* using cellulose as the carbon source.

### Example 6

This example mainly aims at the negative regulatory gene cytoplasmic malate dehydrogenase *Mdh* (Mycth_2315052,GeneID:11512768), and adopts the genome editing technology based on CRISPR / Cas9 inactivate the target gene, reduce the shuttle of reducing force of cytoplasmic NADH to mitochondria, and then promote ethanol synthesis.

### 1. Construction of sgRNA expression frame

The protopacer (eg. The target site) of the target gene *mdh* (Mycth_2315052) is designed through the software sgRNACas9 tool. The sgRNA promoter, protopacer and sgRNA were connected by fusion PCR, and the sgRNA expression frame vector was constructed by gene overlap extension (SOE). The specific operations were as follows: firstly, the promoter and protopacer DNA fragments were amplified with primers sgRNA-a and 2315052 sgRNA-b; protopacer and scaffold fragments were amplified with primers 2315052 sgRNA-c and sgRNA-d, and then the full length of sgRNA expression frame was amplified with primers sgRNA-a and sgRNA-d using the two DNA fragments as templates amplified as above mentioned. The PCR reaction system and reaction conditions are as shown in example 1.

sgRNA expression plasmid U6p-mdh-sgRNA was obtained by SOE-PCR amplification, and its sequence is shown in SEQ ID No.32.

### 2. Construction of donor DNA vector

In the invention, the donor DNA fragment is respectively composed of the upstream homologous arm of the target gene, the PtrpC-bar fragment of the glyphosate resistance gene expression frame and the downstream homologous arm, which are connected together by fusion PCR, and finally constructed into the donor DNA fragment donor-mdh. The nucleic acid sequence is shown in SEQ ID No.33. The construction process was as follows: firstly, the upstream homologous arm DNA was amplified with primers 2315052donor-up-F and 2315052donor-up-R, the glyphosate resistance gene expression frame PtrpC-bar fragment was amplified with primers 2315052donor-bar-F and 2315052donor-bar-R, and the downstream homologous arm was amplified with 2315052donor-down-F and 2315052donor-down-R. Then, the upstream homologous arm and PtrpC-bar fragment were used as templates at the same time, and the Fusion DNA fragment of the upstream homologous arm and the glyphosate resistance gene expression frame was amplified with primers 2315052donor-up-F and 2315052donor-bar-R. Then, the Fusion DNA fragment and the downstream homologous arm were used as templates at the same time, and the full-length donor DNA fragment donor-mdh was amplified with primers 2315052donor-up-F and 2315052donor-down-R. Finally, the donor DNA sequence was proved to be correct by gene sequencing.

Construction of cas9 expression frame as published by the inventor (biotechnol biofuels 2017, 10:1), the nucleic acid sequence is shown in SEQ ID No.29.

The PCR reaction system and reaction conditions in this example are the same as those described in Example 1.

The primers used for vector construction in this example are as follows:

| **SE Q ID NO.** | **Primer** | **Sequence (5'-3')** |
|---|---|---|
| 98 | 2315052doner-up-F | GGGTGGTGTGCATGGGGCCGTCT |
| 99 | 2315052doner-up-R | AAAGTGCTCCTTCAATATGCAGCAGGGTCAGCCGGGTCT |
| 100 | 2315052doner-Bar-F | CGGCTGACCCTGCTGCATATTGAAGGAGCACTTTTTGGGC |
| 101 | 2315052doner-Bar-R | GGTCATGCCGGGCTTTTAAGAAACTTTATTGCCAA |
| 102 | 2315052doner-Down-F | GGCAATAAAGTTTCTTAAAAGCCCGGCATGACCCGTGATG A |
| 103 | 2315052doner-Down-R | CGTACTGAAGCTCTTCCTCTATCCT |
| 104 | 2315052 sgRNA-b | TTGGCGGGCAGGTAGCCGAGGAAAGAAAGAAAAGAAGAG |
| 105 | 2315052 sgRNA-c | CTACCTGCCCGCCAACGAGTTTTAGAGCTAGAAATAGCAA |
| 106 | sgRNA-a | AGGATCGGTGGAGTGAAGTTCG |
| 107 | sgRNA-d | AAAAAAAGCACCGACTCGGTGCC |
| | | |
| | | |

### 3. Determination of ethanol production capacity of M. thermophila transformants

Cas9 expression frame, sgRNA expression plasmid U6p-mdh-sgRNA and donor DNA fragment donor-mdh were mixed in equal proportion and co-transformed into protoplast cells of strain E3 and strain E1. Cas9, mediated by gRNA, identified the target site by pairing the target sequence with the DNA strand of *mdh* on the host cell genome, and then homologous recombination occurred between the donor DNA fragment and the sequences on both sides of the target site. In order to achieve the purpose of gene editing, the transformants were screened by adding glufosinate to the plate. The transformation method and verification method of introducing the target gene fragment into *M. thermophila* are the same as those described in Example 1, and strain E5 (starting strain E3) and strain E7 (starting strain E1) are obtained respectively.

Strain E5 and its starting strains E3, E7 and its starting strain E1 were inoculated into 75 g / L glucose and 75 g / L cellulose (Avicel) medium, respectively (see example 1 for the formula). The transformed sporozoites obtained were collected with distilled water and filtered by two layers of distilled mirror paper. The number of spores was calculated. The inoculation concentration was 2.5 × 10⁵ / mL, the volume of medium was 100mL / bottle, cultured at 45 °C for 7 days, and the rotating speed of shaker was 150 rpm. The supernatant was centrifuged to determine the content of ethanol.

The results are shown in Figure 6: when glucose is used as carbon source, the ethanol yield of strain E5 reaches 15.96g/L, which is 48.6% higher than that of the original strain E3 (10.74g / L). When cellulose (Avicel) was used as carbon source, the ethanol yield of strain E5 was 2.568 g / L, which was significantly higher than that of original strain E3 (0.238 g / L), which was increased by 9.8 times. It shows that the knockout of cytoplasmic malate dehydrogenase *mdh* can significantly increase the ethanol production of *M. thermophila* under the conditions of glucose and cellulose (Avicel)

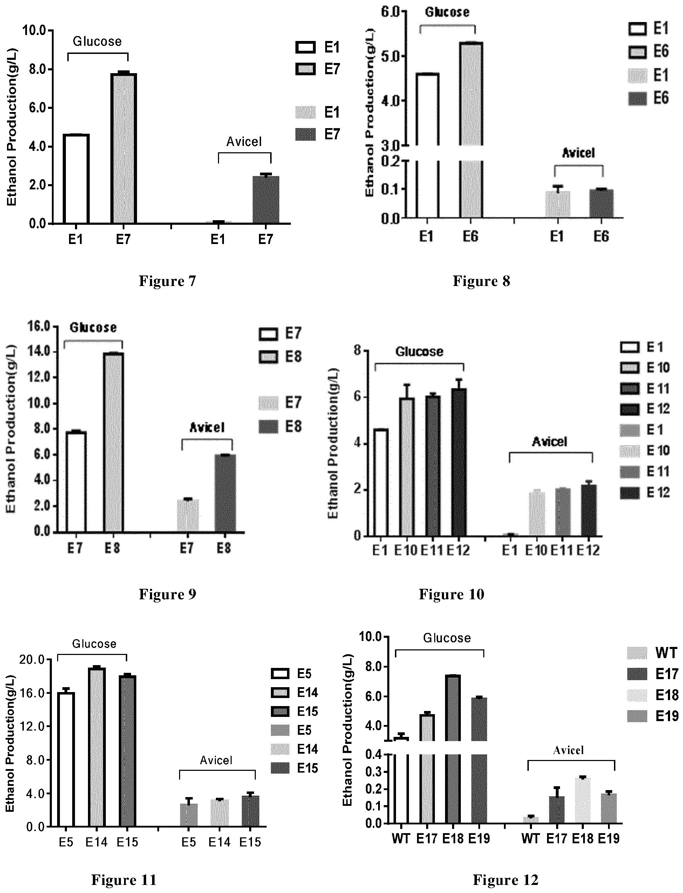

Similarly, as shown in Figure 7: when glucose is used as carbon source, the ethanol yield of strain E7 reaches 7.73g/L, which is 68.04% higher than that of control strain E1 (the yield is 4.60g/L). When cellulose (Avicel) was used as carbon source, the ethanol yield of strain E7 was 2.41 g / L, which was significantly higher than that of strain E1 (0.087 g / L), increased by 26.7 times. It shows that the knockout of cytoplasmic malate dehydrogenase gene *mdh* can increase the content of cytoplasmic NADH, so as to improve the ethanol production capacity under the conditions of glucose and cellulose.

### Example 7

This example mainly applies CRISPR / dCas9 technology to down-regulate the transcription level of cytochrome C oxidase coding gene (Mycth_2312390, GeneID:11509370), a key gene of mitochondrial respiratory chain, so as to promote the synthesis of ethanol by *M*. *thermophila.* The details are as follows.

### 1. Construction of expression vector

### (1) Amplification of dCas9 protein coding sequence

dCas9 protein mutates two key domains in Cas9, RuvC and HNH (D10A and H840A), making it lose the activity of cutting DNA. Mutation method: use primers to introduce the two mutation sites (D10A and H840A) at the same time, take the vector p0380-bar-Ptef1-Cas9-TtprC as the template (Biotechnol Biofuels 2017, 10:1), amplify the pre / post Cas9 sequences with primers respectively, and connect the above fragments together by fusion PCR to obtain dCas9 nucleic acid fragment, as shown in SEQ ID No.38.

KRAB domain is a protein-protein interaction region, which can bind to a variety of synergistic transcription inhibitors, so that KRAB zinc finger protein can play a transcription inhibitory function dependent on DNA binding as a transcription factor and / or transcription regulator. The KRAB domain used in the invention comes from human zinc finger protein 10 (homo sapiens zinc finger protein 10), which is synthesized artificially after codon optimization as SEQ ID No.40.

dCas9 was connected with KRAB nucleic acid fragment by fusion PCR to obtaindCas9KRAB fragment. After enzyme digestion with *Pme*I and *Pac*I, it was connected to the linearized vector p0380-bar-Ptef1-Cas9-TtprC digested by the same enzymes, and the recombinant expression plasmidp0380-bar-Ptef1-dCas9KRAB-TtprC was obtained.

### (2) Construction of sgRNA expression frame

The protopacer in the upstream promoter region of the target gene Mycth_2312390 which is the target site was designed by software sgRNACas9 tool. The sequence sgRNA promoter, protopacer and sgRNA were connected by fusion PCR, and the sgRNA expression frame vector U6p-2312390-sgRNA was constructed by gene overlap extension (SOE). The nucleic acid sequence is shown in SEQ ID No.42.

The PCR reaction system and reaction conditions in this example are the same as those described in Example 1.

The primers used for vector construction in this example are as follows:

| **SEQ ID NO.** | **Primer** | **Sequence** (**5'-3'**) |
|---|---|---|
| 120 | dCas9-a | AAGCTTATGGACCCCAAGAAGAAACGCAAGGTTGATAAGAAGTACTCC |
| | | ATCGGCCTCGCCAT |
| 121 | dCas9-b | TGAGGGACGATAGCATCGACATCGTAATCGGA |
| 122 | dCas9-c | CGATGTCGATGCTATCGTCCCTCAGTCCTTCC |
| 123 | dCas9-d | AGCGGCCGCCACCTTCCTCTTTT |
| 124 | dCas9-KR AB-F | GGGTTTAAACATGGACCCCAAGAAGAAACGCAA |
| 125 | dCas9-KR AB-R | TAGCATCCATAGCGGCCGCCACCTTCCT |
| 126 | KRAB-F | CGGCCGCTATGGATGCTAAGTCACTAACT |
| 127 | KRAB-R | CCTTAATTAATGCAGTCTCTGAATCAGGATGGGT |

### 2. Transformation of M. thermophila and identification of transformants

The p0380-bar-Ptef1-dCas9KRAB-TtprC vector was linearized by EcoRV and transformed into the starting strain E1 in the same amount as U6p-2312390-sgRNA (see Example 1 for construction and transformation methods). After obtaining the transformants, the transformants were identified by primers dCas9-KRAB-Fand KRAB-R(verifying that dCas9KRAB was integrated into the genome), 2312390Sg-c and 2312390Sg-d (verifying that U6p-2312390-sgRNAwas integrated into the genome). The obtained transformants were verified by PCR. The PCR system and method are shown in example 1. The transformants successfully transformed into p0380-bar-Ptef1-dCas9KRAB-TtprCand 2312390SgRNA were named strain E6.

### 3. Determination of ethanol production capacity of M. thermophila transformants

Strain E6 and starting strain E1 were inoculated into the culture medium of 75 g / L glucose and 75 g / L cellulose (see example 1 for the formula). The transformed sporozoites collected with distilled water were filtered by two layers of distilled mirror paper, and the number of spores was calculated. The inoculation concentration was 2.5 × 10⁵ / mL, the volume of culture medium was 100mL / bottle, cultured at 45 °C for 7 days, and the rotating speed of shaker was 150 rpm.

The supernatant was centrifuged to determine the ethanol content. The results are shown in Figure 8. When glucose was used as carbon source, the ethanol yield of transformant E6 reached 5.29 g / L, which was 15% higher than that of starting strain E1 (4.60 g / L). When cellulose (Avicel) was used as carbon source for fermentation for 7 days, the ethanol yield of strain E6 was 0.094 g / L, which was higher than that of starting strain E1 (0.087 / L), increased by 8%, indicating that the down-regulation of respiratory chain intensity can improve the ethanol yield of *M*. *thermophila* on glucose and cellulose (Avicel).

### Example 8

In order to improve the level of NADH in the cytoplasm, the glycerol-3-phosphate dehydrogenase gene gpd(Mycth_2313529, GeneID:11508057) in the ethanol synthesis strain of *M*. *thermophila* was knocked out to further improve the ethanol synthesis efficiency of the transformants.

This example constructs the sgRNA expression frame U6p-gpd-sgRNA (as shown in SEQ ID No.34) and its donor DNA expression vector donor-gpd (as shown in SEQ ID No.35) according to the method described in Example 6.

The PCR reaction system and reaction conditions in this example are the same as those described in Example 1.

The primers used for vector construction in this example are as follows:

| **SEQ ID NO.** | **Primer** | **Sequence** (**5'-3'**) |
|---|---|---|
| 110 | 2313529doner-up-F | TGGGACGGCGAGAGCGACGGGT |
| 111 | 2313529doner-up-R | GCGTCGAGAAGGCAGAGTCTGTGGATCGTGC |
| 112 | 2313529doner-Down-F | AGACTCTGCCTTCTCGACGCCGTCAGGG |
| 113 | 2313529doner-Down-R | TAGCGCCAGTTGCGTGCCGAGGT |
| 114 | 2313529sgRNA-b | CGGGGATTGTGACATTCTCCGAGGAAAGAAAGAAAAGAA G |
| 115 | 2313529sgRNA-c | GGAGAATGTCACAATCCCCGGTTTTAGAGCTAGAAATAGC |
| 116 | sgRNA-a | AGGATCGGTGGAGTGAAGTTCG |
| 117 | sgRNA-d | AAAAAAAGCACCGACTCGGTGCC |
| 118 | K02313529YZ-F | CGCTCTCATTCCCCCTCGCAG |
| 119 | K02313529YZ-R | ATATACTCGCAAATCAGCTCGA |

### 2. Determination of ethanol production capacity of M. thermophila transformants

Cas9 expression frame, sgRNA expression plasmid U6p-gpd-sgRNA and donor DNA fragment donor-gpd were mixed in equal proportion and co-transformed into protoplast cells of *M. thermophila* E7 strain (see Example 6 for the construction process). Cas9 identified the target site through the pairing of the target sequence with the DNA strand of *gpd* on the host cell genome mediated by gRNA, and then the donor DNA fragment was homologously recombined with the sequences on both sides of the target site, so as to achieve the purpose of editing the genome. The transformation method and verification method of introducing the target gene fragment into *M. thermophila* are the same as those described in example 1. The transformant that successfully knocked out the *gpd* gene in strain E7 is named strain E8.

Strain E8 and its starting strain E7 were inoculated in 75 g / L cellulose (Avicel) medium and 75 g / L glucose medium (See example 1 for the formula). The transformed sporozoites collected with distilled water were filtered by two layers of distilled mirror paper, and the number of spores was calculated. The inoculation concentration was 2.5 × 10⁵ spores / mL, the medium volume was 100mL / bottle, cultured at 45 °C for 7 days, and the shaker speed was 150 rpm.

The supernatant was centrifuged to determine the ethanol content. The results are shown in Figure 9: on the 7th day, under the condition of glucose as carbon source, the ethanol yield of strain E8 reached 13.84g/L, which was 79% higher than that of control strain E7 (the yield was 7.73g/L). Under the condition of cellulose (Avicel), the ethanol yield of strain E8 was significantly increased to 5.91 g/L, which was 145.2% higher than that of strain E7 (2.41 g / L). It shows that inactivation of cytoplasmic glycerol-3-phosphate dehydrogenase Gpd can significantly improve the ethanol production of *M*. *thermophila* under the conditions of cellulose (Avicel) and glucose.

### Example 9

This example mainly aims at the NADH dehydrogenase gene *nde* outside the negative regulation gene (Mycth_2304268, Gene ID: 11507602 encodes *nde1,* Mycth_2304512, Gene ID: 11507705 encodes *nde2*), and adopts the genome editing technology based on CRISPR / Cas9. Inactivate the target gene, reduce the transmission of cytoplasmic NADH reducing force to mitochondria, and then promote ethanol synthesis.

### 1. Construction of sgRNA expression frame

The protopacer (eg. The target site) of target genes *nde1* (Mycth_2304268) and *nde2* (Mycth_2304512) was designed by software sgRNACas9 tool. The sequence sgRNA promoter, protopacer and sgRNA were connected by fusion PCR, and the sgRNA expression frame vector was constructed by gene overlap extension (SOE).

The PCR reaction system and reaction conditions are shown in example 1.

sgRNA expression plasmids U6p-nde1-sgRNAand U6p-nde2-sgRNA were obtained by SOE-PCR amplification. The sequences are shown in SEQ ID No.144 and SEQ ID No.146, respectively. The detailed construction process is shown in example 6

### 2. Amplification of donor DNA

In the invention, the donor DNA fragment consists of about 700bp homologous fragment upstream / downstream of the target gene and PtrpC-bar fragment of glyphosate resistance gene expression frame respectively. The full-length linearized donor DNA fragments donor-ndel and donor-nde2 are obtained by fusion PCR, and their nucleic acid sequences are shown in SEQ ID No.145 and SEQ ID No.147, respectively. The detailed construction process is shown in example 6.

The PCR reaction system and reaction conditions in this example are the same as those described in Example 1.

The primers used for vector construction in this example are as follows:

| **SEQ ID NO.** | **Primer** | **Sequence (5'-3')** |
|---|---|---|
| 148 | sgRNA-a | AGGATCGGTGGAGTGAAGTTCG |
| 149 | 2304268sgRNA-b | CTGATAGAAGCCAATCGAGGAAAGAAAGAAAAGAAGA |
| 150 | 2304268sgRNA-c | GATTGGCTTCTATCAGGGCTGTTTTAGAGCTAGAAATAGC |
| 151 | sgRNA-d | AAAAAAAGCACCGACTCGGTGCC |
| 152 | 2304268donor-up-F | CGGGCTCAGCCGGAACTTGCTACC |
| 153 | 2304268donor-up-R | CCTTCAATATGGCTAGCAACGGGGTGAAG |
| 154 | 2304268donor-Bar-F | GTTGCTAGCCATATTGAAGGAGCACTTTTTG |
| 155 | 2304268donor-Bar-R | GGGATGACCGGATCACTAGTACAGGATTC |
| 156 | 2304268donor-Down-F | ACTAGTGATCCGGTCATCCCCGCTCCTT |
| 157 | 2304268donor-Down-R | GATCAAGGGGTTTGGCATGAG |
| 158 | sgRNA-a | AGGATCGGTGGAGTGAAGTTCG |
| 159 | 2304512sgRNA-b | TCTGGCGGAGGATGGTCGAGGAAAGAAAGAAAAGAAG |
| 160 | 2304512sgRNA-c | ACCATCCTCCGCCAGAAGAGTTTTAGAGCTAGAAATAGCA |
| 161 | sgRNA-d | AAAAAAAGCACCGACTCGGTGCC |
| 162 | 2304512donor-up-F | TCCTCCTCCCTCTCTCAGGCGCC |
| 163 | 2304512donor-up-R | CCTTCAATATAGTTGCGCGGGGAGATGAC |
| 164 | 2304512donor-Bar-F | CCGCGCAACTATATTGAAGGAGCACTTTTTG |
| 128 | 2304512donor-Bar-R | CCGGATCTCGGATCACTAGTACAGGATTC |
| 129 | 2304512donor-Down-F | ACTAGTGATCCGAGATCCGGGGTGACACC |
| 130 | 2304512donor-Down-R | CAGTCGCCCACGGCCCAGATGT |

### 3. Determination of ethanol production capacity of M. thermophila transformants

After Cas9 expression frame, sgRNA expression plasmid U6p-nde-sgRNA and donor DNA fragment donor-nde are mixed in equal proportion and co-transformed into protoplast cells of *M*. *thermophila* E1 strain, Cas9 recognizes the target site by pairing the target sequence with the DNA strand of *nde* on the host cell genome mediated by gRNA, and then homologous recombination occurs between the donor DNA fragment and the sequences on both sides of the target site, so as to achieve the purpose of genome editing. Transformants were screened by adding glufosinate to the plate. The transformation method and verification method of introducing the target gene fragment into *M. thermophila* are the same as those described in Example 1,

The starting strain of this transformation is strain E1. The strain obtained by knocking out NADH dehydrogenation gene *nde1*is named strain E10, the strain obtained by knocking out *nde2* is named strain E11, and the strain knocked out *nde1*and *nde2* is named strain E12. Strains E10, E11, E12 and E1 were inoculated in 75 g / L glucose medium and 75 g / L cellulose (Avicel) medium respectively (see example 1 for the formula). The ethanol content was measured after 7 days of culture. The results are shown in Figure 10: in glucose medium, the ethanol yield of strains E10, E11 and E12 were 5.94 g / L, 6.02 g / L and 6.33 g / L respectively, which were higher than that of the original strain E1 (4.60 g / L), 29.1%, 30.9% and 37.6% higher than that of E1 respectively. Under the condition of using cellulose (Avicel) as carbon source, the results are shown in Figure 10: the ethanol yields of strains E10, E11 and E12 are 1.82g/L, 2.01g/L and 2.17g/L respectively, which are higher than those of the starting strain E1 (0.087 g / L), 19.92 times, 22.10 times and 23.94 times higher than those of E1 respectively. The results showed that the knockout of NADH dehydrogenase could increase the content of NADH in the cytoplasm of *M. thermophila,* and then increase the yield of ethanol under the conditions of glucose and cellulose.

### Example 10

In vivo, the last step of ethanol synthesis pathway is catalyzed by ethanol dehydrogenase. However, this step is reversible, and some ethanol dehydrogenase is more inclined to catalyze the decomposition of ethanol. In addition, acetaldehyde, the substrate of ethanol dehydrogenase, will also be catalyzed by endogenous acetaldehyde dehydrogenase to produce acetyl coA. In order to avoid the consumption of ethanol and acetaldehyde and improve the accumulation of ethanol in the fermentation broth, the inventor found the potential ethanol and acetaldehyde consumption of ethanol dehydrogenase and acetaldehyde dehydrogenase by analyzing the transcriptome data under ethanol stress, which were verified by experiments. The main genes identified were ethanol dehydrogenase gene *Mtadh* (Mycth_55576) and acetaldehyde dehydrogenase gene *Mtaldh* (Mycth_2140820). Therefore, this example knocks out these two genes.

### 1. Construction of sgRNA expression frame

The protopacer (eg. The target site) of target genes *mtadh* (Mycth_55576) and *mtaldh* (Mycth_2140820) was designed by software sgRNACas9 tool. The sequence sgRNA promoter, protopacer and sgRNA were connected by fusion PCR, and the sgRNA expression frame vector was constructed by gene overlap extension (SOE).

sgRNA expression plasmids U6p-mtadh-sgRNAand U6p-mtaldh-sgRNA were obtained by SOE-PCR amplification. The detailed construction process is shown in example 6. The sequences are shown in SEQ ID No.11 and SEQ ID No.13 respectively.

### 2. Amplification of donor DNA

In the invention, the donor DNA fragment consists of about 700bp homologous fragment upstream / downstream of the target gene and PtrpC-hygR fragment of hygromycin resistance gene expression frame respectively. The full-length linearized donor DNA fragments donor-*mtadh* and donor-*mtaldh* are obtained by fusion PCR, and their nucleic acid sequences are shown in SEQ ID No.8 and SEQ ID No.10, respectively.

The PCR reaction system and reaction conditions in this example are the same as those described in Example 1.

The primers used for vector construction in this example are as follows:

| **SEQ ID NO.** | **Primer** | **Sequence** (**5'-3'**) |
|---|---|---|
| 131 | sgRNA-a | AGGATCGGTGGAGTGAAGTTCG |
| 132 | 55576sgRNA-b | CGGCGCTCTGGATGATCGAGGAAAGAAAGAAAAGAAG |
| 133 | 55576sgRNA-c | CATCCAGAGCGCCGGCAGTTTTAGAGCTAGAAATA |
| 134 | sgRNA-d | AAAAAAAGCACCGACTCGGTGCC |
| 135 | 55576donor-up-F | TCAAGTGTCATGATCTCGCGTG |
| 136 | 55576donor-up-R | tcatcttctgGGTTTGGATGAATGGATG |
| 137 | 55576donor-hygR-F | catccaaaccCAGAAGATGATATTGAAGGAG |
| 138 | 55576donor-hygR-R | cgcaacttgaGAAAGAAGGATTACCTCTAAAC |
| 139 | 55576donor-Down-F | tccttctttcTCAAGTTGCGGGGCGCGT |
| 140 | 55576donor-Down-R | AGGACTATTATCATCCTGGGGGA |
| 142 | 2140820sg RNA-b | GATGGGCGTCTTGAGCTCGAGGAAAGAAAGAAAAGAAG |
| 143 | 2140820sgRNA-c | GCTCAAGACGCCCATCACGTTTTAGAGCTAGAAATAGCA |
| 14 | 2140820donor-up-F | GTGCCGCTATCCAAGCATATTGC |
| 18 | 2140820donor-up-R | tcatcttctgTGGTTGTGGTGGTGGTGG |
| 21 | 2140820donor-hygR-F | accacaaccaCAGAAGATGATATTGAAGGAG |
| 22 | 2140820donor-hygR-R | gtcttcttctGAAAGAAGGATTACCTCTAAAC |
| 23 | 2140820donor-Down-F | tccttctttcAGAAGAAGACGTTCGAGGTC |
| 24 | 2140820donor-Down-R | TCCATGTGCGACGAGAGGGCGGC |

### 3. Determination of ethanol production capacity of M. thermophila transformants

Cas9 expression frame, sgRNA expression plasmid U6p-mtadh-sgRNA,U6p-mtaldh-sgRNA and donor DNA fragments donor-*mtadh* and donor-*mtaldh* were mixed in equal proportion and co-transformed into the protoplast cells of strain E5. Cas9, mediated by gRNA, identified the target site by pairing the target sequence with the DNA strand of the target gene on the host cell genome, and then homologous recombination occurred between the donor DNA fragment and the sequences on both sides of the target site. In order to achieve the purpose of editing the genome, the transformants were screened by adding hygromycin to the plate. The transformation method and verification method of introducing the target gene fragment into *M. thermophila* are the same as those described in Example 1.

The starting strain of this transformation is strain E5 (see example 6 for the construction process). The strain obtained by knocking out the ethanol dehydrogenase gene *Mtadh* (Mycth_55576) is named strain E14, and the strain obtained by knocking out the acetaldehyde dehydrogenase gene *Mtaldh* (Mycth_2140820) is named strain E15. Strains E14, E15 and E5 were inoculated into 75 g / L glucose and 75 g / L cellulose (Avicel) medium respectively (see example 1 for the formula). The transformed sporozoites were collected with distilled water and filtered with two layers of distilled mirror paper. The number of spores was calculated. The inoculation concentration was 2.5 × 10⁵ / mL, the volume of medium was 100mL / bottle, cultured at 45 °C for 7 days, and the shaker speed was 150 rpm.

The supernatant of the sample was centrifuged and the ethanol content was determined. The results are shown in Figure 11: when glucose was used as the carbon source, the ethanol yield of strain E14 and E15 were 18.84 g / L and 17.93 g / L respectively, which were higher than that of the starting strain E5 (15.96 g / L), 18.05% and 12.34% higher than that of E5 respectively. When fermenting with cellulose (Avicel) as carbon source for 7 days, the ethanol yield of strain E14 and E15 was 3.12 g / L and 3.59 g / L respectively, which was higher than that of starting strain E5 (2.568 g / L), which was 21.5% and 39.8% higher than that of E5, respectively. It shows that the knockout of endogenous ethanol dehydrogenase gene *Mtadh* and acetaldehyde dehydrogenase gene *Mtaldh* can reduce the metabolic consumption of ethanol by *M*. *thermophila,* and then improve the ethanol production of the strain under the conditions of glucose and cellulose (Avicel).

### Example 11

Firstly, the coding gene of phosphofructokinase2 (PFK2, Mycth_71484, Gene ID: 11510101) was knocked out in this example.

### 1. Construction of sgRNA expression frame

The protopacer (eg. The target site) of the target gene *pfk2* (Mycth_71484) was designed by the software sgRNACas9 tool. The sgRNA promoter, protopacer and scaffold were connected by fusion PCR to construct the sgRNA expression frame U6p-pfk2-sgRNA. The sequence is shown in SEQ ID No.167. The specific operations were as follows: firstly, the promoter and protopacer DNA fragments were amplified with primers sgRNA-a and 71484SgRNA-b; protopacer and scaffold fragments were amplified with primers 71484SgRNA-cand sgRNA-d, and then the full length of sgRNA expression frame was amplified with primers sgRNA-a and sgRNA-d. The PCR reaction system and reaction conditions are the same as those in example 1.

### 2. Construction of donor DNA vector

In the invention, the donor DNA fragment is respectively composed of the upstream homologous arm of the target gene, the PtrpC-bar fragment of the glyphosate resistance gene expression frame and the downstream homologous arm, which are connected together by fusion PCR, and finally constructed into the donor DNA fragment donor-pfk2. The nucleic acid sequence is shown in SEQ ID No.168. The construction process was as follows: firstly, the upstream homologous arm DNA was amplified with primers 71484up-Fand 71484up-R, the expression frame PtrpC-bar fragment of glufosinate resistance gene was amplified with primers 71484Bar-Fand 71484Bar-R, and the downstream homologous arm was amplified with 71484down-Fand 71484down-R. Then, the upstream homologous arm and PtrpC-bar fragment were used as templates at the same time. The fusion DNA fragment of the upstream homologous arm and the glyphosate resistance gene expression frame was amplified with primers 71484up-Fand 71484Bar-R, then the fusion DNA fragment and the downstream homologous arm were used as templates at the same time, and the full-length donor DNA fragment was amplified with primers 71484up-F and 71484down-R. Finally, the donor DNA sequence was proved to be correct by gene sequencing.

The primers used for vector construction in this example are as follows:

| | | |
|---|---|---|
| 183 | sgRNA-a | AGGATCGGTGGAGTGAAGTTCG |
| 184 | 71484SgRNA-b | ATACTGGTCTGCAATCTCCGAGGAAAGAAAGAAAAGAAGA |
| 185 | 71484SgRNA-c | AGATTGCAGACCAGTATCGTTTTAGAGCTAGAAATAGCAAGT |
| 186 | sgRNA-d | AAAAAAAGCACCGACTCGGTGCC |
| 187 | 71484up-F | tacacagtacacgaggacttctagaGGCTCGGGTCGGTTAATG |
| 188 | 71484up-R | ccttcaatatCTTTGCTGAGCGACAGCC |
| 189 | 71484Bar-F | ctcagcaaagATATTGAAGGAGCACTTTTTGGG |
| 190 | 71484Bar-R | gaggcagatgTCAGATCTCGGTGACGGG |
| 191 | 71484down-F | cgagatctgaCATCTGCCTCACGGGGTG |
| 192 | 71484down-R | aatatcatcttctgtcgaggaattcTCAATGGCGTAAAGTTCAACATCAG |
| 193 | Pfk2m-YZ-F | CATCATCGATACACTGGTTCTTGACAA |
| 194 | Pfk2m-YZ-R | CTCAGGGTCCTGACCCTGGTAATCC |

### 3. Construction of phosphofructokinase 2 mutant

In this example, the mutation sites of phosphofructokinase 2 (PFK2, Mycth_71484) are H233G, E306G and H371G. The inactivation information of phosphokinase can be selected based on the structural mutation of phosphokinase 2, which can only retain the activity of phosphokinase 2. The nucleotide and amino acid sequences after mutation are shown in SEQ ID No. 170 and SEQ ID No.171, respectively. The mutant *pfk2 gene* was amplified by fusion PCR with the genomic DNA of *M*. *thermophila* as the template. The specific operations are as follows: firstly, take genomic DNA as the template, and use 71484mut-a and 71484mut-b, 71484mut-c and 71484mut-d, 71484mut-e and 71484mut-f, 71484mut-g and 71484mut-h four pairs of primers to amplify the four fragments of the mutated *pfk2*gene, and then use the first two fragments as the template at the same time, and 71484mut-a and 71484mut-d as the primers to amplify the fusion fragments of the first two DNA fragments. Similarly, use 71484mut-e and 71484mut-h as the primers, the fusion fragments of the latter two DNA fragments were amplified by using the latter two DNA fragments as templates at the same time. Finally, using the above two fused DNA fragments as templates, the full-length mutant *pfk2* gene was amplified by primers 71484mut-a and 71484mut-h. Then, using the fused full-length mutant *pfk2* motif template, the DNA fragment used to connect to the vector was amplified with primers Pfk2m-Fand Pfk2m-R. the fragment was recombined into the linearized vector pAN52-TB-Intron digested by *BcuI* and *BamHI* by Gibson assembly of NEB. The promoter of the vector was Ptef promoter (nucleic acid sequence is shown in SEQ ID No. 2), and the recombinant expression plasmid pAN52-tef-pfk2 of the mutant *pfk2 gene* was obtained.

The primers used for vector construction in this example are as follows:

| **SEQ ID NO.** | **Primer** | **Sequence** (**5'-3'**) |
|---|---|---|
| 173 | 71484mut-a | ATGGCTCCAAAGATTAACGGCA |
| 174 | 71484mut-b | TCAGACTCGCCGCCGCGAGACAGC |
| 175 | 71484mut-c | GCTGTCTCGCGGCGGCGAGTCTGA |
| 176 | 71484mut-d | CAGCGTCTAGGCCATCCAGCGCCT |
| 177 | 71484mut-e | AGGCGCTGGATGGCCTAGACGCTG |
| 178 | 71484mut-f | ATGACGGCCTGGCCCGAGATGATG |
| 179 | 71484mut-g | CATCATCTCGGGCCAGGCCGTCAT |
| 180 | 71484mut-h | TCAAATGCCGCCTTCTGGGGTTG |
| 181 | Pfk2m-F | gccagtttcgttcttcagaactagtATGGCTCCAAAGATTAACGG |
| 182 | Pfk2m-R | gatttcagtaacgttaagtggatccTCAAATGCCGCCTTCTGG |

### 4. Transformation of M. thermophila

The DNA (donor-pfk2, Cas9 expression frame and U6p-pfk2-sgRNA) used for knockout was co transformed into wild-type strain ATCC42464 of *M*. *thermophila,* and the recombinant expression plasmid pAN52-tef-pfk2m linearized by HindIII was transformed into the wild-type strain ATCC42464 and the wild-type strain with knockout pfk2 gene, respectively. The transformation method and genome extraction method were the same as those in embodiment 1.

### 5. PCR validation of M. thermophila transformants

For the transformant with knockout *pfk2* gene, the extracted genomic DNA of the transformants is used as the template, and the knockout transformantsare verified by PCR with primers 71484up-F and 71484down-R. PCR products were subjected to 1% agarose gel electrophoresis (110V voltage, 30 min). The gene amplification bands were observed under the gel imaging system. It was shown that under the guidance of primers 71484up-F and 71484down-R, the transformant obtained a target band of ∼1380bp by PCR amplification, The wild-type strain obtained the target band of ∼ 2000bp by PCR amplification (the length of donor DNA amplified by this primer was 1380bp, and the length of DNA amplified by wild-type genome was about 2000bp). The results showed that the phosphofructokinase 2 gene (*pfk2,* Mycth_71484) had been knocked out from the genome of *M*. *thermophila,* and the knockout strain was named E17 strain.

Based on the knockout of *pfk2* gene (strain E17), the transformant of the mutant phosphofructokinase 2 gene was further overexpressed. Taking the extracted genomic DNA of the transformant as a template, the transformant of the overexpressed mutant *pfk2* was verified by PCR with primers pfk2m-yz-F and pfk2m-yz-R. The PCR products were subjected to 1% agarose gel electrophoresis (110V, 30 min). The gene amplification bands were observed under the gel imaging system. It was shown that the transformant obtained ~592bp of the target band by PCR amplification, while the starting strain E17 did not amplify any band by PCR amplification (only the strain with overexpression mutation *pfk2* gene could amplify ~592bp of the target band under the above primers), these results showed that the fructose kinase gene had been transferred into the genome of *M*. *thermophila,* and the strain was named strain E18.

In order to verify the effect of direct overexpression of the mutated phosphofructokinase 2 gene, the invention also overexpressed the mutated *pfk2* gene in the wild-type strain, and PCR verified the transformant with primers pfk2m-yz-F and pfk2m-yz-R using the genomic DNA of the obtained transformant as the template. The PCR products were subjected to 1% agarose gel electrophoresis (110V, 30 min). The gene amplification bands were observed under the gel imaging system. It showed that the transformant obtained ~592bp of the target band by PCR amplification, while the original strain WT did not amplify any bands by PCR amplification (only the strain with overexpression mutation *pfk2 gene* could amplify ~592bp of the target band under the above primers), the results showed that the fructose kinase gene had been transferred into the genome of *M*. *thermophila,* and the strain was named strain E19.

### 6. Determination of ethanol production capacity of transformant

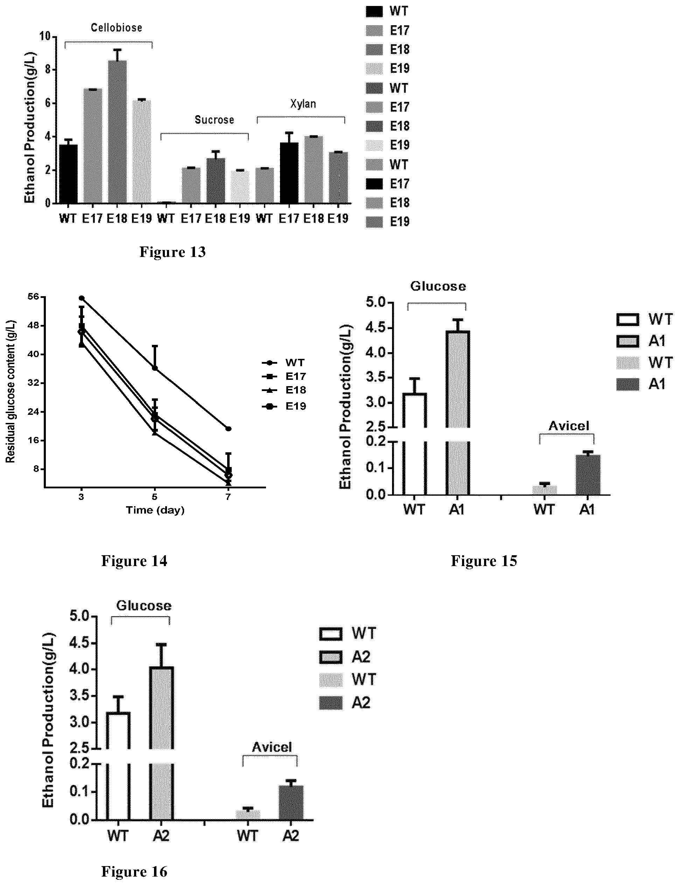

Wild-type strain ATCC42464 (WT), strain E17 with wild-type knockout *pfk2* gene, strain E18 with E17 overexpressing mutated *pfk2* gene and strain E19 with wild-type overexpressing mutated *pfk2 gene* were inoculated into 75g / L glucose, 75g / L cellulose, 75g / L cellobiose, 75g / L xylan and 75g / L sucrose medium, respectively. After 7 days of culture, the samples were centrifuged, the supernatants were taken, and the contents of ethanol in the culture medium were measured. The ethanol yields are shown in Figure 12 (glucose and cellulose conditions) and Figure 13 (cellobiose, sucrose and xylan conditions): when glucose is used as carbon source, the ethanol yield of strain E17 is 4.724g/L, which is 49.1% higher than that of the original strain WT (3.168g / L); the ethanol yield of strain E18 was 7.371g/L, which was 56.03% higher than that of strain E17; the ethanol yield of strain E19 was 5.839 g / L, which was 84.3% higher than that of the original strain WT (3.168g / L). When fermenting with cellulose as carbon source for 7 days, the ethanol yield of strain E17 was 0.153g/L, which was significantly higher than that of the original strain WT (0.0305g / L), increased by 400%; the ethanol yield of strain E18 was 0.259g/L, which was 69.5% higher than that of strain E17; the ethanol yield of strain E19 was 0.168g/L, which was 449% higher than that of the original strain WT (0.0305g / L). When cellobiose was used as carbon source, the ethanol yield of strain E17 was 6.826g/L, which was 97.6% higher than that of the original strain WT (3.454g / L); the ethanol yield of strain E18 was 8.504g/L, which was 24.6% higher than that of strain E17; the ethanol yield of strain E19 was 6.121g/L, which was 77.2% higher than that of the original strain WT (3.454g / L). When sucrose was used as carbon source, the ethanol yield of strain E17 was 2.0875g/L, which was 51.8 times higher than that of the original strain WT (0.0395g / L). The ethanol yield of strain E18 was 2.633g/L, which was 26.1 % higher than that of strain E17; the ethanol yield of strain E19 was 1.924g/L, which was 47.7 times higher than that of the original strain WT (0.0395g / L). When xylan was used as carbon source, the ethanol yield of strain E17 was 3.581 g/L, which was 72.4% higher than that of the original strain WT (2.077g / L). The ethanol yield of strain E18 was 3.962g/L, which was 10.6% higher than that of strain E17; the ethanol yield of strain E19 was 3.002g/L, which was 44.5% higher than that of the original strain WT (2.077g / L). It shows that knockout of *pfk2* gene and overexpression of mutatedpfk2 gene can effectively improve the ethanol production of *M*. *thermophila* under the conditions of glucose, cellulose, cellobiose, sucrose and xylan, that is, it is universal under the conditions of different carbon sources as substrates.

In addition, the glucose metabolism rates of the above four strains were also compared. The four strains were inoculated in the medium with 75g / L glucose as carbon source at the same time (the formula is the same as that in example 4). Samples were taken at the same time on the 3rd, 5th and 7th days after inoculation to determine the residual glucose content in the medium. The results are shown in Figure 14: at the above time points, the residual glucose content in the medium of strain E18 is less than that of strain E17; the content of residual glucose in the medium of strain E17 was less than that of wild-type strain WT; the content of residual glucose in the medium of strain E19 was also less than that of wild-type strain WT. These results showed that strain E18 metabolized glucose faster than strain E17, strain E17 metabolized glucose faster than strain WT, and strain E19 metabolized glucose faster than strain WT. It shows that knockout of *pfk2 gene* and overexpression of mutated*pfk2* gene can accelerate the metabolic rate of glucose.

### Example 12

### 1. Construction of adhE expression vector (pAN52-adhE)

Taking the acetaldehyde dehydrogenase *adhE* (amino acid sequence as shown in SEQ ID No.195) of *Thermoanaerobacterium saccharolyticum* as the template, the gene was synthesized by gene synthesis method through codon optimization, and the DNA sequence is shown in SEQ ID No.196. Using synthetic *adhE* gene as template, *adhE* gene was amplified by primers adhE-F and adhE-R; The mitochondrial localization signal sequence (nucleotide sequence as shown in SEQ ID No.197 and amino acid sequence as shown in SEQ ID No.198) of cis aconitase (Mycth_2309261) was amplified with primers 9261MTS-Fand 9261MTS-R using the genome of *M*. *thermophila* (ATCC42464) as the template, and then the fusion fragments of the two DNA were amplified with primers 9261MTS-Fand adhE-R. Then, the fusion fragment was recombined into the linearized vector pAN52-TB-Intron (Liu Q, Li J, Ying S, Wang J, Sun W, Tian C, Feng M. 2014. Unveiling equal importance of two 14-3-3 proteins for morphogenesis, conidiation, stress tolerance and virulence of an insect pathogen. Environ Microbiol. doi: 10.1111/1462-2920.12634) by Gibson assembly of NEB, The recombinant expression plasmid pAN52-adhE of *adhE* gene was obtained.

The PCR reaction system and reaction conditions were as Example 1. The primers used for vector construction are as follows:

| **SEQ ID NO.** | **Primer** | **Sequence** (**5'-3'**) |
|---|---|---|
| 141 | adhE-F | ccggcgcatgATGGCCACCACCAAGACC |
| 158 | adhE-R | gatttcagtaacgttaagtggatccTCAGGCGCCGTAGGCCTT |
| 161 | 9261 MTS-F | ccacatcacagaaatcaaaactagtATGTTGGCTTCTCGTCAGC |
| 169 | 9261 MTS-R | tcttggtggtggccatCATGCGCCGGAGACCAAG |
| 172 | adhE-YZ-F | CTGTCGGCAGCGCGAGCCTTGGTCTCCG |
| 199 | adhE-YZ-R | GAAGGCGTGTTGGTGTTGTAGATGTCG |

### 2. Construction of adh1overexpression vector (pAN52-adh1)

Using the genomic DNA of *S. cerevisiae* S288C (taxID: 559292) as the template, *adh1*gene was amplified with primers adhl-Fand adh1-R (Gene ID: 854068). The mitochondrial localization signal sequence (nucleotide sequence as shown in SEQ ID No.200) of mitochondrial malate dehydrogenase (Mycth_2305575) was amplified with primers 5575MTS-Fand 5575MTS-R using the genome of *M. thermophila* (ATCC42464) as a template, and then the fusion fragments of the two DNA were amplified with primers 5575MTS-Fand adh1-R. Then, the fusion fragment was recombined into the linearized vector pAN52-TB-Intron (Environ Microbiol. 015.17(4):1444-62) digested by *BcuI* and *BamHI* by Gibson assembly of NEB, and the recombinant expression plasmid pAN52-adh1 of *adh1*gene was obtained.

The PCR reaction system and reaction conditions were as Example 1. The primers used for vector construction are as follows:

| **SEQ ID NO.** | **Primer** | **Sequence** (**5'-3'**) |
|---|---|---|
| 47 | 5575MTS-F | ccacatcacagaaatcaaaactagtATGTTCCTGGGCAGCCGC |
| 48 | 5575MTS-R | tttctgggatagacatACCGAGAACGGCGACCTTG |
| 49 | adh1-F | cgttctcggtATGTCTATCCCAGAAACTC |
| 50 | adh1-R | tgttaataacccacgcgtgggcggatccTTATTTAGAAGTGTCAACAACG |
| 53 | adh1 -YZ-F | CAGCCGCATCCAGACCCGCGCCTTCTC |
| 72 | adh1 -YZ-R | TTATTTAGAAGTGTCAACAACGTATCTACCA |

### 3. Construction of pdcloverexpression vector (pAN52-pdc1)

Using the genomic DNA of *S. cerevisiae* S288C (taxID: 559292) as the template, the *pdc1* gene (Gene ID: 850733, nucleotide sequence shown in SEQ ID No.214 and amino acid sequence shown in SEQ ID No.215) was amplified with primers pdcl-Fand pdc1-R. The mitochondrial localization signal sequence of cis- aconitase (Mycth_2309261) was amplified by primers MTS-pdc1-Fand MTS-pdc1-R with the genome of *M*. *thermophila* (ATCC42464) as the template (nucleotide sequence is shown in SEQ ID No.197). Then, using the two amplified DNA fragments as the template, the fusion fragments of the two DNA were amplified by primers MTS-pdc1-Fand pdc1-R. Then, the fusion fragment was recombined into the linearized vector pAN52-TB-Intron (Environ Microbiol. 015.17(4):1444-62) digested by *BcuI* and *BamHI* by Gibson assembly of NEB, and the recombinant expression plasmid pAN52-pdc1 of *pdc1*gene was obtained.

The PCR reaction and reaction conditions were as Example 1. The primers used for vector construction are as follows:

| **SEQ ID NO.** | **Primer** | **Sequence** (**5'-3'**) |
|---|---|---|
| 73 | pdc1-F | ccggcgcatgATGTCTGAAATTACTTTGGG |
| 74 | pdc1-R | gatttcagtaacgttaagtggatccTTATTGCTTAGCGTTGGTAG |
| 75 | MTS-pdc1-F | ccacatcacagaaatcaaaactagtATGTTGGCTTCTCGTCAGC |
| 108 | MTS-pdc1-R | tttcagacatCATGCGCCGGAGACCAAG |
| 109 | pdc1- YZ-F | ATGTCTGAAATTACTTTGGGTAAA |

The transformation method and verification method of subsequent target gene overexpression vector into *M. thermophila* are the same as those described in Example 1.

### 4. PCR validation of M. thermophila transformants

The transformants obtained by transforming pAN52-adhE and pAN52-adh1 vectors at the same time, took the extracted transformant genomic DNA as template, adhE-YZ-F and adhE-YZ-R as primers, and verified whether *adhE* gene was integrated into the genome by PCR amplification; adh1-YZ-Fand adh1-YZ-R were used as primers to verify whether *Adh1* gene was integrated into the genome. The obtained strain transferred into two expression vectors, pAN52-adhE and pAN52-adh1, was named strain A1.

Similarly, for the transformants obtained by simultaneous transformation of pAN52-pdc1 and pAN52-adh1 vectors, the extracted genomic DNA was used as template, pdc1-YZ-Fand pdc1-YZ-R were used as primers, and the integration of *pdc1*gene into the genome was verified by PCR; adh1-YZ-F and adh1-YZ-R were used as primers to verify whether *adh1*gene was integrated into the genome. The obtained strains transferred into both pAN52-pdc1 and pAN52-adh1 vectors were named as strain A2.

### 5. Determination of ethanol production capacity of transformants of M. thermophila

Strains A1 and A2 and wild-type ATCC42464 of *M*. *thermophila* verified by the above PCR were inoculated into 75 g / L glucose and 75 g / L cellulose (Avicel) medium respectively (see example 1 for the formula). The transformed sporozoites collected with distilled water were filtered by two layers of distilled mirror paper, and the number of spores was calculated. The inoculation amount was 2.5 × 105 spores / mL, the medium volume was 100mL / bottle, cultured under light, 45 °C, and the rotating speed of the shaker was 150rpm. The ethanol content was measured on the 7th day.

The ethanol content of the treated samples was determined by high performance liquid chromatography, in which the detector was differential detector, 5 mM H₂SO₄ was mobile phase, and the flow rate was 0.5mL/min. The ethanol yield of A1 transformant transferred into pAN52-adhE and pAN52-adh1 vectors is shown in Figure 15. When glucose is used as carbon source for fermentation for 7 days, the ethanol yield of strain A1 is 4.42g/l, which is 39.4% higher than that of wild-type strain *(M. thermophila* ATCC42464, 3.17g/l). When fermenting with cellulose (avivel) as carbon source for 7 days, the ethanol yield of strain A1 was 145mg / L, which was 3.75 times higher than that of wild-type strain *(M. thermophila* ATCC42464, 30.5mg / L). The results showed that the expression of *adhE* and *adh1* genes in mitochondria could improve the ethanol production of *M*. *thermophila* when glucose and cellulose (Avicel) were used as carbon sources.

The ethanol yield of A2 transformant transferred into pAN52-pdc1 and pAN52-adh1 vectors is shown in Figure 16. When glucose is used as carbon source for fermentation for 7 days, the ethanol yield of A2 strain is 4.03g/L, which is 27% higher than that of wild-type strain *(M. thermophila* ATCC42464, 3.17g/L). When fermenting with cellulose (avivel) as carbon source for 7 days, the ethanol yield of strain A2 was 118mg / L, which was 2.87 times higher than that of wild-type strain *(M. thermophila* ATCC42464, 30.5mg / L). The results showed that the expression of *pdc1* and *adh1* genes in mitochondria could improve the ethanol production of *M. thermophila* with glucose and cellulose (Avicel) as carbon sources.

## Claims

1. A construction method of genetic engineering fungi of filamentous fungi, which is **characterized in that** the filamentous fungi overexpress the positive regulation genes of ethanol synthesis, and / or down regulate the expression of the negative regulation genes of endogenous ethanol synthesis to obtain genetic engineering fungi, compared with the original strain, the ethanol synthesis ability of the genetically engineered strainsare improved.

2. The construction method as claim 1, which is **characterized in that** the filamentous fungiare cellulose degrading filamentous fungi; Preferably, the filamentous fungi are selected from *Neurospora, Aspergillus, Trichoderma, Penicillium, Myceliophthora, Sporotrichum, Fusarium, Rhizopus Mucor* and *Paecilomyces*; more preferably, the Myceliophthora fungi are selected from *Myceliophthora thermophila* and *M. heterothalica.*

3. The construction method as claim 1, which is **characterized in that** the positive regulatory genes of ethanol synthesis are selected from the ethanol synthesis genes with strengthening the ethanol synthesis pathway, improving the sugar transport capacity, accelerating the glycolysis rate, improving cytoplasmic reducing power and containing mitochondrial localization signal sequence. The negative regulation genes of ethanol synthesis are selected from the genes in the branch pathway of ethanol synthesis, the shuttle pathway of cytoplasmic reducing force to mitochondria, the endogenous ethanol metabolism pathway and the electron transfer chain (respiratory chain).

4. The construction method as claim 1, which is **characterized in that** the genetically engineered fungi with the shuttle of cytoplasmic reducing force to mitochondria is reduced or blocked, and / or the intensity of respiratory chain is weakened, and / or the by-product pathway of ethanol synthesis is weakened, and / or the ethanol synthesis pathway is strengthened, and / or the transport of sugar molecules is strengthened, and / or the glycolysis rate is accelerated.

5. The construction method as claim 1, which is **characterized in that** the overexpression of the positive regulation genes of ethanol synthesis are realized through the introduction of exogenous and / or endogenous positive regulation genes of ethanol synthesis, wherein the positive regulation genes of ethanol synthesis are selected from one or more of ethanol dehydrogenase, glucose transporter, cellobiose transporter and pyruvate decarboxylase.

6. The construction method as claim 1, which is **characterized in that** the introduction is to transfer the expression vector carrying the positive regulation genes of exogenous or endogenous ethanol synthesis into the filamentous fungi, and the preferred promoters are tef, gpdA, trpC, cbh1 and glaA promoters. The imported positive regulatory genes of exogenous ethanol synthesis come from *Saccharomyces cerevisiae* and / or *Zymomonas mobilis.*

7. The construction method as claim 1, which is **characterized in that** one of the ethanol dehydrogenase coding gene*Scadh1*, pyruvate decarboxylase gene *Scpdc1*, glucose transporter coding gene *glt-1,* cellobiose transporter coding gene *cdt-1* / *cdt-2* or a combination thereof is overexpressed in the filamentous fungus. Preferably, the overexpression of the positive regulatory genes of ethanol synthesis in the filamentous fungi are selected from ethanol dehydrogenase and pyruvate decarboxylase. The preferred ethanol dehydrogenases are ethanol dehydrogenase ScADH1 from *Saccharomyces cerevisiae* and ZmADH1 from *Zymomonas mobilis,* and the preferred pyruvate decarboxylases are pyruvate decarboxylases ScPDC1 and ScPDC5 from *Saccharomyces cerevisiae* and ZmPDC from *Zymomonas mobilis.*

8. The construction method as claim 1, which is **characterized in that** the down-regulated gene expression is to inactivate or reduce the expression or decrease the activity of the negative regulation genes of endogenous ethanol synthesis through gene knockout or small RNA interference or gene editing or replacement of promoter or gene mutation. Preferably, the gene editing is a genome editing method based on CRISPR / Cas9.

9. The construction method as claim 8, which is **characterized in that** the negative regulation genes of endogenous ethanol synthesis in the strain are selected from one or more combination genes of lactate dehydrogenase, 1-phosphate mannitol dehydrogenase, cytoplasmic malate dehydrogenase, glycerol-3-phosphate dehydrogenase, external NADH dehydrogenase, ethanol dehydrogenase, acetaldehyde dehydrogenase and phosphofructokinase 2 genes, and the expression level is reduced or lost, and / or reduce the expression of cytochrome C oxidase, a negative regulation gene of endogenous ethanol synthesis in the strain. Preferably, the endogenous ethanol synthesis negative regulatory genes are selected from the cytoplasmic malate dehydrogenase, glycerol-3-phosphate dehydrogenase. Alternatively, the endogenous ethanol synthesis negative regulatory genes are cytochrome C oxidase coding gene and / or phosphofructokinase 2 gene.

10. The construction method as claim 1, which is **characterized in that** the filamentous fungi overexpress the cellobiose transporter coding genes *cdt-1* and / or *cdt-2* of *Neurospora crassa*, and down regulate the expression of lactate dehydrogenase genes *Idh-1* and / or *Idh-2.*

11. The construction method as claim 1, which is **characterized in that** the ethanol dehydrogenase of *S. cerevisiae* is overexpressed in the filamentous fungus and the expression of external NADH dehydrogenase is down regulated, wherein one or both of the external NADH dehydrogenase genes *nde1* and *nde2* are down regulated at the same time.

12. The construction method as claim 1, which is **characterized in that** the ethanol dehydrogenase of *S. cerevisiae* is overexpressed in the filamentous fungus and the expression of cytochrome C oxidase gene is down regulated.

13. The construction method as claim 1, which is **characterized in that** the ethanol dehydrogenase of *S. cerevisiae* is overexpressed in the filamentous fungus, the expression of cytoplasmic malate dehydrogenase Mdh is down regulated / knocked out, and the expression of glycerol-3-phosphate dehydrogenase gene *gpd* is optionally further down regulated / knocked out.

14. The construction method as claim 1, which is **characterized in that** the filamentous fungi overexpressing the ethanol dehydrogenaseScADH1is from *S. cerevisiae* and ZmADHI is from *Zymomonas mobilis*, overexpression of pyruvate decarboxylase is the pyruvate decarboxylase ScPDC1 and ScPDC5 from *Saccharomyces cerevisiae* and ZmPDC from *Zymomonas mobilis.* Optionally, further overexpress glucose transporter coding gene *glt-1,* and down-regulate cytoplasmic malate dehydrogenase Mdh, down regulate / knockout ethanol dehydrogenase gene *Mtadh,* and down regulate / knockout acetaldehyde dehydrogenase gene *Mtadh.*

15. The construction method as claim 1, which is **characterized in that** the filamentous fungi overexpress the ethanol dehydrogenase and pyruvate decarboxylase gene *pdc1*from *Saccharomyces cerevisiae* or *Zymomonas mobilis,* optionally further overexpress the glucose transporter coding gene *glt-1,* and further regulate the lactate dehydrogenase gene and 1-phosphate mannitol dehydrogenase.

16. The construction method as claim 1, which is **characterized in that** the filamentous fungi overexpress *Saccharomyces cerevisiae* ethanol dehydrogenase ScADH1, and / or *Zymomonas mobilis* derived ZmADH1, and / or overexpress *Saccharomyces cerevisiae* derived pyruvate decarboxylases ScPDC1, ScPDC5, and / or *Zymomonas mobilis* derived pyruvate decarboxylase ZmPDC.

17. The construction method as claim 9, which is **characterized in that** the endogenous phosphofructokinase 2 gene is knocked out in the filamentous fungus, and the mutant phosphofructokinase 2 gene is preferably further expressed, wherein the mutant phosphofructokinase 2 refers to the loss or reduction of phosphatase activity due to the retention of kinase activity after mutation. The phosphofructose kinase 2 refers to the gene encoding the synthesis and degradation of fructose-2,6-diphosphate or its homologous gene. The mutated phosphofructose kinase 2 refers to the corresponding amino acid sequence as gene ID: 11510101 which has one or two or three mutation sites in H233, E306 and H371 to reduce or lose phosphatase activity.

18. The construction method as claim 1, which is **characterized in that** overexpression of ethanol synthesis gene containing mitochondrial localization signal sequence in the filamentous fungus for ethanol synthesis is overexpression of acetaldehyde dehydrogenase and ethanol dehydrogenase genes containing mitochondrial localization signal sequence, or overexpression of pyruvate decarboxylase and ethanol dehydrogenase genes containing mitochondrial localization signal sequence, or overexpression of acetaldehyde dehydrogenase, ethanol dehydrogenase and pyruvate decarboxylase genes containing mitochondrial localization signal sequences.

19. The construction method as claim 18, which is **characterized in that** the pyruvate decarboxylase and ethanol dehydrogenase are derived from *S. cerevisiae,* and / or *Zymomonas mobilis*; The acetaldehyde dehydrogenase is derived from *Thermoanaerobacterium saccharolyticum.*

20. The construction method as claim 18, which is **characterized in that** the mitochondrial localization signal sequence refers to the N-terminal amino acid sequence used to guide the transmembrane transfer of protein to the mitochondria, preferably from the localization signal sequence of mitochondrial matrix protein of *M*. *thermophila.*

21. The usage of genetically engineered fungi with improved ethanol production capacity obtained by the construction method according to any one of claims 1 to 20.

22. The usage of genetically engineered fungi in the production of ethanol according to claim 21.

23. The usage as claim 22, which is **characterized in that** it adopts monosaccharide or / and glycan, or a substance containing monosaccharide or / and glycan as the substrate. Preferably, the monosaccharide is glucose, xylose, arabinose or a combination thereof; the glycan includes cellobiose, xylobiose, sucrose, maltose, xylooligosaccharide, cellooligosaccharide, cellulose, crystalline cellulose, hemicellulose, starch, plant biomass or a combination thereof. More preferably, the plant biomass is selected from crop straw, forestry waste, energy plants or some or all decomposition products thereof; further preferably, the crop straw is selected from corn straw, wheat straw, rice straw, sorghum straw, soybean straw, cotton straw, bagasse and corncob. The forestry waste is selected from branches and leaves and sawdust; the energy plant is selected from sweet sorghum, switchgrass, miscanthus, reed or a combination thereof.

24. A method for producing ethanol, which is **characterized in that** the genetically engineered fungi obtained by the construction method according to any one of claims 1 to 20 are cultured in a culture medium containing monosaccharide or / and glycan, and ethanol is collected from the culture.

25. The method for producing ethanol as claim 24, which is **characterized in that** the monosaccharide is glucose, xylose, arabinose or a combination thereof; the glycan includes cellobiose, xylobiose, sucrose, maltose, xylooligosaccharide, cellooligosaccharide, cellulose, crystalline cellulose, hemicellulose, starch, plant biomass or a combination thereof. Preferably, the plant biomass is selected from crop straw, forestry waste, energy plants or some or all of their decomposition products, more preferably, the crop straw is selected from corn straw, wheat straw, rice straw, sorghum straw, soybean straw, cotton straw, bagasse and corncob. The forestry waste is selected from branches and leaves and sawdust; the energy plant is selected from sweet sorghum, switchgrass, miscanthus, reed or a combination thereof.

26. The method for producing ethanol according to any one of claims 24 to 25, which is **characterized in that** the filamentous fungus is Myceliophthora or Trichoderma; preferably, the Myceliophthora is selected from the group consisting of *M*. *thermophila* and *M*. *heterothallica.* It is most preferred that the *Myceliophthora* fungus is selected from *M*. *thermophila.* Further, it is preferred that the fermentation temperature is 40-60 °C, preferably 45-52 °C, more preferably 48-50 °C.


Sequence
<110>Tianjin Institute of industrial biotechnology, Chinese Academy of Sciences
<120>A recombinant filamentous fungus for producing ethanol and its construction and Application
<160>200
<170> PatentIn Version 3.1

<210> 1
<211> 1439
<212> DNA
<213> Myceliophthora thermophila
<400> 1
ccacatcatg taaacagagg ctcagaagct cctgccacac tgggagggtt tcgaagtctg 60
acgactgcca atggacccca gccatcgcga gcacacagca gttcgcacgc tcccattggg 120
ttcctcatca cgcagtcgct ctccccgcca accagcgcca ggtccgggaa cagcggcgca 180
aatgcgtatt tgagggcgcc tcgctcgagc aacctgtgcc tgaccttctc ctcctccttc 240
tgcaccttgc atctcgtcgc gtccactcgc aggcaaccac acatcctcct cctctcccaa 300
aacccccccg ctttttcttt cccttgttgg aattcgattg aaaaagaaga cgggtccgtc 360
tagagaccgc cttctcacct ttctctcgac ttctttctag gaaaagaagc aagagtcatt 420
cttcttgtcc accttctggt tcacggaagg tcgaggagaa gattgcctct gcccccaaag 480
tcgccaacct ggactttgaa gcacgtgttc cggtcccttt cagtgtcttc ccgtcctcgt 540
acagggagtc cgagaccgcc acccaaaccc actcccacga agaggttgag atcaagctcc 600
cccagctcgc cggacgggaa ggtcaacact cttcattcca agcccaagca catcttcctc 660
ccagcggaga gggtcgcttc agagaagaag aggtccgcat cactcgtcaa gaggaacatc 720
accgccgtcc cggcatccgt gaagagttcg ttcaccgcga ggagcgtcac cggtaagttt 780
agtttttgtt ttgattcacc acccattgtc ttccccgcct ttttcttttt cttcccttgc 840
tctcttgccc ctgtctagtg tagggcattg ccaaggccat cttcacacac acacaccccc 900
ccccccaccc tcagctgggg gggggggggt ggcctgggtt gaccaaggga cggtgaagac 960
tactactact tgagccactc aaacccatgc atgacacagg gttttccttt ttcttttctc 1020
ttttccttta actaaccaac cactccaaca ttagccctca gtcaacctac tccgagtctc 1080
gcatcgagtt cgatactgag caccgcactc acaactccgt cattgacgtt gctgagagcg 1140
agtatcgtgc ccgtgtccag cccaactacc gcaaggaagc ttccgtagtc ggtaccaccg 1200
tcgacggatc ccgcttcagc cacagccgca aggccagcag caccacctcc acccacaccg 1260
acgagtacac cgtcgatccc cctagccacc gccccgtcta caagaaggag tcggttgaag 1320
tcgccggtac cactgttgac ccccctgctc ctcgttcgac ctaccacgag caggtgaaca 1380
ttgttgaaga gaccgttgac gctcaccgtt acgctcctca acacaacaac aacaacaag 1439

<210> 2
<211> 1216
<212> DNA
<213> Myceliophthora thermophila
<400> 2
catgtacctt gacgtcctcc gaggttcgac atcagggttc gtcataggga gtgaaacacc 60
cgccatgatt ccgtagccgc gcgcgaagat acgaagcaga tatttcacgg acatggcgga 120
gatacttgtt tcccgtacta aggtagtcat gtcggagaca tctgaacgac agagctggcc 180
aagagaaccg accagttgcc ccaggacgat ctagacaaaa aaaaagagag atgagtgggc 240
cacttttgcc acaacatcga cggccctgcg accgccccca ggcaaacaaa caaaccgccg 300
aacaataata cttttgtcat tttaggagga gcgttgtatg gataaaaaca acatctcgtt 360
gctgcagaat gtggacttca aacttgcaga aaatgggagg cggatttgca tgatcggagg 420
gtagttgact cacgccgcag gctgcaaatc cgtcctccat tattccatga acaacttcgt 480
aaggttgggc tgagcgccaa tgcctaacgg accgggggcc acagcgcaac gtcccactta 540
aaggccagcg tgacatgcca gttccatacc aagtagtggc accagaggcg gccaatgctc 600
agtaagggca gggagggagg ctcaaacgat tggcaaaaag aggggcttgc cagttcagtt 660
ccctgtgcga gcgcgagagg ggcagtttca aatctggagg ggtgtgttgc gctggtctga 720
agagaaagag aagactgtac ttaataattg ttcaaagagt ccatcatcgc gttgcggact 780
cctctagctg tatttagagc cctatcatta cttgtcgggt gcgaatcaaa ataccgggat 840
gcagccctct ggcgatttgc atgcggttgt ggaggaagtg aagcctgaat cgcggggctg 900
ggcggcaaag cacgacgtga aattcctggc gaaattcgag ggcttgcccc accgtggttg 960
aagtttttgt gctgcgtaac cccaccaacc cgccttgccc ctcccgcctg cccataaaaa 1020
cttcgacccc tcctcaaatc ttcttcgatt cttcctcttc acttccttcg tcggcatacc 1080
tgattcaagc aatcacctgc cactttcaag tgcgtatacc atcatcgata cactggttct 1140
tgacaagtac atcgtctcta actttccttt ttgcagtttt cattaagcgc aagtcgccag 1200

tttcgttctt cagaac                                                    1216

<210>  3
<211>  44
<212>  DNA
<213>  Primer
<400>  3
tctgcagatc tttaattaac tcgagccaca tcatgtaaac agag                      44

<210>  4
<211>  28
<212>  DNA
<213>  Primer
<400>  4
ggatagacat cttgttgttg ttgttgtg                                        28

<210>  5
<211>  29
<212>  DNA
<213>  Primer
<400>  5
caacaacaag atgtctatcc cagaaactc                                       29

<210>  6
<211>  47
<212>  DNA
<213>  Primer
<400>  6
gatttcagta acgttaagtg gatccttatt tagaagtgtc aacaacg                   47

<210>  7
<211>  359
<212>  DNA
<213>  Myceliophthora thermophila
<400>  7
acgaacctct ctgaaggagg ttctgagaca cgcgcgattc ttctgtatat agttttattt     60
ttcactctgg agtgcttcgc tccaccagta cataaacctt ttttttcacg taacaaaatg      120
gcttcttttc agaccatgtg aaccatcttg atgccttgac ctcttcagtt ctcactttaa     180
cgtagttcgc gtttgtctgt atgtcccagt tgcatgtagt tgagataaat acccctggaa    240
gtgggtctgg gcctttgtgg gacggagccc tctttctgtg gtctggagag cccgctctct     300
accgcctacc ttcttaccac agtacactac tcacacattg ctgaactgac ccatcatac      359

<210>  8
<211>  3311
<212>  DNA
<213>  Myceliophthora thermophila
<400>  8
tcaagtgtca tgatctcgcg tgtcatccac tttcatcagc gttgacctgg cccgcactcc     60
aggaacccca ccctatctgt gcctacataa cataagtcgt ccccaaacct gtcggcgtta     120
agactccact attaccagaa ctgcccaaaa ttcttcatca actagccccg aaaacctcgg     180
attaccgagt cgtggcattc ttctaggagt gtgagataag gagaattcaa gtcctcggaa     240
taattatgac tgcgtgaata tttgttgtcg aagaactatc gtctccacca ttgttgaaga     300
cgacccacat tccaccgctt tccattgtca acaatgacgt cttgtccctg accttaagcg     360
gcttattcga gaggggccca tagctgccga gcaggtagaa gcgggacaat gaaggtatat     420
atttctgaag agagacgtct gtaacacgcg agcaccaaag actctcatca acacatcaca     480
acttctgaac ttgttccacg ttgtcatcct gtccacttcc ctctaatagt tcgcaccacg     540
ccagcaatta taggatacaa acacgcttac cacagtacat ccattcatcc aaacccagaa     600
gatgatattg aaggagcatt tttgggcttg gctggagcta gtggaggtca acacatcaat     660
gctattttgg tttagtcgtc caggcggatc acaaaatttg tgtcgtttga caagatggtt     720
catttaggca actggtcaga tcagcccact tgtaagcagt agcggcggcg ctcgaagtgt     780
gactcttatt agcagacagg aacgaggaca ttattatcat ctgctgcttg gtgcacgata     840
acttgtgcgt ttgtcaagca aggtaagtga acgacccggt cataccttct taagttcgcc    900
cttcctccct ttatttcaga ttcaatctga cttacctatt ctaccgaagc atcgatatga     960

aaaagcctga actcaccgcg acgtctgtcg agaagtttct gatcgaaaag ttcgacagcg 1020
tctccgacct gatgcagctc tcggagggcg aagaatctcg tgctttcagc ttcgatgtag 1080
gagggcgtgg atatgtcctg cgggtaaata gctgcgccga tggtttctac aaagatcgtt 1140
atgtttatcg gcactttgca tcggccgcgc tcccgattcc ggaagtgctt gacattgggg 1200
aattcagcga gagcctgacc tattgcatct cccgccgtgc acagggtgtc acgttgcaag 1260
acctgcctga aaccgaactg cccgctgttc tgcagccggt cgcggaggcc atggatgcga 1320
tcgctgcggc cgatcttagc cagacgagcg ggttcggccc attcggaccg caaggaatcg 1380
gtcaatacac tacatggcgt gatttcatat gcgcgattgc tgatccccat gtgtatcact 1440
ggcaaactgt gatggacgac accgtcagtg cgtccgtcgc gcaggctctc gatgagctga 1500
tgctttgggc cgaggactgc cccgaagtcc ggcacctcgt gcacgcggat ttcggctcca 1560
acaatgtcct gacggacaat ggccgcataa cagcggtcat tgactggagc gaggcgatgt 1620
tcggggattc ccaatacgag gtcgccaaca tcttcttctg gaggccgtgg ttggcttgta 1680
tggagcagca gacgcgctac ttcgagcgga ggcatccgga gcttgcagga tcgccgcggc 1740
tccgggcgta tatgctccgc attggtcttg accaactcta tcagagcttg gttgacggca 1800
atttcgatga tgcagcttgg gcgcagggtc gatgcgacgc aatcgtccga tccggagccg 1860
ggactgtcgg gcgtacacaa atcgcccgca gaagcgcggc cgtctggacc gatggctgtg 1920
tagaagtact cgccgatagt ggaaaccgac gccccagcac tcgtccgagg gcaaaggaat 1980
agagtagatg ccgaccggga tccacttaac gttactgaaa tcatcaaaca gcttgacgaa 2040
tctggatata agatcgttgg tgtcgatgtc agctccggag ttgagacaaa tggtgttcag 2100
gatctcgata agatacgttc atttgtccaa gcagcaaaga gtgccttcta gtgatttaat 2160
agctccatgt caacaagaat aaaacgcgtt ttcgggttta cctcttccag atacagctca 2220
tctgcaatgc attaatgcat tgactgcaac ctagtaacgc cttcaggctc cggcgaagag 2280
aagaatagct tagcagagct attttcattt tcgggagacg agatcaagca gatcaacggt 2340
cgtcaagaga cctacgagac tgaggaatcc gctcttggct ccacgcgact atatatttgt 2400
ctctaattgt actttgacat gctcctcttc tttactctga tagcttgact atgaaaattc 2460
cgtcaccagc cctgggttcg caaagataat tgcatgtttc ttccttgaac tctcaagcct 2520
acaggacaca cattcatcgt aggtataaac ctcgaaatca ttcctactaa gatggtatac 2580
aatagtaacc atggttgcct agtgaatgct ccgtaacacc caatacgccg gccgaaactt 2640
ttttacaact ctcctatgag tcgtttaccc agaatgcaca ggtacacttg tttagaggta 2700
atccttcttt ctcaagttgc ggggcgcgtc gctcaactat cgcgacttgt tgattcccaa 2760
ggtaacttgg ctacgttgcc ctccgcgacg tcccttctca ttccgtgacc ccaaccaaga 2820
tatacacaca cacacacaca tgataatacg aaccagcaga gttctaacct tccctcgtcc 2880
aacttctagg gtcaataccc cttcccgctc aacctccccg tcgtcgcgtg ctccgacggc 2940
gccggcgagg tggtggcggt cgggccccgc gtgaccaagt ggaagcccgg cgatcgtgtg 3000
gtgacgctct tcaaccaggg ccaccagtcg ggccccctcg acgaggccgc cagcaagacg 3060
ggcctgggcg ggtgcatcga cggcacgctg cggcagtacg gcgccttcga ccaggacggc 3120
ctcgtccgca tgcccgcgaa cctcgacttt gtcgaggccg ccaccctcac ctgcgccgcg 3180
ctgaccagct ggaacgccct ctacggcctg aggaggctgc agaagggtga gacggtgctc 3240
gtgcagggca cgggaggtgt tagcttgttg cgctgcaggt gtgtattatc ccccaggatg 3300
ataatagtcc t 3311

<210> 9
<211> 567
<212> DNA
<213> Myceliophthora thermophila
<400> 9
aggatcggtg gagtgaagtt cggaatcgag gttcggcgat gggtcgtaag catggcgact 60
tcgaacttac ttgcactggc aagcgttgcc agaacggcga gaaaaagaag ggtaagcgat 120
attcgcgtca tgatggactg ttccttttgg aacagtagtt gttgtgggaa gactatgtca 180
cacttgccca cctgcaaggc cagggtcgtg gtcgaacgag accagcctcg gcgctgctgg 240
gagctcaaga tgggcacgtt tgattcgtta gacgtcaaca aggctggagt tcctagtgac 300
agccaaaggc acagccacat taagtggcgc tttatctgtc cactaaggtt caattgtggc 360
tttgagccgc gcagtgtgca gtcgtgcatt ggccacctag ctagcagtat ttaagatcct 420
cttctctccc gagatcttcc tcctcttctt ttctttcttt cctcgctgct ccttgatagg 480
ttaggtttta gagctagaaa tagcaagtta aaataaggct agtccgttat caacttgaaa 540
aagtggcacc gagtcggtgc ttttttt 567

<210> 10
<211> 3672
<212> DNA
<213> Myceliophthora thermophila
<400> 10
gtgccgctat ccaagcatat tgcggggaag ccgggggtcg catccgcaaa tcagaggaag 60

aatctggcca tggggcgcgg acgagtggga ctcccccgca gcatcggagg tttggagaga 120
aagtggaggg tgacccatta caaatgtatg tacaacgtat ctccgtatgt acggactaca 180
tacctatgtc cactctatgg atctagtacc ccatagtagt aggctgagag aagccccgaa 240
aaagtcgcga caggctggct tctgttttct ttcttccagc gtgctggtct cctgatgaac 300
gtcgtcgaga cagaaagatt caagatggac aagtgggaaa acgcaataac ggcaatgggc 360
atgcgcgcct ctggtgcttc tcgagtgttc gttgagaggc cgacgtcact tccattaatc 420
cgttgaggcg tcgaccccgc ttggctgccg cctagcttgg ctatcatcgt gttccactcg 480
gccgtggcgg ggaggggtga acggggggag gagtcggggc agcagcgatg cgatcgccgt 540
gcttgcattc cttgtctcga ctgcattgcc acacccaccc ggtcgtctcc atacccctca 600
accccatctc aactgttcag gaattcatca atcgtttata agttcaggcc aaacccttca 660
tcccatctct ttcctcctgc tcctgcacct catcctccca agtcgtctca ttcttctcaa 720
atttaattcc ctcgattcgc actcctaaag gcgttcttcc cgcgtacaca gaggaagctc 780
tgagtgaacc tccgctagct ccagcaccac atccgccacg gctgatctcc tcctcctctt 840
ccaccaccac cacaaccaca gaagatgata ttgaaggagc atttttgggc ttggctggag 900
ctagtggagg tcaacacatc aatgctattt tggtttagtc gtccaggcgg atcacaaaat 960
ttgtgtcgtt tgacaagatg gttcatttag gcaactggtc agatcagccc acttgtaagc 1020
agtagcggcg gcgctcgaag tgtgactctt attagcagac aggaacgagg acattattat 1080
catctgctgc ttggtgcacg ataacttgtg cgtttgtcaa gcaaggtaag tgaacgaccc 1140
ggtcatacct tcttaagttc gcccttcctc cctttatttc agattcaatc tgacttacct 1200
attctaccga agcatcgata tgaaaaagcc tgaactcacc gcgacgtctg tcgagaagtt 1260
tctgatcgaa aagttcgaca gcgtctccga cctgatgcag ctctcggagg gcgaagaatc 1320
tcgtgctttc agcttcgatg taggagggcg tggatatgtc ctgcgggtaa atagctgcgc 1380
cgatggtttc tacaaagatc gttatgttta tcggcacttt gcatcggccg cgctcccgat 1440
tccggaagtg cttgacattg gggaattcag cgagagcctg acctattgca tctcccgccg 1500
tgcacagggt gtcacgttgc aagacctgcc tgaaaccgaa ctgcccgctg ttctgcagcc 1560
ggtcgcggag gccatggatg cgatcgctgc ggccgatctt agccagacga gcgggttcgg 1620
cccattcgga ccgcaaggaa tcggtcaata cactacatgg cgtgatttca tatgcgcgat 1680
tgctgatccc catgtgtatc actggcaaac tgtgatggac gacaccgtca gtgcgtccgt 1740
cgcgcaggct ctcgatgagc tgatgctttg ggccgaggac tgccccgaag tccggcacct 1800
cgtgcacgcg gatttcggct ccaacaatgt cctgacggac aatggccgca taacagcggt 1860
cattgactgg agcgaggcga tgttcgggga ttcccaatac gaggtcgcca acatcttctt 1920
ctggaggccg tggttggctt gtatggagca gcagacgcgc tacttcgagc ggaggcatcc 1980
ggagcttgca ggatcgccgc ggctccgggc gtatatgctc cgcattggtc ttgaccaact 2040
ctatcagagc ttggttgacg gcaatttcga tgatgcagct tgggcgcagg gtcgatgcga 2100
cgcaatcgtc cgatccggag ccgggactgt cgggcgtaca caaatcgccc gcagaagcgc 2160
ggccgtctgg accgatggct gtgtagaagt actcgccgat agtggaaacc gacgccccag 2220
cactcgtccg agggcaaagg aatagagtag atgccgaccg ggatccactt aacgttactg 2280
aaatcatcaa acagcttgac gaatctggat ataagatcgt tggtgtcgat gtcagctccg 2340
gagttgagac aaatggtgtt caggatctcg ataagatacg ttcatttgtc caagcagcaa 2400
agagtgcctt ctagtgattt aatagctcca tgtcaacaag aataaaacgc gttttcgggt 2460
ttacctcttc cagatacagc tcatctgcaa tgcattaatg cattgactgc aacctagtaa 2520
cgccttcagg ctccggcgaa gagaagaata gcttagcaga gctattttca ttttcgggag 2580
acgagatcaa gcagatcaac ggtcgtcaag agacctacga gactgaggaa tccgctcttg 2640
gctccacgcg actatatatt tgtctctaat tgtactttga catgctcctc ttctttactc 2700
tgatagcttg actatgaaaa ttccgtcacc agccctgggt tcgcaaagat aattgcatgt 2760
ttcttccttg aactctcaag cctacaggac acacattcat cgtaggtata aacctcgaaa 2820
tcattcctac taagatggta tacaatagta accatggttg cctagtgaat gctccgtaac 2880
acccaatacg ccggccgaaa cttttttaca actctcctat gagtcgttta cccagaatgc 2940
acaggtacac ttgtttagag gtaatccttc tttcagaaga agacgttcga ggtcattaac 3000
ccgagcaccg aagaggtcat ctgctcggtg cacgaggcca cagagaagga tgtcgacatc 3060
gccgtcgccg ccgcccgcaa ggcgtttgag ggcgagtggc gccgcgtcac cccgcagcag 3120
cgcggcgtcc tgctgctaaa gctggccgat ctcgccgaga agaacctgga ccttctggcc 3180
gcggtcgagt cgctcgacaa cggcaagtcc atcaccatgg cccgcggcga cgtcgccgcc 3240
gttgtcgggt gcatccgcta ctacggcggc tgggccgaca agatcgaggg caggaccatc 3300
gacatcaacc cggacatgtt cagctacacg aggcgcgagc cggtatgcga ttcctttcac 3360
ctctctgctg tgctccccta ccctactccg agagtacaag ctaaccttgg actttttgtt 3420
ccccgccctc cggtagattg gtgtctgcgg ccaaatcatt ccctggaact tcccgctgct 3480
catgcttgcg tggaagatcg gccctgcgct ggccaccgga aacacggttg tcctcaagac 3540
ggccgagcag accccgctgt cggccctcgt ctttgcccag ttcgtcaagg aggccggctt 3600
ccccccgggc gtcctcaaca tcatctcggg tttcggcagg accgccggcg ccgccctctc 3660
gtcgcacatg ga 3672

<210> 11

<211>  567
<212>  DNA
<213>  Myceliophthora thermophila
<400>  11
aggatcggtg gagtgaagtt cggaatcgag gttcggcgat gggtcgtaag catggcgact      60
tcgaacttac ttgcactggc aagcgttgcc agaacggcga gaaaaagaag ggtaagcgat     120
attcgcgtca tgatggactg ttccttttgg aacagtagtt gttgtgggaa gactatgtca     180
cacttgccca cctgcaaggc cagggtcgtg gtcgaacgag accagcctcg gcgctgctgg     240
gagctcaaga tgggcacgtt tgattcgtta gacgtcaaca aggctggagt tcctagtgac     300
agccaaaggc acagccacat taagtggcgc tttatctgtc cactaaggtt caattgtggc     360
tttgagccgc gcagtgtgca gtcgtgcatt ggccacctag ctagcagtat ttaagatcct     420
cttctctccc gagatcttcc tcctcttctt ttctttcttt cctcgatcat ccagagcgcc     480
ggcagtttta gagctagaaa tagcaagtta aaataaggct agtccgttat caacttgaaa     540
aagtggcacc gagtcggtgc ttttttt                                         567

<210>  12
<211>  567
<212>  DNA
<213>  Myceliophthora thermophila
<400>  12
aggatcggtg gagtgaagtt cggaatcgag gttcggcgat gggtcgtaag catggcgact      60
tcgaacttac ttgcactggc aagcgttgcc agaacggcga gaaaaagaag ggtaagcgat     120
attcgcgtca tgatggactg ttccttttgg aacagtagtt gttgtgggaa gactatgtca     180
cacttgccca cctgcaaggc cagggtcgtg gtcgaacgag accagcctcg gcgctgctgg     240
gagctcaaga tgggcacgtt tgattcgtta gacgtcaaca aggctggagt tcctagtgac     300
agccaaaggc acagccacat taagtggcgc tttatctgtc cactaaggtt caattgtggc     360
tttgagccgc gcagtgtgca gtcgtgcatt ggccacctag ctagcagtat ttaagatcct     420
cttctctccc gagatcttcc tcctcttctt ttctttcttt cctcgcacct ttctggactc     480
ggtggtttta gagctagaaa tagcaagtta aaataaggct agtccgttat caacttgaaa     540
aagtggcacc gagtcggtgc ttttttt                                         567

<210>  13
<211>  567
<212>  DNA
<213>  Myceliophthora thermophila
<400>  13
aggatcggtg gagtgaagtt cggaatcgag gttcggcgat gggtcgtaag catggcgact      60
tcgaacttac ttgcactggc aagcgttgcc agaacggcga gaaaaagaag ggtaagcgat     120
attcgcgtca tgatggactg ttccttttgg aacagtagtt gttgtgggaa gactatgtca     180
cacttgccca cctgcaaggc cagggtcgtg gtcgaacgag accagcctcg gcgctgctgg     240
gagctcaaga tgggcacgtt tgattcgtta gacgtcaaca aggctggagt tcctagtgac     300
agccaaaggc acagccacat taagtggcgc tttatctgtc cactaaggtt caattgtggc     360
tttgagccgc gcagtgtgca gtcgtgcatt ggccacctag ctagcagtat ttaagatcct     420
cttctctccc gagatcttcc tcctcttctt ttctttcttt cctcgagctc aagacgccca     480
tcacgtttta gagctagaaa tagcaagtta aaataaggct agtccgttat caacttgaaa     540
aagtggcacc gagtcggtgc ttttttt                                         567

<210>  14
<211>  23
<212>  DNA
<213>  Primer
<400>  14
gtgccgctat ccaagcatat tgc                                              23

<210>  15
<211>  1372
<212>  DNA
<213>  Myceliophthora thermophila
<400>  15
ccgtcgacac gtgtgtcatc cttccattgc aagcgatcac tcgcagggcg tgacgatgaa      60
cgagattttt gcccggaccg attcgcggat atagcggcag ccgaccagcc ctaccacact     120
gatggccgtg tccctagtgt atgctcccag aaccgcaagc atacactggg caatgcttgg     180
tatgcagttg aggcagcttt atgtttccat acccttccac ttcggctcgg ggactcggcg     240

gggtcgcgga agtttgacgg cagccgtcgg gccttaggcc gagattaccg tggttgtggc 300
ccagttttag ccgttcccgt ccgtttccta ccggaccatg attttcgtga accattgcaa 360
tcccgaagcg catttccgac gttaaggagt tacctccgct gcccacaatt catgatcgtg 420
gccggctcaa ggcagcgtgg cggggcatcc gtgtcaagct cccaggagga ggtgcgcgat 480
ttcaaatccg ggccaaaaca ggccaagact ggctggccaa aaaaaggagc gtagacggcc 540
cgggacatcg gacgtcagct cgcagccacc caaaaccggt ccgatctact cgcttactgt 600
ggtagttcag gtacttttga gtagtaaaaa cgctacggca gggccggggg gttccccggt 660
gacggaggtg cctctgcggt ggcgaacatc ccacgcacta tcgagctacg gtgacacctc 720
gtgtcctgtt ggtcttgcaa tgctggggcg gcaggaaatg cgtcgcgctc ctcccggcca 780
agacctaaaa cagacagcgc cgcaaagtcg ctcactagca ccgcgaaacg aagatgcccc 840
acctcaacgc aatctgtgat gcaagcaatt gggaaggctc accccacctc agcgaggggc 900
tcaaccattt ttattatcag ctcatgccac cacaacatga ctgttttctt tccttgctca 960
tcccacattt gacaaaaatc gtcgattaat ctctttccat acaggccgtc cgcgctctga 1020
taaccacata aaagtctctt cagtcaacag ctcaaagctc cctcatccct ccaggtaagc 1080
agccaaagag ctcccccacg gaccccgcac tgcctcatcc cgcctgtatc ggacctgcgc 1140
gacccagcag agaatcccaa acctttgctg cttgctgccc ggttccggac tgagctgcaa 1200
cccaagcctt taaaaagcta ttcccttctc ccacggtgtc aactctgtcc tatccctccg 1260
acatccgttg agctcaacaa ctccccgaac cttttacccc gcgccgagct acccctccat 1320
caaaccaccc tgacagctcg ctcactcacc tccccacatc acagaaatca aa 1372

<210> 16
<211> 5188
<212> DNA
<213> Myceliophthora thermophila
<400> 16
caggatattg ataacacctt taattttcaa gacgcctaat taaaaagcca tcatccatga 60
gcggaaacga gaagccgacg tcgcggatcg cgatcgtggg ggtgggccag gtgggcgcgg 120
cggcggcgta cgcgctgatc ctcgagtcgg tcgcgggcga gctgctgctg gtggacatca 180
aagcagactg gcgagacggg caggtacgcg atctggccga cgtggcgtac ggcatcggca 240
gcggcacgcg cgtgcgggcg gcgacgcacc gcgaggcggg ccagtgcgac attgtggtca 300
tcaccgccgg ctccaagtgg tccatcggtc agtttctgat taattattaa ttttctcatc 360
tcaggggatg caaaaagggg ctcacgcatg agaataatta attaggcgag acgaacgtcc 420
agcacatgca ccgcaacgtg tcggtcatgc gcagcgtgat gcgggacatg acgccgttcc 480
ggtccgacgc tatcgtgatc gtggtgtcaa acccggtcga cctgctcacc tccatcgcgc 540
aggagctctc ggggctcccg aggttccaag tgtttggctc gggacgagga cttctagaga 600
tcgttgcatc gtcccaaagc tattggcggg atattctgtt tgcagttggc tgacttgaag 660
taatctctgc agatctttaa ttaactcgag tgacggtgct tttcacctct cgatgcccga 720
aatcgggtct aagctgagtt tgatcaaata tgtgactcca acatcgcccc cttcggcaaa 780
ccccgtcgac acgtgtgtca tccttccatt gcaagcgatc actcgcaggg cgtgacgatg 840
aacgagattt ttgcccggac cgattcgcgg atatagcggc agccgaccag ccctaccaca 900
ctgatggccg tgtccctagt gtatgctccc agaaccgcaa gcatacactg ggcaatgctt 960
ggtatgcagt tgaggcagct ttatgtttcc atacccttcc acttcggctc ggggactcgg 1020
cggggtcgcg gaagtttgac ggcagccgtc gggccttagg ccgagattac cgtggttgtg 1080
gcccagtttt agccgttccc gtccgtttcc taccggacca tgattttcgt gaaccattgc 1140
aatcccgaag cgcatttccg acgttaagga gttacctccg ctgcccacaa ttcatgatcg 1200
tggccggctc aaggcagcgt ggcggggcat ccgtgtcaag ctcccaggag gaggtgcgcg 1260
atttcaaatc cgggccaaaa caggccaaga ctggctggcc aaaaaaagga gcgtagacgg 1320
cccgggacat cggacgtcag ctcgcagcca cccaaaaccg gtccgatcta ctcgcttact 1380
gtggtagttc aggtactttt gagtagtaaa aacgctacgg cagggccggg gggttccccg 1440
gtgacggagg tgcctctgcg gtggcgaaca tcccacgcac tatcgagcta cggtgacacc 1500
tcgtgtcctg ttggtcttgc aatgctgggg cggcaggaaa tgcgtcgcgc tcctcccggc 1560
caagacctaa aacagacagc gccgcaaagt cgctcactag caccgcgaaa cgaagatgcc 1620
ccacctcaac gcaatctgtg atgcaagcaa ttgggaaggc tcaccccacc tcagcgaggg 1680
gctcaaccat ttttattatc agctcatgcc accacaacat gactgttttc tttccttgct 1740
catcccacat ttgacaaaaa tcgtcgatta atctctttcc atacaggccg tccgcgctct 1800
gataaccaca taaaagtctc ttcagtcaac agctcaaagc tccctcatcc ctccaggtaa 1860
gcagccaaag agctccccca cggaccccgc actgcctcat cccgcctgta tcggacctgc 1920
gcgacccagc agagaatccc aaacctttgc tgcttgctgc ccggttccgg actgagctgc 1980
aacccaagcc tttaaaaagc tattcccttc tcccacggtg tcaactctgt cctatccctc 2040
cgacatccgt tgagctcaac aactccccga accttttacc ccgcgccgag ctacccctcc 2100
atcaaaccac cctgacagct cgctcactca cctccccaca tcacagaaat caaaactaga 2160
atgtcgtctc acggctccca tgacggggcc agcaccgaga agcatcttgc tactcatgac 2220
attgcgccca cccacgacgc catcaagata gtgcccaagg gccatggcca gacagccaca 2280

aagcccggtg cccaagagaa ggaggtccgc aacgccgccc tatttgcggc catcaaggag 2340
tccaatatca agccctggag caaggagtcc atccacctct atttcgccat cttcgtcgcc 2400
ttttgttgtg catgcgccaa cggttacgat ggttcactca tgaccggaat catcgctatg 2460
gacaagttcc agaaccaatt ccacactggt gacactggtc ctaaagtctc tgtcatcttt 2520
tctctctata ccgttggtgc catggttgga gctcccttcg ctgctatcct ctctgatcgt 2580
tttggccgta agaagggcat gttcatcggt ggtatcttta tcattgtcgg ctccattatt 2640
gttgctagct cctccaagct cgctcagttt gtcgttggcc gcttcgttct tggcctcggt 2700
atcgccatca tgaccgttgc tgccccggcc tactccatcg aaatcgcccc tcctcactgg 2760
cgcggccgct gcactggctt ctacaactgc ggttggttcg gaggttcgat tcctgccgcc 2820
tgcatcacct atggctgcta cttcattaag agcaactggt catggcgtat ccccttgatc 2880
cttcaggctt tcacgtgcct tatcgtcatg tcctccgtct tcttcctccc agaatcccct 2940
cgcttcctat ttgccaacgg ccgcgacgct gaggctgttg cctttcttgt caagtatcac 3000
ggcaacggcg atcccaattc caagctggtg ttgctcgaga ctgaggagat gagggacggt 3060
atcaggaccg acggtgtcga caaggtctgg tgggattacc gcccgctctt catgacccac 3120
agcggccgct ggcgcatggc ccaggtgctc atgatctcca tctttggcca gttctccggc 3180
aacggtctcg gttacttcaa taccgtcatc ttcaagaaca ttggtgtcac cagcacctcc 3240
caacagctcg cctacaacat cctcaactcc gtcatctccg ctatcggtgc cttgaccgcc 3300
gtctccatga ctgatcgtat gccccgccgc gcggtgctca ttatcggtac cttcatgtgc 3360
gccgctgctc ttgccaccaa ctcgggtctt tcggctactc tcgacaagca gactcaaaga 3420
ggcacgcaaa tcaacctgaa ccagggtatg aacgagcagg atgccaagga caacgcctac 3480
ctccacgtcg acagcaacta cgccaagggt gccctggccg cttacttcct cttcaacgtc 3540
atcttctcct tcacctacac tcccctccag ggtgttattc ccaccgaggc tctcgagacc 3600
accatccgtg gcaagggtct tgccctttcc ggcttcattg tcaacgccat gggcttcatc 3660
aaccagttcg ctggccccat cgctctccac aacattggct acaagtacat ctttgtcttt 3720
gtcggctggg atcttatcga gaccgtcgct tggtacttct ttggtgtcga atcccaaggc 3780
cgtaccctcg agcagctcga atgggtctac gaccagccca accccgtcaa ggcctcccta 3840
aaagtcgaaa aggtcgtcgt ccaggccgac ggccatgtgt ccgaagctat cgttgcttag 3900
gatatcgaat tcggatccac ttaacgttac tgaaatcatc aaacagcttg acgaatctgg 3960
atataagatc gttggtgtcg atgtcagctc cggagttgag acaaatggtg ttcaggatct 4020
cgataagata cgttcatttg tccaagcagc aaagagtgcc ttctagtgat ttaatagctc 4080
catgtcaaca agaataaaac gcgtttcggg tttacctctt ccagatacag ctcatctgca 4140
atgcattaat gcattggacc tcgcaaccct agtacgccct tcaggctccg gcgaagcaga 4200
agaatagctt agcagagtct attttcattt tcgggagacg agatcaagca gatcaacggt 4260
cgtcaagaga cctacgagac tgaggaatcc gctcttggct ccacgcgact atatatttgt 4320
ctctaattgt actttgacat gctcctcttc tttactctga tagcttgact atgaaaattc 4380
cgtcaccagc ccctgggttc gcaaagataa ttgcactgtt tcttccttga actctcaagc 4440
ctacaggaca cacattcatc gtaggtataa acctcgaaaa tcattcctac taagatgggt 4500
atacaatagt aaccatgcat ggttgcctag tgaatgctcc gtaacaccca atacgccggc 4560
cgaaactttt ttacaactct cctatgagtc gtttacccag aatgcacagg tacacttgtt 4620
tagaggccgc tggacaggtt cgtggctcca gtcgtgtcgg gcgtggaact catgcacgcg 4680
gacctcgagg acgagtgcaa gttccgttcc gagcacatca tccgggccaa ggggggcaca 4740
ccttttggca tcggctcggt cgtggccagc ctctgctcga ccgtcctccg ggacaagggc 4800
gacgtgcggc cggtcagcta ctaccagccc gactatggct gctgcttcag cctgcccgtg 4860
gttctgggga ggaagggcat catgcggacg atcgcgatgc cgctggacga gcgtgagaag 4920
gccacggtgt cccggtcggc ccagaggctc aaggagacga ttgagcgatt gcacaagagc 4980
cagtgagagt gcagtacgga tacataggta cagtatgtac atacatgtta tgtacggacc 5040
gtgctagtaa ggttggtact tacttgtagg tctcgttagc ttgtcaaaaa aaaaaaaaaa 5100
aaaaaaaaaa aaaaaaaaaa aagggggggg gggcgggggg gggggggagg ttgaagtcag 5160
gggtgtcagg ggttaacaac agatgggc 5188

<210> 17
<211> 730
<212> DNA
<213> Myceliophthora thermophila
<400> 17
tcaagtccag catagcgaca accaaggagc attcgatatg caacccgatc tcaagcagag 60
aaaggtgtta gggccgacga aaagaccagt cttgcgagcc aacctgcgct ggacctatgg 120
tactgcgtgt gtgtgggcgg ataccgagtg tactccgtaa ggagggttgg tctcatgcct 180
cttggcggga gccgcccgat aactagtata actagttgta actccgtatc cggttacgga 240
aacggaaagg cccgctcggc tgttctccgg cggctccccg atcgctgatc agagcatgga 300
acagatgtca attacatcac tcccgcgtaa acgaaccata gttatcgaac cacagttatc 360
gaaccacaga gccagcccat gggaacgtct gaacagctcg gaggatgcaa ccgatattgc 420
aatgcaaaac gtcacccatg ctacaattaa ttccctgcac aactacttgt aagccgcgag 480

gcctagaaca cagttgcaga acctgggtat cgtgcctgtg gtctgatgca gatatgtgtc 540
accactcaag accccgccaa cacgccgctt cgaggccctg aacagtacaa agggcgcttc 600
aaattcgtac aagccccccc gaggccgttt tcaagtcttt gtatgaccat ctattttccg 660
attgacgtcc ctcacggatt ctctttcgtt gctgacctcc ttgtgaccac aaacatcgcc 720
aacaacagac 730

<210> 18
<211> 28
<212> DNA
<213> Primer
<400> 18
tcatcttctg tggttgtggt ggtggtgg 28

<210> 19
<211> 30
<212> DNA
<213> Primer
<400> 19
taccgtcaaa atgtctgaaa ttactttggg 30

<210> 20
<211> 5496
<212> DNA
<213> Myceliophthora thermophila
<400> 20
cgtcaggtgt ctgttctcgc gaaatacata taatctcctc ggttagattt gttcaggaga 60
aagatgatgg tgccaaactc tacagtttag gcttctctac cgaggaaaga gaatgagagg 120
tcggaccacc agcgtgctaa caggacgaga cttgagccaa tttgtcaata gacaagatag 180
tctcgtgtgc caagccaaca tcatcatccg atctcagctg agcccctcct tctgaaaaga 240
gtataattac agcagcttca gacgttgagg catcactgtt gctgccgcac gtcagtcaac 300
cacttaatcc gctctgtgtg accggtgacg gattcgatca ttctacgatc cgctccgcat 360
cgccggttaa agggggtgac gagttattta ttacctttac ggattcaaga tgcctcagcc 420
aaacgcagca gatgtcaagg tcagtgaaga ggattgtttc gaattactca gaaatgaaac 480
acctgcttac ctaggtacac gttctcatgc tccgcccaac ggatagtcaa tcaagatcgt 540
tatcgtcggt gccggctcgg taggcgtgac gactgcgtac tcaagtccag catagcgaca 600
accaaggagc attcgatatg caacccgatc tcaagcagag aaaggtgtta gggccgacga 660
aaagaccagt cttgcgagcc aacctgcgct ggacctatgg tactgcgtgt gtgtgggcgg 720
ataccgagtg tactccgtaa ggagggttgg tctcatgcct cttggcggga gccgcccgat 780
aactagtata actagttgta actccgtatc cggttacgga aacggaaagg cccgctcggc 840
tgttctccgg cggctccccg atcgctgatc agagcatgga acagatgtca attacatcac 900
tcccgcgtaa acgaaccata gttatcgaac cacagttatc gaaccacaga gccagcccat 960
gggaacgtct gaacagctcg gaggatgcaa ccgatattgc aatgcaaaac gtcacccatg 1020
ctacaattaa ttccctgcac aactacttgt aagccgcgag gcctagaaca cagttgcaga 1080
acctgggtat cgtgcctgtg gtctgatgca gatatgtgtc accactcaag accccgccaa 1140
cacgccgctt cgaggccctg aacagtacaa agggcgcttc aaattcgtac aagccccccc 1200
gaggccgttt tcaagtcttt gtatgaccat ctattttccg attgacgtcc ctcacggatt 1260
ctctttcgtt gctgacctcc ttgtgaccac aaacatcgcc aacaacagac atgggcatct 1320
tcaacaagaa gcccgtggct caggccgtcg acctcaatca gatacaggag gaggctcctc 1380
agtttgagag ggttgactgg aagaaggacc ccggtcttcg caagctctac ttctacgcct 1440
tcattctttg cattgcttcg gccaccaccg gttacgatgg catgttcttc aactcggtgc 1500
agaacttcga gacctggatt aagtactttg gcgacccgcg aggatccgag cttggtctgc 1560
tcggtgctct ctaccagatt ggttccattg gctccatccc ctttgtcccc ctccttaccg 1620
acaactttgg ccgcaagacc cccatcatca tcggctgcgt tatcatgatc gtcggtgccg 1680
ttctccaggc cacggccaag aacctcgata cattcatggg cggccgtacc atgctcggct 1740
ttggcaactc cctcgcccag atcgcctccc ccatgcttct caccgagctc gcccatcctc 1800
aacaccgcgc tcgtctcacc accatctaca actgcttgtg gaacgttggt gccctcgtcg 1860
tctcgtggtt ggcctttggc accaactaca tcaacaacga ctggtcatgg cgcattcccg 1920
ccttgctcca ggctttcccc tccatcattc agctcctcgg tatctggtgg gttcccgagt 1980
ctccccgttt cctcatcgcc aaggacaagc acgacgaggc cctccacatc ctcgccaagt 2040
accacgccaa cggcgacccc aaccacccca ccgtccagtt tgagttccgc gagatcaagg 2100
agaccatccg cctcgagatg gaatcgacca agaacagcag ctacctcgac ttcttcaaga 2160
gccgcggcaa ccgctaccgc ctcgccatcc tcctctcgct cggcttcttc tcccaatggt 2220
ccggcaacgc catcatctcc aactactcct ccaagctgta cgagaccgcc ggcgtcaccg 2280

actccaccgc caaactcggt ctttccgccg gacagaccgg tctcgcgctc atcgtgtccg 2340
tcaccatggc gctgctcgtc gacaagctcg gtcgtcgtct tgctttcctc gcttccacgg 2400
gcggcatgtg cggcaccttt gtcatttgga cgttgacagc cggcctgtac ggcgagcacc 2460
gcctcaaggg cgccgacaag gccatgatct tctttatctg ggtgttcggc atcttctact 2520
cgctcgcctg gtccgggttg ctggtcggct acgccatcga aatcctccct taccgacttc 2580
gcggcaaggg gttgatggtc atgaacatgt cggtgcagtg cgcgctgacg ctcaatactt 2640
atgcgaaccc tgttgcgttt gattactttg gtcctgatca ctcgtggaag ctttatctta 2700
tttacacttg ctggatcgcc gccgagttcg tcttcgtctt cttcatgtac gtcgagacca 2760
agggccccac gctcgaggag cttgccaagg tcattgatgg cgatgaggcc gatgttgccc 2820
acatcgacat tcaccaggtc gagaaggagg tggagattca cgagcatgag ggcaagtctg 2880
ttgcttgaga tatcgaattc gggatccacg aacctctctg aaggaggttc tgagacacgc 2940
gcgattcttc tgtatatagt tttatttttc actctggagt gcttcgctcc accagtacat 3000
aaaccttttt tttcacgtaa caaaatggct tcttttcaga ccatgtgaac catcttgatg 3060
ccttgacctc ttcagttctc actttaacgt agttcgcgtt tgtctgtatg tcccagttgc 3120
atgtagttga gataaatacc cctggaagtg ggtctgggcc tttgtgggac ggagccctct 3180
ttctgtggtc tggagagccc gctctctacc gcctaccttc ttaccacagt acactactca 3240
cacattgctg aactgaccca tcatactcga cagaagatga tattgaagaa gcactttttg 3300
ggcttggctg gagctagtgg aggtcaacaa tgaatgccta ttttggttta gtcgtccagg 3360
cggtgagcac aaaatttgtg tcgtttgaca agatggttca tttaggcaac tggtcagatc 3420
agccccactt gtagcagtag cggcggcgct cgaagtgtga ctcttattag cagacaggaa 3480
cgaggacatt attatcatct gctgcttggt gcacgataac ttggtgcgtt tgtcaagcaa 3540
ggtaagtgaa cgacccggtc ataccttctt aagttcgccc ttcctccctt tatttcagat 3600
tcaatctgac ttacctattc tacccaagca aagcttcgat taggaagtaa ccatgagccc 3660
agaacgacgc ccggccgaca tccgccgtgc caccgaggcg gacatgccgg cggtctgcac 3720
catcgtcaac cactacatcg agacaagcac ggtcaacttc cgtaccgagc cgcaggaacc 3780
gcaggagtgg acggacgacc tcgtccgtct gcgggagcgc tatccctggc tcgtcgccga 3840
ggtggactgc gaggtcgccg gcatcgccta cgcgggcccc tggaaggcac gcaacgccta 3900
cgactggacg gccgagtcga ccgtgtacgt ctccccccgc caccagcgga cgggactggg 3960
ctccacgctc tacacccacc tgctgaagtc cctggaggca cagggcttca agagcgtggt 4020
cgctgtcatc gggctgccca acgacccgag cgtgcgcatg cacgaggcgc tcggatatgc 4080
cccccgcggc atgctgcggg cggccggctt caagcacggg aactggcatg acgtgggttt 4140
ctggcagctg gacttcagcc tgccggtacc gccccgtccg gtcctgcccg tcaccgagat 4200
ctgaacttaa cgttactgaa atcatcaaac agcttgacga atctggatat aagatcgttg 4260
gtgtcgatgt cagctccgga gttgagacaa atggtgttca ggatctcgat aagatacgtt 4320
catttgtcca agcagcaaag agtgccttct agtgatttaa tagctccatg tcaacaagaa 4380
taaaacgcgt ttcgggttta cctcttccag atacagctca tctgcaatgc attaatgcat 4440
tggacctcgc aaccctagta cgcccttcag gctccggcga agcagaagaa tagcttagca 4500
gagtctattt tcattttcgg gagacgagat caagcagatc aacggtcgtc aagagaccta 4560
cgagactgag gaatccgctc ttggctccac gcgactatat atttgtctct aattgtactt 4620
tgacatgctc ctcttcttta ctctgatagc ttgactatga aaattccgtc accagcccct 4680
gggttcgcaa agataattgc actgtttctt ccttgaactc tcaagcctac aggacacaca 4740
ttcatcgtag gtataaacct cgaaaatcat tcctactaag atgggtatac aatagtaacc 4800
atgcatggtt gcctagtgaa tgctccgtaa cacccaatac gccggccgaa acttttttac 4860
aactctccta tgagtcgttt acccagaatg cacaggtaca cttgtttaga ggtattccgg 4920
gacgtagtgc cccgcatcgc acgccacgcg cccgacacca tcctcgtcgt ggccaccaac 4980
ccggtcgacg tgctcacgca cgcggcccac cacctctccg gcttcccact cgagagggtg 5040
atcgggtccg gcacggccat ggacaccacc aggttccggc acgagctggg caagcacttt 5100
ggggtcaacc cgcggaacgt gcacgccatg atcgtgggcg agcacggcga cagccagctg 5160
cccgtgtggt cgctcgccac catctgcggg atgcggctgc acgactactg caggcggccc 5220
cgcatggagc acgacgaggc cgcactcgag gcctgcgcca agcggaccag ggaggccgcc 5280
tacgagatca tccggcgcaa gggcaagacc aactacggcg tcgcctcggt gctcgtcagc 5340
atcctgcagc ccatcgtcac cgacagcgac gccatcatga cggtctcgag ggtcggcacg 5400
tacgccggaa tccaggacgt ggccctcagc atgccctgca agctgaaccg gcatggcgcg 5460
taccaggacg tgcccctact cctcagcgag ttggag 5496

<210> 21
<211> 31
<212> DNA
<213> Primer
<400> 21
accacaacca cagaagatga tattgaagga g 31

<210> 22

<211>  32
<212>  DNA
<213>  Primer
<400>  22
gtcttcttct gaaagaagga ttacctctaa ac                                    32

<210>  23
<211>  30
<212>  DNA
<213>  Primer
<400>  23
tccttctttc agaagaagac gttcgaggtc                                       30

<210>  24
<211>  23
<212>  DNA
<213>  Primer
<400>  24
tccatgtgcg acgagagggc ggc                                              23

<210>  25
<211>  45
<212>  DNA
<213>  Primer
<400>  25
gatttcagta acgttaagtg gatccttatt gcttagcgtt ggtag                      45

<210>  26
<211>  567
<212>  DNA
<213>  Myceliophthora thermophila
<400>  26
aggatcggtg gagtgaagtt cggaatcgag gttcggcgat gggtcgtaag catggcgact      60
tcgaacttac ttgcactggc aagcgttgcc agaacggcga gaaaaagaag ggtaagcgat     120
attcgcgtca tgatggactg ttccttttgg aacagtagtt gttgtgggaa gactatgtca     180
cacttgccca cctgcaaggc cagggtcgtg gtcgaacgag accagcctcg gcgctgctgg     240
gagctcaaga tgggcacgtt tgattcgtta gacgtcaaca aggctggagt tcctagtgac     300
agccaaaggc acagccacat taagtggcgc tttatctgtc cactaaggtt caattgtggc     360
tttgagccgc gcagtgtgca gtcgtgcatt ggccacctag ctagcagtat ttaagatcct     420
cttctctccc gagatcttcc tcctcttctt ttctttcttt cctcgacttg gttacctgct     480
ccgtgtttta gagctagaaa tagcaagtta aaataaggct agtccgttat caacttgaaa     540
aagtggcacc gagtcggtgc ttttttt                                         567

<210>  27
<211>  2197
<212>  DNA
<213>  Myceliophthora thermophila
<400>  27
agtttatgtg gaaaatggca tttgaaatga ggggtattcg gaatctaggg cgtttgctac      60
tgcgattaga tgacgtcagg tgtctgttct cgcgaaatac atataatctc ctcggttaga     120
tttgttcagg agaaagatga tggtgccaaa ctctacagtt taggcttctc taccgaggaa     180
agagaatgag aggtcggacc accagcgtgc taacaggacg agacttgagc caatttgtca     240
atagacaaga tagtctcgtg tgccaagcca acatcatcat ccgatctcag ctgagcccct     300
ccttctgaaa agagtataat tacagcagct tcagacgttg aggcatcact gttgctgccg     360
cacgtcagtc aaccacttaa tccgctctgt gtgaccggtg acggattcga tcattctacg     420
atccgctccg catcgccggt taaagggggt gacgagttat ttattacctt tacggattca     480
agatgcctca gccaaacgca gcagatgtca aggtcagtga agaggattgt ttcgaattac     540
tcagaaatga aacacctgct tacctaggta cacgttctca tgctccgccc aacggatagt     600
catattgaag gagcactttt tgggcttggc tggagctagt ggaggtcaac aatgaatgcc     660
tattttggtt tagtcgtcca ggcggtgagc acaaaatttg tgtcgtttga caagatggtt     720
catttaggca actggtcaga tcagccccac ttgtagcagt agcggcggcg ctcgaagtgt     780
gactcttatt agcagacagg aacgaggaca ttattatcat ctgctgcttg gtgcacgata     840
acttggtgcg tttgtcaagc aaggtaagtg aacgacccgg tcataccttc ttaagttcgc     900

ccttcctccc tttatttcag attcaatctg acttacctat tctacccaag caaagcttcg 960
attaggaagt aaccatgagc ccagaacgac gcccggccga catccgccgt gccaccgagg 1020
cggacatgcc ggcggtctgc accatcgtca accactacat cgagacaagc acggtcaact 1080
tccgtaccga gccgcaggaa ccgcaggagt ggacggacga cctcgtccgt ctgcgggagc 1140
gctatccctg gctcgtcgcc gaggtggacg gcgaggtcgc cggcatcgcc tacgcgggcc 1200
cctggaaggc acgcaacgcc tacgactgga cggccgagtc gaccgtgtac gtctcccccc 1260
gccaccagcg gacgggactg ggctccacgc tctacaccca cctgctgaag tccctggagg 1320
cacagggctt caagagcgtg gtcgctgtca tcgggctgcc caacgacccg agcgtgcgca 1380
tgcacgaggc gctcggatat gccccccgcg gcatgctgcg ggcggccggc ttcaagcacg 1440
ggaactggca tgacgtgggt ttctggcagc tggacttcag cctgccggta ccgccccgtc 1500
cggtcctgcc cgtcaccgag atctgatccg tcgacctgca gatcgttcaa acatttggca 1560
ataaagtttc ttaagattga atcctgtact agtgatccgt cggcgactac gaggactgcg 1620
cccacgcggc ggcggtcatc atcacggcgg gcgtcaacca aaagcccggc cagacgcgcc 1680
tcgacctcgt caagaccaac tacgcgctat tccgggacgt agtgccccgc atcgcacgcc 1740
acgcgcccga caccatcctc gtcgtggcca ccaacccggt cgacgtgctc acgcacgcgg 1800
cccaccacct ctccggcttc ccactcgaga gggtgatcgg gtccggcacg gccatggaca 1860
ccaccaggtt ccggcacgag ctgggcaagc actttggggt caacccgcgg aacgtgcacg 1920
ccatgatcgt gggcgagcac ggcgacagcc agctgcccgt gtggtcgctc gccaccatct 1980
gcgggatgcg gctgcacgac tactgcaggg cggcccgcat ggagcacgac gaggccgcac 2040
tcgaggcctg cgccaagcgg accagggagg ccgcctacga gatcatccgg cgcaagggca 2100
agaccaacta cggcgtcgcc tcggtgctcg tcagcatcct gcagcccatc gtcaccgaca 2160
gcgacgccat catgacggtc tcgagggtcg gcacgta 2197

<210> 28
<211> 1200
<212> DNA
<213> Myceliophthora thermophila
<400> 28
cctgactgta taagagaaat ggcctcccct cggagtctga gcgcttcctt tcctttatct 60
gcagactttt cagtcatttc atcttcttta tcctaaggtt ttctgccaac tcgtattaag 120
gcccttgccg ttgttaagaa cacacgtcat catctcgtct tcgactgcct ttcctattgc 180
accgagattc tgagccgtcg atcatctatc tctctttcga cttccttctt caaccctcaa 240
cctactcaac acatcctacc ttccaaaatg gcgcaaagga atgcccccaa gaaggccgtc 300
cacttcggtg ctggaaacat cggtatgaat cctcttggga gattggcatc gacgaccgac 360
cttgactgac cgtgatgatt tccaggtcgt ggcttcgtcg cctgcttttt gcacaattca 420
ggctacgagg tcgtcttctc tgaggttaac gacgcaactg tatccaagct caactcccaa 480
aagagctatc gggtgattga agtcggcgcc gacgggacgt cggagtccat tatcaccaat 540
taccgcgcca tcaactcgag gacccacgag gccgaactgg ttcgggaaat tgccacggcg 600
ggagcttact ccggtcgctg tcattgcctg cgagaacgcc atcggggcca ccgatactct 660
tgctggtttc atcaagtcgc cagagaacac caatcctgac cggcttgccg actacgataa 720
gcgtgctcgg ttcgccaact ccgccatcga ccgcatcgtc cccgcacagg accccaacgc 780
cggtctcgac gttaggctcg agaagttcta cgagtgggtg gtagacagca ccccatttgc 840
cgaccatgcc ccccccgaca tcaagggtgt tcgctgggtg aacaacctgc agcccttcat 900
cgagcgcaag ctgtacacgg tcaacacggg ccacgccacg gccgcgtacc acggctacat 960
ccggcgtaag agcaccgtct acgacgcgct gcaagaccgc gagattcaag aagaggtcaa 1020
gaaggcgctg cagaacacgg ccgacctcat cacgcagaag cacggcatct cgcacgagga 1080
gcagcaggcc tacgtcgaga agattgtccg ccgtatcggc aatcctcacc tcgaggacgc 1140
cgtcgagcgc gtcggccgcg ccccgttgcg caagctcagc cgtaaggaac gcttcattgg 1200

<210> 29
<211> 6134
<212> DNA
<213> Synthetic
<220>
<221> misc_feature
<222> (5718)..(5718)
<223> n is a, c, g, or t
<220>
<221> misc_feature
<222> (5926)..(5926)
<223> n is a, c, g, or t
<220>
<221> misc_feature

<222>   (6017)..(6017)
<223>   n is a, c, g, or t
<400>   29
tcctccgagg ttcgacatca gggttcgtca tagggagtga aacacccgcc atgattccgt      60
agccgcgcgc gaagatacga agcagatatt tcacggacat ggcggagata cttgtttccc     120
gtactaaggt agtcatgtcg gagacatctg aacgacagag ctggccaaga gaaccgacca     180
gttgccccag gacgatctag acaaaaaaaa agagagatga gtgggccact tttgccacaa     240
catcgacggc cctgcgaccg cccccaggca aacaaacaaa ccgccgaaca ataatacttt     300
tgtcatttta ggaggagcgt tgtatggata aaaacaacat ctcgttgctg cagaatgtgg     360
acttcaaact tgcagaaaat gggaggcgga tttgcatgat cggagggtag ttgactcacg     420
ccgcaggctg caaatccgtc ctccattatt ccatgaacaa cttcgtaagg ttgggctgag     480
cgccaatgcc taacggaccg ggggccacag cgcaacgtcc cacttaaagg ccagcgtgac     540
atgccagttc cataccaagt agtggcacca gaggcggcca atgctcagta agggcaggga     600
gggaggctca aacgattggc aaaaagaggg gcttgccagt tcagttccct gtgcgagcgc     660
gagaggggca gtttcaaatc tggaggggtg tgttgcgctg gtctgaagag aaagagaaga     720
ctgtacttaa taattgttca aagagtccat catcgcgttg cggactcctc tagctgtatt     780
tagagcccta tcattacttg tcgggtgcga atcaaaatac cgggatgcag ccctctggcg     840
atttgcatgc ggttgtggag gaagtgaagc ctgaatcgcg gggctgggcg gcaaagcacg     900
acgtgaaatt cctggcgaaa ttcgagggct tgccccaccg tggttgaagt ttttgtgctg     960
cgtaacccca ccaacccgcc ttgcccctcc cgcctgccca taaaaacttc gacccctcct    1020
caaatcttct tcgattcttc ctcttcactt ccttcgtcgg catacctgat tcaagcaatc    1080
acctgccact ttcaagtgcg tataccatca tcgatacact ggttcttgac aagtacatcg    1140
tctctaactt tcctttttgc agttttcatt aagcgcaagt cgccagtttc gttcttcaga    1200
aagcttatgg actacaagga ccatgatggc gattacaagg accacgacat cgattataag    1260
gatgatgatg acaagcctcc gaggaaacgt gccaaaacag aagatgagat ggataagaag    1320
tactccatcg gcctcgacat cggcaccaac tccgtcggct gggccgtcat caccgatgag    1380
tacaaggtcc cttccaagaa gttcaaggtc ctcggcaaca ccgatcgcca ttccatcaag    1440
aagaacctga tcggcgccct cctgttcgat tccggcgaaa ccgccgaggc cacccgcctt    1500
aaacgcaccg cccgtcgccg ctacacccgc cgcaagaacc gcatctgcta cctccaagaa    1560
atcttctcca acgagatggc caaggtcgat gatagcttct tccaccgcct cgaagagtcc    1620
ttcctggtcg aagaggataa gaagcacgag cgccatccta tcttcggcaa catcgtcgat    1680
gaggtcgcct accatgagaa gtaccctacc atctaccatc tccgcaagaa gctcgtcgat    1740
tccaccgata aggccgatct ccgcctcatc tacctcgccc tcgcccatat gatcaagttc    1800
cgcggccatt tcctcatcga gggcgatctc aaccctgata actccgatgt cgataagctg    1860
ttcatccagc tcgtccagac ctacaaccag ctgttcgagg aaaaccctat caacgcctcc    1920
ggcgtcgatg ccaaggccat cctctccgct cgcctctcca agtctcgccg ccttgagaac    1980
cttatcgccc agctccctgg cgagaagaag aacggcctct tcggcaacct gatcgccctc    2040
tccctcggcc tcacccctaa cttcaagtcc aacttcgatc tcgccgagga tgccaagctc    2100
cagctctcca aggataccta cgatgatgat ctcgataacc tcctcgccca gatcggcgat    2160
cagtacgccg atctgttcct cgccgccaag aacctctccg atgccatcct cctctccgac    2220
atcctccgcg tcaacaccga gatcaccaag gcccctctgt ccgcctccat gatcaagcgc    2280
tacgatgagc atcatcagga cctcaccctg ctcaaggccc tcgtccgcca gcagctccct    2340
gagaagtaca aagagatttt cttcgatcag tccaagaacg gctacgccgg ctacatcgat    2400
ggcggcgctt cccaagaaga gttctacaag ttcatcaagc ctatccttga gaagatggat    2460
ggcaccgagg aactcctcgt caagctcaac cgcgaggacc tcctccgcaa gcagcgcacc    2520
ttcgataacg gctccatccc tcatcaaatc catctcggcg agctgcatgc catcttgcgc    2580
cgccaagagg atttctaccc attcctcaag gataaccgcg agaagatcga aaagattctc    2640
accttccgca tcccttacta cgtcggccct ctcgctcgcg gcaactcccg cttcgcctgg    2700
atgacccgca agtccgagga aaccatcacc ccttggaact tcgaggaagt cgtcgataag    2760
ggcgcctccg cccagtcctt catcgagcgc atgaccaact tcgataagaa cctccctaac    2820
gagaaggtcc tccctaagca ctccctgctc tacgagtact tcaccgtcta caacgagctg    2880
accaaggtca agtacgtcac cgagggtatg cgcaagcctg ccttcctgtc cggcgagcag    2940
aagaaggcca tcgtcgatct gctgttcaag accaaccgca aggtcaccgt caagcagctc    3000
aaagaggatt acttcaagaa aatcgagtgc ttcgattccg tcgagatcag cggcgtcgag    3060
gaccgcttca acgcctccct cggaacctac catgatctcc tcaagattat caaggataag    3120
gatttcctcg acaacgagga aaacgaggac atccttgagg acatcgtcct caccctcacc    3180
ctcttcgagg accgcgaaat gatcgaggaa cgcctcaaga cctacgccca tctcttcgat    3240
gataaggtca tgaagcagct caagcgccgt cgctacaccg gctggggtcg cctctcccgc    3300
aagctcatca acggcatccg cgataagcag tccggcaaga ctatcctcga tttcctcaag    3360
tccgatggct tcgccaaccg caacttcatg cagctcatcc atgatgattc cctcaccttc    3420
aaagaggaca tccagaaggc ccaggtcagc ggccagggcg attccctcca tgagcatatc    3480
gccaacctcg ccggctcccc tgccatcaag aagggcatcc tccagaccgt caaggtcgtc    3540
gatgagctgg tcaaggtcat gggccgccat aagcctgaga acatcgtcat cgagatggcc    3600

cgcgagaacc agaccaccca gaagggccag aagaactccc gcgagcgcat gaagcgcatc 3660
gaggaaggca tcaaagagct gggcagccaa atcctcaaag agcatcctgt cgagaacacc 3720
cagctccaga acgagaagct ctacctctac tacctccaga acggccgcga tatgtacgtc 3780
gatcaagagc tggacatcaa ccgcctctcc gattacgatg tcgatcatat cgtccctcag 3840
tccttcctga aggatgattc catcgataac aaggtcctca cccgctccga taagaaccgc 3900
ggcaagtccg ataacgtccc ttccgaagag gtcgtcaaga agatgaagaa ctactggcgc 3960
cagctcctca acgccaagct catcacccag cgcaagttcg ataacctcac caaggccgag 4020
cgcggtggcc tctccgagct ggataaggcc ggcttcatca agcgccagct cgtcgaaacc 4080
cgccagatca ccaagcacgt cgcccaaatc ctcgattccc gcatgaacac caagtacgat 4140
gagaacgata agctcatccg cgaagtcaag gtcatcaccc tcaagtccaa gctcgtcagc 4200
gatttccgca aggatttcca gttctacaag gtccgcgaga tcaacaacta ccatcatgcc 4260
catgatgcct acctcaacgc cgtcgtcggc accgccctca tcaagaagta ccccaagctc 4320
gaatccgagt tcgtctacgg tgattacaag gtctacgatg tccgcaagat gatcgccaag 4380
tccgagcaag agatcggcaa ggctaccgcc aagtacttct tctactccaa catcatgaat 4440
ttcttcaaga ccgaaatcac cctcgccaac ggcgaaatcc gcaagcgccc tctcatcgag 4500
actaacggcg agactggcga gatcgtctgg gataagggcc gcgatttcgc caccgtccgc 4560
aaggtcctct ccatgcctca ggtcaacatc gtcaagaaaa ccgaggtcca gaccggcggc 4620
ttctccaaag agtccatcct ccccaagcgc aactccgata agctgatcgc ccgcaagaag 4680
gattgggacc ctaagaagta cggcggcttc gattccccta ccgtcgccta ctccgtcctc 4740
gtcgtcgcca aggtcgagaa gggcaagtcc aagaagctca agtccgtcaa agagctgctc 4800
ggcatcacta ttatggaacg ctccagcttc gagaagaacc ctatcgattt ccttgaggcc 4860
aagggctaca aagaggtcaa gaaggacctc atcatcaagc tccccaagta ctccctgttc 4920
gagcttgaga acggccgcaa gcgcatgctc gcctccgccg gtgagcttca gaagggcaac 4980
gagctggccc tgccttccaa gtacgtcaac ttcctctacc tcgcctccca ttacgagaag 5040
ctcaagggct cccctgagga taacgagcag aagcagctgt tcgtcgagca gcataagcac 5100
tacctcgatg agatcatcga gcagatcagc gagttctcca agcgcgtcat cctcgccgat 5160
gccaacctcg ataaggtcct gtccgcctac aacaagcacc gcgataagcc tatccgcgag 5220
caggccgaga acatcatcca tctcttcacc ctcaccaacc tcggtgcccc tgccgccttc 5280
aagtacttcg ataccaccat cgatcgcaag cgctacacct ccaccaaaga ggtcctggac 5340
gccaccctca tccatcagtc catcaccggc ctctacgaaa cccgcatcga tctctcccag 5400
ctcggcggcg accctccgag gaaacgtgcc aaaacagaag atgagtgatg aggatccact 5460
taacgttact gaaatcatca aacagcttga cgaatctgga tataagatcg ttggtgtcga 5520
tgtcagctcc ggagttgaga caaatggtgt tcaggatctc gataagatac gttcatttgt 5580
ccaagcagca aagagtgcct tctagtgatt taatagctcc atgtcaacaa gaataaaacg 5640
cgttttcggg tttacctctt ccagatacag ctcatctgca atgcattaat gcattgactg 5700
caacctagta acgccttnca ggctccggcg aagagaagaa tagcttagca gagctatttt 5760
cattttcggg agacgagatc aagcagatca acggtcgtca agagacctac gagactgagg 5820
aatccgctct tggctccacg cgactatata tttgtctcta attgtacttt gacatgctcc 5880
tcttctttac tctgatagct tgactatgaa aattccgtca ccagcncctg ggttcgcaaa 5940
gataattgca tgtttcttcc ttgaactctc aagcctacag gacacacatt catcgtaggt 6000
ataaacctcg aaatcanttc ctactaagat ggtatacaat agtaaccatg catggttgcc 6060
tagtgaatgc tccgtaacac ccaatacgcc ggccgaaact tttttacaac tctcctatga 6120
gtcgtttacc caga 6134

<210> 30
<211> 45
<212> DNA
<213> Primer
<400> 30
tctgcagatc tttaattaac tcgagcaccc gccatgattc cgtag 45

<210> 31
<211> 37
<212> DNA
<213> Primer
<400> 31
tttcagacat tttgacggta tttgtgttct gaagaac 37

<210> 32
<211> 567
<212> DNA
<213> Myceliophthora thermophila
<400> 32

aggatcggtg gagtgaagtt cggaatcgag gttcggcgat gggtcgtaag catggcgact 60
tcgaacttac ttgcactggc aagcgttgcc agaacggcga gaaaaagaag ggtaagcgat 120
attcgcgtca tgatggactg ttccttttgg aacagtagtt gttgtgggaa gactatgtca 180
cacttgccca cctgcaaggc cagggtcgtg gtcgaacgag accagcctcg gcgctgctgg 240
gagctcaaga tgggcacgtt tgattcgtta gacgtcaaca aggctggagt tcctagtgac 300
agccaaaggc acagccacat taagtggcgc tttatctgtc cactaaggtt caattgtggc 360
tttgagccgc gcagtgtgca gtcgtgcatt ggccacctag ctagcagtat ttaagatcct 420
cttctctccc gagatcttcc tcctcttctt ttctttcttt cctcggctac ctgcccgcca 480
acgagtttta gagctagaaa tagcaagtta aaataaggct agtccgttat caacttgaaa 540
aagtggcacc gagtcggtgc ttttttt 567

<210> 33
<211> 2373
<212> DNA
<213> Myceliophthora thermophila
<400> 33
gggtggtgtg catggggccg tctcggcggg gtgtgtgaac gcggggcaga actgagtgtt 60
cccgcggttc gacgggcctt caaagcaagc tgccccacga ccccgccaac tctctgagca 120
tgacttaggt acttaagcta ccaaagcttc gagtctgcaa catcgtgacc ttccaacttt 180
cccagcgact tacttgtttc aaccattgca tcactttcca ggtcagtgct ccagtctaat 240
attcgcactc tgcgaactcc aattgcattt ttacaatcga gagctgaccc tcagactgcg 300
agacgtcaac agttccccca cgatcctccg ctgcctccga tcctaactcc aactccaaac 360
aaaatggtca aagctggtaa gcacacgacg ccatcttctg ggcgatgtag gcatcaactg 420
gtctgacatt gttcctaaac agtcgttgct ggcgcctccg gcggtattgg acaggttcgt 480
atgccctcaa gctctctgga gccagcccgc tgacgccatc ggtaccccgc agccgctgtc 540
gctgctcctg aagctctctc cgctcgtcag cgagctcgca ctctacgatg ttgtcaacac 600
ccccggtgtt gcgaccgacc tgtcgcacat ttcatccaac gccgtaagtg gaaaagccgt 660
taaacccggc agcgttctca gacccggctg accctgctgc atattgaagg agcacttttt 720
gggcttggct ggagctagtg gaggtcaaca atgaatgcct attttggttt agtcgtccag 780
gcggtgagca caaaatttgt gtcgtttgac aagatggttc atttaggcaa ctggtcagat 840
cagccccact tgtagcagta gcggcggcgc tcgaagtgtg actcttatta gcagacagga 900
acgaggacat tattatcatc tgctgcttgg tgcacgataa cttggtgcgt ttgtcaagca 960
aggtaagtga acgacccggt cataccttct taagttcgcc cttcctccct ttatttcaga 1020
ttcaatctga cttacctatt ctacccaagc aaagcttcga ttaggaagta accatgagcc 1080
cagaacgacg cccggccgac atccgccgtg ccaccgaggc ggacatgccg gcggtctgca 1140
ccatcgtcaa ccactacatc gagacaagca cggtcaactt ccgtaccgag ccgcaggaac 1200
cgcaggagtg gacggacgac ctcgtccgtc tgcgggagcg ctatccctgg ctcgtcgccg 1260
aggtggacgg cgaggtcgcc ggcatcgcct acgcgggccc ctggaaggca cgcaacgcct 1320
acgactggac ggccgagtcg accgtgtacg tctccccccg ccaccagcgg acgggactgg 1380
gctccacgct ctacacccac ctgctgaagt ccctggaggc acagggcttc aagagcgtgg 1440
tcgctgtcat cgggctgccc aacgacccga gcgtgcgcat gcacgaggcg ctcggatatg 1500
ccccccgcgg catgctgcgg gcggccggct tcaagcacgg gaactggcat gacgtgggtt 1560
tctggcagct ggacttcagc ctgccggtac cgccccgtcc ggtcctgccc gtcaccgaga 1620
tctgatccgt cgacctgcag atcgttcaaa catttggcaa taaagtttct taaaagcccg 1680
gcatgacccg tgatgacctc ttcaacatca acgccggcat cgtgaagggc ctcattgagg 1740
tggccgccga ggtcgccccc aaggccttca tcctggttat ttccaacccc gtcaactcga 1800
ccgtccccat ctcggccgag gtgctcaagg ccaagggcgt cttcaacccc cagcgcctct 1860
tcggtgtcac caccctcgac attgtccgtg ccgagacctt cgttgccgag attgccggca 1920
agagcaaccc ccaggagttg actgtccccg tcgttggcgg tcactccggc gagaccattg 1980
ttccgctttt cagcaagatc gctccgtctg tcacgattcc ggacgacaag tacgacgctc 2040
ttgtcaaccg cgtccagttc ggtggtgatg aggttgtcaa ggccaaggac ggcgctggtt 2100
ctgccacact ttcgatggcg tatgctggtt acaggtaagc gccagacagt aaccaccctt 2160
cgacttctat ttttgctaac cgctatcaag gttcgccgag aagctcctca aggccgccgc 2220
gggcgtcaag ggcctcgttg aaccaagcta cgtctacctt cccggcatcc caggtggcaa 2280
ggagattgcc gagaagaccg gtgtcgagtt cttctccgtt cccatcgagc ttggcgtaag 2340
tcacaaagag gatagaggaa gagcttcagt acg 2373

<210> 34
<211> 567
<212> DNA
<213> Myceliophthora thermophila
<400> 34
aggatcggtg gagtgaagtt cggaatcgag gttcggcgat gggtcgtaag catggcgact 60

tcgaacttac ttgcactggc aagcgttgcc agaacggcga gaaaaagaag ggtaagcgat 120
attcgcgtca tgatggactg ttccttttgg aacagtagtt gttgtgggaa gactatgtca 180
cacttgccca cctgcaaggc cagggtcgtg gtcgaacgag accagcctcg gcgctgctgg 240
gagctcaaga tgggcacgtt tgattcgtta gacgtcaaca aggctggagt tcctagtgac 300
agccaaaggc acagccacat taagtggcgc tttatctgtc cactaaggtt caattgtggc 360
tttgagccgc gcagtgtgca gtcgtgcatt ggccacctag ctagcagtat ttaagatcct 420
cttctctccc gagatcttcc tcctcttctt ttctttcttt cctcggagaa tgtcacaatc 480
cccggtttta gagctagaaa tagcaagtta aaataaggct agtccgttat caacttgaaa 540
aagtggcacc gagtcggtgc ttttttt 567

<210> 35
<211> 1400
<212> DNA
<213> Myceliophthora thermophila
<400> 35
tgggacggcg agagcgacgg gttttggccg aggtgggcgg gttcgtcaac ggccttggcg 60
ctgaacgact gaaggcggtc aatgttttct gccgcccctt gcgtcatggc tcgcagaatg 120
ggccaacgga atttcgtacc aggaggaaaa tttccttgtt tggcggaaaa ccgccgggag 180
ggggtcgtct gatgaaccac gaggcgatgc acccagaagg ctgcacaggt agagcgtttt 240
tgcctacata gctgtgttca gttttcttga gtttgctgtg taagggtcca ggatggtacg 300
gggcgggctt ttgcggccgc ccctactgcc cacataccag agcagcggtg caattttgtc 360
ggcgttttcc tgcgacccat cgtccctccc cagcgtcaga caccggtcac cgcctgcctt 420
tttttctacg cccaacgtga cccatttcca ccacaggcct acccacttaa gaggctgcac 480
gttccgactg cccaagccca aaccttgtca gccaagtcgg ttcttcatat gccaagcgtc 540
tcaccgtccc aatctttctt cctgctctcg tccgaagtct ttgccggcaa tccgtggccg 600
ctctctcaaa cagacaaact cggctcccgc tctcattccc cctcgcagca tccgtctgtt 660
atcgcacaca gcaaaaatgg cacgatccac agactctgcc ttctcgacgc cgtcagggac 720
tcttccattc ttatcttcaa cctcccgcac gagttcatcg gcaacatttg caagcaaatc 780
aagggtcata tcctcccctt tgcccgggga atcagctgca tcaagggggt aacagtgacg 840
gaggacaagg tcgagctgat ttgcgagtat attggcgaga cgctgggtat ttactgcggc 900
gccctgagcg gggccaacat tgccaatgag atcgccaacg agctatggtg cgagacgacc 960
atcgcataca acgtaccacc gtgcgactcg cggggcgaga acggcgagag cggcgtcaat 1020
ggagagcact atcgtgacgc gcgcggtgca atcagcaaaa cgcagctcgc tcccgtacct 1080
caggagtacc cgccagtgga ccatgacgtg ctccacaagc tctttaaccg gccttatttt 1140
accgtctcca tggtgtcgga cgtggttggt gtctcgctcg ccggtgcgct caagaacatc 1200
gttgcgctcg cctgcggctt cattgaaggt cacggttgga acatgacggc taagacggcc 1260
gttatgcgcc gtggcatgct ggagacgatt cagttctgcc aagaattctt ccccgagacg 1320
gtcgacgaga tgacgttctg ggagagcgcc ggctggagcg acatgattgt gagctgcacc 1380
tcggcacgca actggcgcta 1400

<210> 36
<211> 40
<212> DNA
<213> Primer
<400> 36
aagaggagtc caccatggta ctcgacagaa gatgatattg 40

<210> 37
<211> 28
<212> DNA
<213> Primer
<400> 37
aacgttaagt tcagatctcg gtgacggg 28

<210> 38
<211> 4227
<212> DNA
<213> Synthetic fragment
<400> 38
aaggttgata agaagtactc catcggcctc gccatcggca ccaactccgt cggctgggcc 60
gtcatcaccg atgagtacaa ggtcccttcc aagaagttca aggtcctcgg caacaccgat 120
cgccattcca tcaagaagaa cctgatcggc gccctcctgt tcgattccgg cgaaaccgcc 180
gaggccaccc gccttaaacg caccgcccgt cgccgctaca cccgccgcaa gaaccgcatc 240

tgctacctcc aagaaatctt ctccaacgag atggccaagg tcgatgatag cttcttccac 300
cgcctcgaag agtccttcct ggtcgaagag gataagaagc acgagcgcca tcctatcttc 360
ggcaacatcg tcgatgaggt cgcctaccat gagaagtacc ctaccatcta ccatctccgc 420
aagaagctcg tcgattccac cgataaggcc gatctccgcc tcatctacct cgccctcgcc 480
catatgatca agttccgcgg ccatttcctc atcgagggcg atctcaaccc tgataactcc 540
gatgtcgata agctgttcat ccagctcgtc cagacctaca accagctgtt cgaggaaaac 600
cctatcaacg cctccggcgt cgatgccaag gccatcctct ccgctcgcct ctccaagtct 660
cgccgccttg agaaccttat cgcccagctc cctggcgaga agaagaacgg cctcttcggc 720
aacctgatcg ccctctccct cggcctcacc cctaacttca agtccaactt cgatctcgcc 780
gaggatgcca agctccagct ctccaaggat acctacgatg atgatctcga taacctcctc 840
gcccagatcg gcgatcagta cgccgatctg ttcctcgccg ccaagaacct ctccgatgcc 900
atcctcctct ccgacatcct ccgcgtcaac accgagatca ccaaggcccc tctgtccgcc 960
tccatgatca agcgctacga tgagcatcat caggacctca ccctgctcaa ggccctcgtc 1020
cgccagcagc tccctgagaa gtacaaagag attttcttcg atcagtccaa gaacggctac 1080
gccggctaca tcgatggcgg cgcttcccaa gaagagttct acaagttcat caagcctatc 1140
cttgagaaga tggatggcac cgaggaactc ctcgtcaagc tcaaccgcga ggacctcctc 1200
cgcaagcagc gcaccttcga taacggctcc atccctcatc aaatccatct cggcgagctg 1260
catgccatct tgcgccgcca agaggatttc tacccattcc tcaaggataa ccgcgagaag 1320
atcgaaaaga ttctcacctt ccgcatccct tactacgtcg gccctctcgc tcgcggcaac 1380
tcccgcttcg cctggatgac ccgcaagtcc gaggaaacca tcaccccttg gaacttcgag 1440
gaagtcgtcg ataagggcgc ctccgcccag tccttcatcg agcgcatgac caacttcgat 1500
aagaacctcc ctaacgagaa ggtcctccct aagcactccc tgctctacga gtacttcacc 1560
gtctacaacg agctgaccaa ggtcaagtac gtcaccgagg gtatgcgcaa gcctgccttc 1620
ctgtccggcg agcagaagaa ggccatcgtc gatctgctgt tcaagaccaa ccgcaaggtc 1680
accgtcaagc agctcaaaga ggattacttc aagaaaatcg agtgcttcga ttccgtcgag 1740
atcagcggcg tcgaggaccg cttcaacgcc tccctcggaa cctaccatga tctcctcaag 1800
attatcaagg ataaggattt cctcgacaac gaggaaaacg aggacatcct tgaggacatc 1860
gtcctcaccc tcaccctctt cgaggaccgc gaaatgatcg aggaacgcct caagacctac 1920
gcccatctct tcgatgataa ggtcatgaag cagctcaagc gccgtcgcta caccggctgg 1980
ggtcgcctct cccgcaagct catcaacggc atccgcgata agcagtccgg caagactatc 2040
ctcgatttcc tcaagtccga tggcttcgcc aaccgcaact tcatgcagct catccatgat 2100
gattccctca ccttcaaaga ggacatccag aaggcccagg tcagcggcca gggcgattcc 2160
ctccatgagc atatcgccaa cctcgccggc tcccctgcca tcaagaaggg catcctccag 2220
accgtcaagg tcgtcgatga gctggtcaag gtcatgggcc gccataagcc tgagaacatc 2280
gtcatcgaga tggcccgcga gaaccagacc acccagaagg gccagaagaa ctcccgcgag 2340
cgcatgaagc gcatcgagga aggcatcaaa gagctgggca gccaaatcct caaagagcat 2400
cctgtcgaga acacccagct ccagaacgag aagctctacc tctactacct ccagaacggc 2460
cgcgatatgt acgtcgatca agagctggac atcaaccgcc tctccgatta cgatgtcgat 2520
gctatcgtcc ctcagtcctt cctgaaggat gattccatcg ataacaaggt cctcacccgc 2580
tccgataaga accgcggcaa gtccgataac gtcccttccg aagaggtcgt caagaagatg 2640
aagaactact ggcgccagct cctcaacgcc aagctcatca cccagcgcaa gttcgataac 2700
ctcaccaagg ccgagcgcgg tggcctctcc gagctggata aggccggctt catcaagcgc 2760
cagctcgtcg aaacccgcca gatcaccaag cacgtcgccc aaatcctcga ttcccgcatg 2820
aacaccaagt acgatgagaa cgataagctc atccgcgaag tcaaggtcat caccctcaag 2880
tccaagctcg tcagcgattt ccgcaaggat ttccagttct acaaggtccg cgagatcaac 2940
aactaccatc atgcccatga tgcctacctc aacgccgtcg tcggcaccgc cctcatcaag 3000
aagtacccca agctcgaatc cgagttcgtc tacggtgatt acaaggtcta cgatgtccgc 3060
aagatgatcg ccaagtccga gcaagagatc ggcaaggcta ccgccaagta cttcttctac 3120
tccaacatca tgaatttctt caagaccgaa atcaccctcg ccaacggcga aatccgcaag 3180
cgccctctca tcgagactaa cggcgagact ggcgagatcg tctgggataa gggccgcgat 3240
ttcgccaccg tccgcaaggt cctctccatg cctcaggtca acatcgtcaa gaaaaccgag 3300
gtccagaccg gcggcttctc caaagagtcc atcctcccca agcgcaactc cgataagctg 3360
atcgcccgca agaaggattg ggaccctaag aagtacggcg gcttcgattc ccctaccgtc 3420
gcctactccg tcctcgtcgt cgccaaggtc gagaagggca agtccaagaa gctcaagtcc 3480
gtcaaagagc tgctcggcat cactattatg gaacgctcca gcttcgagaa gaaccctatc 3540
gatttccttg aggccaaggg ctacaaagag gtcaagaagg acctcatcat caagctcccc 3600
aagtactccc tgttcgagct tgagaacggc cgcaagcgca tgctcgcctc cgccggtgag 3660
cttcagaagg gcaacgagct ggccctgcct tccaagtacg tcaacttcct ctacctcgcc 3720
tcccattacg agaagctcaa gggctcccct gaggataacg agcagaagca gctgttcgtc 3780
gagcagcata agcactacct cgatgagatc atcgagcaga tcagcgagtt ctccaagcgc 3840
gtcatcctcg ccgatgccaa cctcgataag gtcctgtccg cctacaacaa gcaccgcgat 3900
aagcctatcc gcgagcaggc cgagaacatc atccatctct tcaccctcac caacctcggt 3960
gcccctgccg ccttcaagta cttcgatacc accatcgatc gcaagcgcta cacctccacc 4020

aaagaggtcc tggacgccac cctcatccat cagtccatca ccggcctcta cgaaacccgc 4080
atcgatctct cccagctcgg cggcgaccct ccgaggaaac gtgccaaaac agaagatgag 4140
agcgctggag gaggtggaag cggaggagga ggaagcggag gaggaggtag cggacctaag 4200
aaaaagagga aggtggcggc cgcttaa 4227

<210> 39
<211> 32
<212> DNA
<213> Primer
<400> 39
cgagatctga acttaacgtt actgaaatca tc 32

<210> 40
<211> 273
<212> DNA
<213> Synthetic sequence
<400> 40
atggatgcta agtcactaac tgcctggtcc cggacactgg tgaccttcaa ggatgtattt 60
gtggacttca ccagggagga gtggaagctg ctggacactg ctcagcagat cgtgtacaga 120
aatgtgatgc tggagaacta taagaacctg gtttccttgg gttatcagct tactaagcca 180
gatgtgatcc tccggttgga gaagggagaa gagccctggc tggtggagag agaaattcac 240
caagagaccc atcctgattc agagactgca tga 273

<210> 41
<211> 38
<212> DNA
<213> Primer
<400> 41
gctagcgtta acactagtca cctctaaaca agtgtacc 38

<210> 42
<211> 567
<212> DNA
<213> Myceliophthora thermophila
<400> 42
aggatcggtg gagtgaagtt cggaatcgag gttcggcgat gggtcgtaag catggcgact 60
tcgaacttac ttgcactggc aagcgttgcc agaacggcga gaaaaagaag ggtaagcgat 120
attcgcgtca tgatggactg ttccttttgg aacagtagtt gttgtgggaa gactatgtca 180
cacttgccca cctgcaaggc cagggtcgtg gtcgaacgag accagcctcg gcgctgctgg 240
gagctcaaga tgggcacgtt tgattcgtta gacgtcaaca aggctggagt tcctagtgac 300
agccaaaggc acagccacat taagtggcgc tttatctgtc cactaaggtt caattgtggc 360
tttgagccgc gcagtgtgca gtcgtgcatt ggccacctag ctagcagtat ttaagatcct 420
cttctctccc gagatcttcc tcctcttctt ttctttcttt cctcgggctc agcacaaagt 480
ctgggtttta gagctagaaa tagcaagtta aaataaggct agtccgttat caacttgaaa 540
aagtggcacc gagtcggtgc ttttttt 567

<210> 43
<211> 31
<212> DNA
<213> Primer
<400> 43
gcagatctca tgtaccttga cgtcctccga g 31

<210> 44
<211> 33
<212> DNA
<213> Primer
<400> 44
aaacactagt tctgaagaac gaaactggcg act 33

<210> 45
<211> 40
<212> DNA

<213>  Primer
<400>  45
tttaaacgat atcgaattcg agcatacaat caactatctc                    40

<210>  46
<211>  39
<212>  DNA
<213>  Primer
<400>  46
gatttcagta acgttaagtg ctttaaaatt tgtatacac                     39

<210>  47
<211>  43
<212>  DNA
<213>  Primer
<400>  47
ccacatcaca gaaatcaaaa ctagtatgtt cctgggcagc cgc                43

<210>  48
<211>  35
<212>  DNA
<213>  Primer
<400>  48
tttctgggat agacataccg agaacggcga ccttg                         35

<210>  49
<211>  29
<212>  DNA
<213>  Primer
<400>  49
cgttctcggt atgtctatcc cagaaactc                                29

<210>  50
<211>  50
<212>  DNA
<213>  Primer
<400>  50
tgttaataac ccacgcgtgg gcggatcctt atttagaagt gtcaacaacg         50

<210>  51
<211>  38
<212>  DNA
<213>  Primer
<400>  51
agccatggag aggtttagca cgaacctctc tgaaggag                      38

<210>  52
<211>  19
<212>  DNA
<213>  Primer
<400>  52
gtatgatggg tcagttcag                                           19

<210>  53
<211>  27
<212>  DNA
<213>  Primer
<400>  53
cagccgcatc cagacccgcg ccttctc                                  27

<210>  54
<211>  18
<212>  DNA

<213>  Primer
<400>  54
atgggtctct tctcgaaa                                                    18

<210>  55
<211>  18
<212>  DNA
<213>  Primer
<400>  55
ctaaacctct ccatggct                                                    18

<210>  56
<211>  38
<212>  DNA
<213>  Primer
<400>  56
aagaggagtc caccatggta cttgcatcgt cccaaagc                              38

<210>  57
<211>  38
<212>  DNA
<213>  Primer
<400>  57
tttcgagaag agacccattt tgatttctgt gatgtggg                              38

<210>  58
<211>  37
<212>  DNA
<213>  Primer
<400>  58
tcacagaaat caaaactaga atgtcgtctc acggctc                               37

<210>  59
<211>  46
<212>  DNA
<213>  Primer
<400>  59
ttaagtggat ccgaattcga tatcctaagc aacgatagct tcggac                     46

<210>  60
<211>  38
<212>  DNA
<213>  Primer
<400>  60
aagaggagtc caccatggta caggatattg ataacacc                              38

<210>  61
<211>  38
<212>  DNA
<213>  Primer
<400>  61
cgatctctag aagtcctcgt cccgagccaa acacttgg                              38

<210>  62
<211>  38
<212>  DNA
<213>  Primer
<400>  62
caggtacact tgtttagagg ccgctggaca ggttcgtg                              38

<210>  63
<211>  38
<212>  DNA

<213>  Primer
<400>  63
gctagcgtta acactagtca gcccatctgt tgttaacc                              38

<210>  64
<211>  37
<212>  DNA
<213>  Primer
<400>  64
acgaggactt ctagagatcg ttgcatcgtc ccaaagc                               37

<210>  65
<211>  20
<212>  DNA
<213>  Primer
<400>  65
cctctaaaca agtgtacctg                                                  20

<210>  66
<211>  19
<212>  DNA
<213>  Primer
<400>  66
tcaagtccag catagcgac                                                   19

<210>  67
<211>  20
<212>  DNA
<213>  Primer
<400>  67
cctctaaaca agtgtacctg                                                  20

<210>  68
<211>  38
<212>  DNA
<213>  Primer
<400>  68
aagaggagtc caccatggta cgtcaggtgt ctgttctc                              38

<210>  69
<211>  37
<212>  DNA
<213>  Primer
<400>  69
gtcgctatgc tggacttgag tacgcagtcg tcacgcc                               37

<210>  70
<211>  38
<212>  DNA
<213>  Primer
<400>  70
caggtacact tgtttagagg tattccggga cgtagtgc                              38

<210>  71
<211>  38
<212>  DNA
<213>  Primer
<400>  71
gctagcgtta acactagtca ctccaactcg ctgaggag                              38

<210>  72
<211>  31
<212>  DNA

<213>  Primer
<400>  72
ttatttagaa gtgtcaacaa cgtatctacc a                                    31

<210>  73
<211>  30
<212>  DNA
<213>  Primer
<400>  73
ccggcgcatg atgtctgaaa ttactttggg                                      30

<210>  74
<211>  45
<212>  DNA
<213>  Primer
<400>  74
gatttcagta acgttaagtg gatccttatt gcttagcgtt ggtag                     45

<210>  75
<211>  44
<212>  DNA
<213>  Primer
<400>  75
ccacatcaca gaaatcaaaa ctagtatgtt ggcttctcgt cagc                      44

<210>  76
<211>  20
<212>  DNA
<213>  Primer
<400>  76
agtttatgtg gaaaatggca                                                 20

<210>  77
<211>  38
<212>  DNA
<213>  Primer
<400>  77
ctcgtagtcg ccgacggact atccgttggg cggagcat                             38

<210>  78
<211>  36
<212>  DNA
<213>  Primer
<400>  78
ccaacggata gtccgtcggc gactacgagg actgcg                               36

<210>  79
<211>  20
<212>  DNA
<213>  Primer
<400>  79
tacgtgccga ccctcgagac                                                 20

<210>  80
<211>  25
<212>  DNA
<213>  Primer
<400>  80
tcctccgagg ttcgacatca gggtt                                           25

<210>  81
<211>  24
<212>  DNA

<213>  Primer
<400>  81
ctctaaacaa gtgtacctgt gcat                                              24

<210>  82
<211>  20
<212>  DNA
<213>  Primer
<400>  82
agtttatgtg gaaaatggca                                                   20

<210>  83
<211>  20
<212>  DNA
<213>  Primer
<400>  83
tacgtgccga ccctcgagac                                                   20

<210>  84
<211>  31
<212>  DNA
<213>  Primer
<400>  84
acggatagtc atattgaagg agcacttttt g                                     31

<210>  85
<211>  29
<212>  DNA
<213>  Primer
<400>  85
gtcgccgacg gatcactagt acaggattc                                        29

<210>  86
<211>  22
<212>  DNA
<213>  Primer
<400>  86
cggattcaag atgcctcagc ca                                                22

<210>  87
<211>  20
<212>  DNA
<213>  Primer
<400>  87
gaccgggttg gtggccacga                                                   20

<210>  88
<211>  21
<212>  DNA
<213>  Primer
<400>  88
cctgactgta taagagaaat g                                                 21

<210>  89
<211>  40
<212>  DNA
<213>  Primer
<400>  89
agcgaccgga gtaagctccc gccgtggcaa tttcccgaac                             40

<210>  90
<211>  37
<212>  DNA

<213>  Primer
<400>  90
aaattgccac ggcgggagct tactccggtc gctgtca                                37

<210>  91
<211>  23
<212>  DNA
<213>  Primer
<400>  91
ccaatgaagc gttccttacg gct                                               23

<210>  92
<211>  22
<212>  DNA
<213>  Primer
<400>  92
aggatcggtg gagtgaagtt cg                                                22

<210>  93
<211>  40
<212>  DNA
<213>  Primer
<400>  93
acggagcagg taaccaagtc gaggaaagaa agaaaagaag                             40

<210>  94
<211>  40
<212>  DNA
<213>  Primer
<400>  94
gacttggtta cctgctccgt gttttagagc tagaaatagc                             40

<210>  95
<211>  23
<212>  DNA
<213>  Primer
<400>  95
aaaaaaagca ccgactcggt gcc                                               23

<210>  96
<211>  23
<212>  DNA
<213>  Primer
<400>  96
tgactgaccg tgatgatttc cag                                               23

<210>  97
<211>  22
<212>  DNA
<213>  Primer
<400>  97
agcctaacgt cgagaccggc gt                                                22

<210>  98
<211>  23
<212>  DNA
<213>  Primer
<400>  98
gggtggtgtg catggggccg tct                                               23

<210>  99
<211>  39
<212>  DNA

<213>  Primer
<400>  99
aaagtgctcc ttcaatatgc agcagggtca gccgggtct                                39

<210>  100
<211>  40
<212>  DNA
<213>  Primer
<400>  100
cggctgaccc tgctgcatat tgaaggagca ctttttgggc                               40

<210>  101
<211>  35
<212>  DNA
<213>  Primer
<400>  101
ggtcatgccg ggcttttaag aaactttatt gccaa                                    35

<210>  102
<211>  41
<212>  DNA
<213>  Primer
<400>  102
ggcaataaag tttcttaaaa gcccggcatg acccgtgatg a                             41

<210>  103
<211>  25
<212>  DNA
<213>  Primer
<400>  103
cgtactgaag ctcttcctct atcct                                               25

<210>  104
<211>  39
<212>  DNA
<213>  Primer
<400>  104
 ttggcgggca ggtagccgag gaaagaaaga aaagaagag                                39

<210>  105
<211>  40
<212>  DNA
<213>  Primer
<400>  105
ctacctgccc gccaacgagt tttagagcta gaaatagcaa                                40

<210>  106
<211>  22
<212>  DNA
<213>  Primer
<400>  106
aggatcggtg gagtgaagtt cg                                                  22

<210>  107
<211>  23
<212>  DNA
<213>  Primer
<400>  107
aaaaaaagca ccgactcggt gcc                                                 23

<210>  108
<211>  28
<212>  DNA

<213>  Primer
<400>  108
tttcagacat catgcgccgg agaccaag                                          28

<210>  109
<211>  24
<212>  DNA
<213>  Primer
<400>  109
atgtctgaaa ttactttggg taaa                                              24

<210>  110
<211>  22
<212>  DNA
<213>  Primer
<400>  110
tgggacggcg agagcgacgg gt                                                22

<210>  111
<211>  31
<212>  DNA
<213>  Primer
<400>  111
gcgtcgagaa ggcagagtct gtggatcgtg c                                      31

<210>  112
<211>  31
<212>  DNA
<213>  Primer
<400>  112
agactctgcc ttctcgacgc cgtcaggg                                          28

<210>  113
<211>  23
<212>  DNA
<213>  Primer
<400>  113
tagcgccagt tgcgtgccga ggt                                               23

<210>  114
<211>  22
<212>  DNA
<213>  Primer
<400>  114
cggggattgt gacattctcc gaggaaagaa agaaaagaag                             40

<210>  115
<211>  23
<212>  DNA
<213>  Primer
<400>  115
ggagaatgtc acaatccccg gttttagagc tagaaatagc                             40

<210>  116
<211>  22
<212>  DNA
<213>  Primer
<400>  116
aggatcggtg gagtgaagtt cg                                                22

<210>  117
<211>  23
<212>  DNA

<213>  Primer
<400>  117
aaaaaaagca ccgactcggt gcc                                              23

<210>  118
<211>  21
<212>  DNA
<213>  Primer
<400>  118
cgctctcatt ccccctcgca g                                                21

<210>  119
<211>  22
<212>  DNA
<213>  Primer
<400>  119
atatactcgc aaatcagctc ga                                               22

<210>  120
<211>  62
<212>  DNA
<213>  Primer
<400>  120
aagcttatgg accccaagaa gaaacgcaag gttgataaga agtactccat cggcctcgcc
at                                                                     62

<210>  121
<211>  32
<212>  DNA
<213>  Primer
<400>  121
tgagggacga tagcatcgac atcgtaatcg ga                                    32

<210>  122
<211>  32
<212>  DNA
<213>  Primer
<400>  122
cgatgtcgat gctatcgtcc ctcagtcctt cc                                    32

<210>  123
<211>  23
<212>  DNA
<213>  Primer
<400>  123
agcggccgcc accttcctct ttt                                              23

<210>  124
<211>  33
<212>  DNA
<213>  Primer
<400>  124
gggtttaaac atggacccca agaagaaacg caa                                   33

<210>  125
<211>  28
<212>  DNA
<213>  Primer
<400>  125
tagcatccat agcggccgcc accttcct                                         28

<210>  126
<211>  29

<212>  DNA
<213>  Primer
<400>  126
cggccgctat ggatgctaag tcactaact                                        29

<210>  127
<211>  34
<212>  DNA
<213>  Primer
<400>  127
ccttaattaa tgcagtctct gaatcaggat gggt                                  34

<210>  128
<211>  29
<212>  DNA
<213>  Primer
<400>  128
ccggatctcg gatcactagt acaggattc                                        29

<210>  129
<211>  29
<212>  DNA
<213>  Primer
<400>  129
actagtgatc cgagatccgg ggtgacacc                                        29

<210>  130
<211>  22
<212>  DNA
<213>  Primer
<400>  130
cagtcgccca cggcccagat gt                                               22

<210>  131
<211>  22
<212>  DNA
<213>  Primer
<400>  131
aggatcggtg gagtgaagtt cg                                               22

<210>  132
<211>  37
<212>  DNA
<213>  Primer
<400>  132
cggcgctctg gatgatcgag gaaagaaaga aaagaag                               37

<210>  133
<211>  35
<212>  DNA
<213>  Primer
<400>  133
catccagagc gccggcagtt ttagagctag aaata                                 35

<210>  134
<211>  23
<212>  DNA
<213>  Primer
<400>  134
aaaaaaagca ccgactcggt gcc                                              23

<210>  135
<211>  22

<212>  DNA
<213>  Primer
<400>  135
tcaagtgtca tgatctcgcg tg                                              22

<210>  136
<211>  28
<212>  DNA
<213>  Primer
<400>  136
tcatcttctg ggtttggatg aatggatg                                        28

<210>  137
<211>  31
<212>  DNA
<213>  Primer
<400>  137
catccaaacc cagaagatga tattgaagga g                                    31

<210>  138
<211>  32
<212>  DNA
<213>  Primer
<400>  138
cgcaacttga gaaagaagga ttacctctaa ac                                   32

<210>  139
<211>  28
<212>  DNA
<213>  Primer
<400>  139
tccttctttc tcaagttgcg ggcgcgt                                         28

<210>  140
<211>  23
<212>  DNA
<213>  Primer
<400>  140
aggactatta tcatcctggg gga                                             23

<210>  141
<211>  28
<212>  DNA
<213>  Primer
<400>  141
ccggcgcatg atggccacca ccaagacc                                        28

<210>  142
<211>  38
<212>  DNA
<213>  Primer
<400>  142
gatgggcgtc ttgagctcga ggaaagaaag aaaagaag                             38

<210>  143
<211>  39
<212>  DNA
<213>  Primer
<400>  143
gctcaagacg cccatcacgt tttagagcta gaaatagca                            39

<210>  144
<211>  567

<212>  DNA
<213>  Myceliophthora thermophila
<400>  144
aggatcggtg gagtgaagtt cggaatcgag gttcggcgat gggtcgtaag catggcgact      60
tcgaacttac ttgcactggc aagcgttgcc agaacggcga gaaaaagaag ggtaagcgat     120
attcgcgtca tgatggactg ttccttttgg aacagtagtt gttgtgggaa gactatgtca     180
cacttgccca cctgcaaggc cagggtcgtg gtcgaacgag accagcctcg gcgctgctgg     240
gagctcaaga tgggcacgtt tgattcgtta gacgtcaaca aggctggagt tcctagtgac     300
agccaaaggc acagccacat taagtggcgc tttatctgtc cactaaggtt caattgtggc     360
tttgagccgc gcagtgtgca gtcgtgcatt ggccacctag ctagcagtat ttaagatcct     420
cttctctccc gagatcttcc tcctcttctt ttctttcttt cctcgattgg cttctatcag     480
ggctgtttta gagctagaaa tagcaagtta aaataaggct agtccgttat caacttgaaa     540
aagtggcacc gagtcggtgc ttttttt                                         567

<210>  145
<211>  2266
<212>  DNA
<213>  Myceliophthora thermophila
<400>  145
cgggctcagc cggaacttgc tacctctgta aactttcgac tgtggatttt ggcgctcgct      60
tctgttcgtg gacgccccat gacatcagtg aacaatttca taaaccccca ccatgttcag     120
acttttgtca aacctcttct gtcctttggt tctttcgttt attatttttt ttttccgtaa     180
aatgcatctt tggggacaca ggtagtttac acacagcctc gggttgaata tgtgaatgag     240
cctcgtgata tctatccagg agttggtagc ggaaagcagc gttgactgaa ccgggatatt     300
aagccgccga tatcaccttt atctgcctcg aagatgcccc tttacggtcc taacatgact     360
tggtgccgcc aacttgtagg ccggacaaac gctccaagac gatgctttag tatcgctaaa     420
agtaagtact ttcggcttcg aaccaagtgc gtccgtctcg ttaaccgatc ccaccagaca     480
atggcacaca caaggagcgg gtagtgatcc ttggctcagg ttgggcaggg tatggtttcg     540
ctcgcactct tgacccagcc aagtacgagc gcattatcat ctcgccacgc tcctactttg     600
tcttcacccc gttgctagcc atattgaagg agcacttttt gggcttggct ggagctagtg     660
gaggtcaaca atgaatgcct attttggttt agtcgtccag gcggtgagca caaaatttgt     720
gtcgtttgac aagatggttc atttaggcaa ctggtcagat cagccccact tgtagcagta     780
gcggcggcgc tcgaagtgtg actcttatta gcagacagga acgaggacat tattatcatc     840
tgctgcttgg tgcacgataa cttggtgcgt ttgtcaagca aggtaagtga acgacccggt     900
cataccttct taagttcgcc cttcctccct ttatttcaga ttcaatctga cttacctatt     960
ctacccaagc aaagcttcga ttaggaagta accatgagcc cagaacgacg cccggccgac    1020
atccgccgtg ccaccgaggc ggacatgccg gcggtctgca ccatcgtcaa ccactacatc    1080
gagacaagca cggtcaactt ccgtaccgag ccgcaggaac cgcaggagtg gacggacgac    1140
ctcgtccgtc tgcgggagcg ctatccctgg ctcgtcgccg aggtggacgg cgaggtcgcc    1200
ggcatcgcct acgcgggccc ctggaaggca cgcaacgcct acgactggac ggccgagtcg    1260
accgtgtacg tctccccccg ccaccagcgg acgggactgg gctccacgct ctacacccac    1320
ctgctgaagt ccctggaggc acagggcttc aagagcgtgg tcgctgtcat cgggctgccc    1380
aacgacccga gcgtgcgcat gcacgaggcg ctcggatatg ccccccgcgg catgctgcgg    1440
gcggccggct tcaagcacgg gaactggcat gacgtgggtt tctggcagct ggacttcagc    1500
ctgccggtac cgccccgtcc ggtcctgccc gtcaccgaga tctgatccgt cgacctgcag    1560
atcgttcaaa catttggcaa taaagtttct taagattgaa tcctgtacta gtgatccggt    1620
catccccgct ccttctccct catcatcatc agaaaccggc gggccgggca aggcacccaa    1680
gggcgacctc atcgagatcc cttacgacaa gcttgtgatc gcttgcggcg cgtacagcca    1740
aacctttggc atcgagggag tccgcgaata tgcacacttc ctccgcgaca tcggcgacgc    1800
ccggcgcatc cgccttcgcg tcctctccct ctttgagcag tgcgcctatc cccgcgggtc    1860
cgaccacctc agcgacgacg acaagcgcca gctcctccac ttcgccatcg tgggcggcgg    1920
accgaccggc atcgaattcg ccgccgagct ccacgacctt atccgcgatg acctcgcccc    1980
catctacccc gagctcgccc cgctggtcag catcacggtc tacgacgtgg cccccaaggt    2040
tcttccaatg ttcgaccggg cgctcgcaaa gtacgccatg gacaccttct cacgacaaaa    2100
catcaaggtc aagaccgagc accatctcga gcgcctccgc ctggccgacg gggagctcgg    2160
ccgccgccgc ggagtcctca agatcaagat caaggagtac ggcgacgagg aagtcggcgc    2220
gggcctggtc gtctggagca caggcctcat gccaaacccc ttgatc                   2266

<210>  146
<211>  567
<212>  DNA
<213>  Myceliophthora thermophila
<400>  146

aggatcggtg gagtgaagtt cggaatcgag gttcggcgat gggtcgtaag catggcgact 60
tcgaacttac ttgcactggc aagcgttgcc agaacggcga gaaaaagaag ggtaagcgat 120
attcgcgtca tgatggactg ttccttttgg aacagtagtt gttgtgggaa gactatgtca 180
cacttgccca cctgcaaggc cagggtcgtg gtcgaacgag accagcctcg gcgctgctgg 240
gagctcaaga tgggcacgtt tgattcgtta gacgtcaaca aggctggagt tcctagtgac 300
agccaaaggc acagccacat taagtggcgc tttatctgtc cactaaggtt caattgtggc 360
tttgagccgc gcagtgtgca gtcgtgcatt ggccacctag ctagcagtat ttaagatcct 420
cttctctccc gagatcttcc tcctcttctt ttctttcttt cctcgaccat cctccgccag 480
aagagtttta gagctagaaa tagcaagtta aaataaggct agtccgttat caacttgaaa 540
aagtggcacc gagtcggtgc ttttttt 567

<210> 147
<211> 2341
<212> DNA
<213> Myceliophthora thermophila
<400> 147
tcctcctccc tctctcaggc gccctccgcc gtgtcctacc tcgcgcgctc gtcaattgag 60
ccaatggcat ccacgtcgag agctctgagc aagctcgcgg tgccccaaac gggcgccgtt 120
atgcttcagc cgtcttcgtc cgcgcccaga ctgctcctcc agacatcgcg caggaccgta 180
ggcgcgaggc ccgtcttcgg cagctcacaa gggcccgctc tccggcagtt ccggcgcggc 240
gtcaccagcg acgccgcccc cgtcccgccg cccaagaagc ggcgattccg caagctgcgg 300
tgggcatggc ggttcacata cctctccgcc atcgccagca tcgcctacat cggctacggc 360
gtctaccagg acaggcaccc ggagccccag gtcgagcccg acccgtccaa gaagaccttg 420
gtgattctcg gtaagttttg gccccgtccc gtcatcccgt cacaccgcac tgttaaagaa 480
gcttgttccg cggttggggg gaatgtagaa agggaaaaaa aaaaagagag agagagagag 540
agagaaaata ttctccgggg ctaacaagca tgtgccgcag gaaccggctg gggctctgtc 600
tcgctgctca agcgcctcga cacggagaac tacaacgtca tcgtcatctc cccgcgcaac 660
tatattgaag gagcactttt tgggcttggc tggagctagt ggaggtcaac aatgaatgcc 720
tattttggtt tagtcgtcca ggcggtgagc acaaaatttg tgtcgtttga caagatggtt 780
catttaggca actggtcaga tcagccccac ttgtagcagt agcggcggcg ctcgaagtgt 840
gactcttatt agcagacagg aacgaggaca ttattatcat ctgctgcttg gtgcacgata 900
acttggtgcg tttgtcaagc aaggtaagtg aacgacccgg tcataccttc ttaagttcgc 960
ccttcctccc tttatttcag attcaatctg acttacctat tctacccaag caaagcttcg 1020
attaggaagt aaccatgagc ccagaacgac gcccggccga catccgccgt gccaccgagg 1080
cggacatgcc ggcggtctgc accatcgtca accactacat cgagacaagc acggtcaact 1140
tccgtaccga gccgcaggaa ccgcaggagt ggacggacga cctcgtccgt ctgcgggagc 1200
gctatccctg gctcgtcgcc gaggtggacg gcgaggtcgc cggcatcgcc tacgcgggcc 1260
cctggaaggc acgcaacgcc tacgactgga cggccgagtc gaccgtgtac gtctcccccc 1320
gccaccagcg gacgggactg ggctccacgc tctacaccca cctgctgaag tccctggagg 1380
cacagggctt caagagcgtg gtcgctgtca tcgggctgcc caacgacccg agcgtgcgca 1440
tgcacgaggc gctcggatat gccccccgcg gcatgctgcg ggcggccggc ttcaagcacg 1500
ggaactggca tgacgtgggt ttctggcagc tggacttcag cctgccggta ccgccccgtc 1560
cggtcctgcc cgtcaccgag atctgatccg tcgacctgca gatcgttcaa acatttggca 1620
ataaagtttc ttaagattga atcctgtact agtgatccga gatccggggt gacaccaccg 1680
agaccgaggt gccgtacgac atgctggtgg tcggcgtggg cgccgagaac gccacgttcg 1740
gcatccccgg cgtgcgcgag cactcgtgct tcctcaagga gatcggcgac gcccaaaaga 1800
tccgcaagcg catcatggac tgcgtcgaga cggcggcgtt caaggaccag tcgcccgagg 1860
agatcgaccg gctcctgcac atggtggtcg tcgggggcgg cccgaccggc gtcgagttcg 1920
ccggcgagct gcaggacttc ttcgaggagg acatcaagaa gctggtgccc gagatcagcg 1980
accgcttccg ggtgacgctc atcgaggcgc tgcctaacgt gctgcccatg ttctccaagc 2040
agctgatcga gtacaccgag agcaccttca aggaggagaa gatcaacatc cacaccaaga 2100
ccgtggtcaa gaaggtgacg gacaagacgg tcgaggccga ggtgacgcgc ccggacggca 2160
agaaggagac ggtcgtcttc ccctacggcc tgctcgtctg ggccaccggt aacgccgtcc 2220
gccccgtcgt ccgggacctg atgtcgcgca tcccggccca ggccggctcg cgccgcggcc 2280
tcgccgtcaa cgagtacctc gtcgtccagg gcacgcgcga catctgggcc gtgggcgact 2340
g 2341

<210> 148
<211> 22
<212> DNA
<213> Primer
<400> 148
aggatcggtg gagtgaagtt cg 22

<210>  149
<211>  37
<212>  DNA
<213>  Primer
<400>  149
ctgatagaag ccaatcgagg aaagaaagaa aagaaga                                37

<210>  150
<211>  40
<212>  DNA
<213>  Primer
<400>  150
gattggcttc tatcagggct gttttagagc tagaaatagc                             40

<210>  151
<211>  23
<212>  DNA
<213>  Primer
<400>  151
aaaaaaagca ccgactcggt gcc                                               23

<210>  152
<211>  24
<212>  DNA
<213>  Primer
<400>  152
cgggctcagc cggaacttgc tacc                                              24

<210>  153
<211>  29
<212>  DNA
<213>  Primer
<400>  153
ccttcaatat ggctagcaac ggggtgaag                                         29

<210>  154
<211>  31
<212>  DNA
<213>  Primer
<400>  154
gttgctagcc atattgaagg agcacttttt g                                      31

<210>  155
<211>  29
<212>  DNA
<213>  Primer
<400>  155
gggatgaccg gatcactagt acaggattc                                         29

<210>  156
<211>  28
<212>  DNA
<213>  Primer
<400>  156
actagtgatc cggtcatccc cgctcctt                                          28

<210>  157
<211>  21
<212>  DNA
<213>  Primer
<400>  157

gatcaagggg tttggcatga g                                                21

<210>  158
<211>  43
<212>  DNA
<213>  Primer
<400>  158
gatttcagta acgttaagtg gatcctcagg cgccgtaggc ctt                          43

<210>  159
<211>  37
<212>  DNA
<213>  Primer
<400>  159
tctggcggag gatggtcgag gaaagaaaga aaagaag                                 37

<210>  160
<211>  40
<212>  DNA
<213>  Primer
<400>  160
accatcctcc gccagaagag ttttagagct agaaatagca                              40

<210>  161
<211>  44
<212>  DNA
<213>  Primer
<400>  161
ccacatcaca gaaatcaaaa ctagtatgtt ggcttctcgt cagc                         44

<210>  162
<211>  23
<212>  DNA
<213>  Primer
<400>  162
tcctcctccc tctctcaggc gcc                                                23

<210>  163
<211>  29
<212>  DNA
<213>  Primer
<400>  163
ccttcaatat agttgcgcgg ggagatgac                                          29

<210>  164
<211>  31
<212>  DNA
<213>  Primer
<400>  164
ccgcgcaact atattgaagg agcacttttt g                                       31

<210>  165
<211>  1332
<212>  DNA
<213>  Myceliophthora thermophila
<400>  165
atggctccaa agattaacgg cactggcgtc atgcccgagg acacgcgcat ctgcgttgtc        60
atggtcggcc ttccggctcg gggcaagagt tacatagccc aaagagcgca acgctatctc       120
aagtggctgt caatcccggc gaagaccttc aacgtgggca actaccgccg gaaagatgcg       180
ccacatccat cagccgactt cttcgacacc aacaacgccg aaggcgagag acagcgccgc       240
gcagctgcca acgccgccgt gacagacatg atccagtggt tcaaaaccgg cggtgttgtc       300
ggaatccttg acgctacaaa cagcaccaaa gaacggcgga aatgggtact cgagcgactc       360
tctaaagagg ggatcgaggt cctctttgtc gagtccaagt gcgacgacga ggaactgata       420

atggccaaca tccgcgatgt caaaaccact tccccggatt accagggtca ggaccctgag 480
caggctgccc ttgacttccg cgaacgcatc cgccattacg agaaggtcta caagtcgatc 540
aacgacgata atgatgaaga ccacttgacg tacctcaaga taatgaatgt gggcaagaag 600
gtcttcatca accgcattca ggactaccta cagagccgcg tcgtctactt cctcatgaac 660
ctgcatatcc ggccccgctc ggtttggctg tctcgccatg gcgagtctga atacaatttg 720
gaagggcgga ttggcggtga ttcattgctc tctcaccgcg gcgaagagta cgcccgcaaa 780
ttgcccgaat tggttcgcca gtctgttggt agcgaccgac cattaacggt ctggacgtcg 840
acgctgaaac ggaccatcgc cacagcccgg cacttgccaa aacactacaa ccaactgcag 900
tggaaggcgc tggatgagct agacgctggt gtctgcgacg ggttgacgta ccaggagatt 960
gcagaccagt atccggagga ctttcaggcg cgcgacgaag ataaatacaa ctaccgatat 1020
cgcggcggcg agagctaccg cgacgtcgtc attcgcctcg aacccatcat catggaactc 1080
gagcgcagcg aaaacatcct catcatctcg caccaggccg tcatccgctg catctacgcc 1140
tacttcatgc aaaagcctca agaggaaagc ccgtgggtgc ctgtaccgct gcacacccta 1200
atcaagctca cgccccgcgc ctacgacacc ctggtggaga agtacgatgc gaaaatcaag 1260
gccgtgagca cctggcgcgg caaggggagc accgccaaac acgaagatcc aaccccagaa 1320
ggcggcattt ga 1332

<210> 166
<211> 443
<212> PRT
<213> Myceliophthora thermophila
<400> 166
MET Ala Pro Lys Ile Asn Gly Thr Gly Val MET Pro Glu Asp Thr Arg
1               5                   10                  15
Ile Cys Val Val MET Val Gly Leu Pro Ala Arg Gly Lys Ser Tyr Ile
            20                  25                  30
Ala Gln Arg Ala Gln Arg Tyr Leu Lys Trp Leu Ser Ile Pro Ala Lys
        35                  40                  45
Thr Phe Asn Val Gly Asn Tyr Arg Arg Lys Asp Ala Pro His Pro Ser
    50                  55                  60
Ala Asp Phe Phe Asp Thr Asn Asn Ala Glu Gly Glu Arg Gln Arg Arg
65                  70                  75                  80
Ala Ala Ala Asn Ala Ala Val Thr Asp MET Ile Gln Trp Phe Lys Thr
                85                  90                  95
Gly Gly Val Val Gly Ile Leu Asp Ala Thr Asn Ser Thr Lys Glu Arg
            100                 105                 110
Arg Lys Trp Val Leu Glu Arg Leu Ser Lys Glu Gly Ile Glu Val Leu
        115                 120                 125
Phe Val Glu Ser Lys Cys Asp Asp Glu Glu Leu Ile MET Ala Asn Ile
    130                 135                 140
Arg Asp Val Lys Thr Thr Ser Pro Asp Tyr Gln Gly Gln Asp Pro Glu
145                 150                 155                 160
Gln Ala Ala Leu Asp Phe Arg Glu Arg Ile Arg His Tyr Glu Lys Val
                165                 170                 175
Tyr Lys Ser Ile Asn Asp Asp Asn Asp Glu Asp His Leu Thr Tyr Leu
            180                 185                 190
Lys Ile MET Asn Val Gly Lys Lys Val Phe Ile Asn Arg Ile Gln Asp
        195                 200                 205
Tyr Leu Gln Ser Arg Val Val Tyr Phe Leu MET Asn Leu His Ile Arg
    210                 215                 220
Pro Arg Ser Val Trp Leu Ser Arg His Gly Glu Ser Glu Tyr Asn Leu
225                 230                 235                 240
Glu Gly Arg Ile Gly Gly Asp Ser Leu Leu Ser His Arg Gly Glu Glu
                245                 250                 255
Tyr Ala Arg Lys Leu Pro Glu Leu Val Arg Gln Ser Val Gly Ser Asp
            260                 265                 270
Arg Pro Leu Thr Val Trp Thr Ser Thr Leu Lys Arg Thr Ile Ala Thr
        275                 280                 285
Ala Arg His Leu Pro Lys His Tyr Asn Gln Leu Gln Trp Lys Ala Leu
    290                 295                 300
Asp Glu Leu Asp Ala Gly Val Cys Asp Gly Leu Thr Tyr Gln Glu Ile
305                 310                 315                 320
Ala Asp Gln Tyr Pro Glu Asp Phe Gln Ala Arg Asp Glu Asp Lys Tyr

                325                 330                 335
Asn Tyr Arg Tyr Arg Gly Gly Glu Ser Tyr Arg Asp Val Val Ile Arg
            340                 345                 350
Leu Glu Pro Ile Ile MET Glu Leu Glu Arg Ser Glu Asn Ile Leu Ile
        355                 360                 365
Ile Ser His Gln Ala Val Ile Arg Cys Ile Tyr Ala Tyr Phe MET Gln
    370                 375                 380
Lys Pro Gln Glu Glu Ser Pro Trp Val Pro Val Pro Leu His Thr Leu
385                 390                 395                 400
Ile Lys Leu Thr Pro Arg Ala Tyr Asp Thr Leu Val Glu Lys Tyr Asp
                405                 410                 415
Ala Lys Ile Lys Ala Val Ser Thr Trp Arg Gly Lys Gly Ser Thr Ala
            420                 425                 430
Lys His Glu Asp Pro Thr Pro Glu Gly Gly Ile
        435                 440

<210> 167
<211> 567
<212> DNA
<213> Myceliophthora thermophila
<400> 167
aggatcggtg gagtgaagtt cggaatcgag gttcggcgat gggtcgtaag catggcgact 60
tcgaacttac ttgcactggc aagcgttgcc agaacggcga gaaaaagaag ggtaagcgat 120
attcgcgtca tgatggactg ttccttttgg aacagtagtt gttgtgggaa gactatgtca 180
cacttgccca cctgcaaggc cagggtcgtg gtcgaacgag accagcctcg gcgctgctgg 240
gagctcaaga tgggcacgtt tgattcgtta gacgtcaaca aggctggagt tcctagtgac 300
agccaaaggc acagccacat taagtggcgc tttatctgtc cactaaggtt caattgtggc 360
tttgagccgc gcagtgtgca gtcgtgcatt ggccacctag ctagcagtat ttaagatcct 420
cttctctccc gagatcttcc tcctcttctt ttctttcttt cctcggagat tgcagaccag 480
tatcgtttta gagctagaaa tagcaagtta aaataaggct agtccgttat caacttgaaa 540
aagtggcacc gagtcggtgc ttttttt 567

<210> 168
<211> 2954
<212> DNA
<213> Myceliophthora thermophila
<400> 168
ggctcgggtc ggttaatggg aactgctcgc ttcccggttt cagccggccg agtatctaga 60
aggcactctg actggactgg aagttgtgca tatttggaaa tatgaggagc aattattgat 120
tcggcagagg gctcgcaatc agacagagcg tgggggaggg cgatttgtgg ttggcattgc 180
tagatggcgg tcagacgggt ccactggcca aaggctgcca gctttcgttc ctgcagggag 240
aaagcgaaaa cccatcgcgt cacgggcgct cgaacccagt cgaacccagc ctgagcgccc 300
ccacttttag tctgcctctt cgggttcgtc cggggaagaa cggctggagc caaaaacccg 360
ctgttggcca ggctctggct gaacttggcg gcgccatgat gcccaggaac caggatcttc 420
gcccgctggc ccgccttact tgccgtccct gggaatcccg atgcaacaag acaagtgatg 480
tagctcgatg ctaccgaggt gcagaaagca aaattgcggt tgctcttctt cctttcgacc 540
taaaatttcc acaccgacga cgtcgccgtc gtccctccga gattttgcag ccgttggagc 600
ccattgtttc attcccgccg tgcctcgctg cttcgacgcg cggcgacagc gccaaaggtg 660
tgacgtcgga agtgggagag ctcccctctc aatagcccac tatcgccaac cgccttctcg 720
gcccgcagcc ggtggcccac gaagcgctca cgccatcaac atctacgcac gctccccgct 780
gacatctccg gctctgcgct gttcctcggc cccaatcagg ctgctacatg tctcgatcac 840
ctatcctgcc ctcctccgcc tccagaacgt tgctacagcg gctgtcgctc agcaaagata 900
ttgaaggagc actttttggg cttggctgga gctagtggag gtcaacaatg aatgcctatt 960
ttggtttagt cgtccaggcg gtgagcacaa aatttgtgtc gtttgacaag atggttcatt 1020
taggcaactg gtcagatcag ccccacttgt agcagtagcg gcggcgctcg aagtgtgact 1080
cttattagca gacaggaacg aggacattat tatcatctgc tgcttggtgc acgataactt 1140
ggtgcgtttg tcaagcaagg taagtgaacg acccggtcat accttcttaa gttcgccctt 1200
cctcccttta tttcagattc aatctgactt acctattcta cccaagcaaa gcttcgatta 1260
ggaagtaacc atgagcccag aacgacgccc ggccgacatc cgccgtgcca ccgaggcgga 1320
catgccggcg gtctgcacca tcgtcaacca ctacatcgag acaagcacgg tcaacttccg 1380
taccgagccg caggaaccgc aggagtggac ggacgacctc gtccgtctgc gggagcgcta 1440
tccctggctc gtcgccgagg tggacggcga ggtcgccggc atcgcctacg cgggcccctg 1500
gaaggcacgc aacgcctacg actggacggc cgagtcgacc gtgtacgtct ccccccgcca 1560

ccagcggacg ggactgggct ccacgctcta cacccacctg ctgaagtccc tggaggcaca 1620
gggcttcaag agcgtggtcg ctgtcatcgg gctgcccaac gacccgagcg tgcgcatgca 1680
cgaggcgctc ggatatgccc cccgcggcat gctgcgggcg gccggcttca agcacgggaa 1740
ctggcatgac gtgggtttct ggcagctgga cttcagcctg ccggtaccgc cccgtccggt 1800
cctgcccgtc accgagatct gacatctgcc tcacggggtg aacacgggag gcgtgccaac 1860
gtgatttgcc gtattgtctg ttctcttctc tcagcatatc tagacacgat gtttcgtttc 1920
ctatttttgg cgacaggctg tgccttgcat ttctgggata acatcatatg agaagtccgt 1980
ggttgaaact gggcggaaac ctctgggccg gcgttttgcg gttgggtgtc agcgttggct 2040
tttgctcacg gactgagctt ttgagtccga caaggaaatg atacgctact ggtaccatct 2100
gggagcggtt acttggctgg tttagacggc aaaggaaaac aaaaaacggg ccaactggac 2160
gcatggaatg gacctaaatc gacggctcgc acggaagcac gtatggacgc tgcgtgctct 2220
gccaccgggc agtggcatta tctacatgcc caattgggcc aacatttggc attataaaga 2280
ctaagcattg ccgcttttat ctcttgacca agtttccgat gattctgtca cataccatcc 2340
acgtgtcgag gaaacagcct ttggtcaatg ggagtgtggg cgccatctcc ttattgcgtt 2400
tcgttccttg ataccgcgac aaagtcttct gtccgtggcc taactgtgca gggaggattc 2460
aacttcccaa atatttgaaa cgcatgcttt ctacaggcta gcattgggca ttcggctccg 2520
tgtggatgtc tcagaagaga aaacatggtg ctgctcacat tatacaatga atgccagaga 2580
gtatcagggg caatgacagt gcttaagatg ctaggctaaa gcgtccgttt tgtcgtgcaa 2640
ccatccgccg agattgacac acttgcttct gcaccaaatt gccccttcta gcggtaaacg 2700
ctgaccatga tagcttgaga gcaatacatg gcaaaggcac agcagcgcct atcaataatg 2760
ttggcggggg atgaaacgca tctgggcttg accaacgtcg aagatgacca gcgcgttttg 2820
catgaatgcg tccccaagaa tatatgggcc cgagcctcca tccgcaatgg ccgtcatgca 2880
aagccccgtc accggatcaa ccaaggtacg ataaaggagg tccaaagggc tgatgttgaa 2940
ctttacgcca ttga 2954

<210> 169
<211> 34
<212> DNA
<213> Primer
<400> 169
tcttggtggt ggccatcatg cgccggagac caag 34

<210> 170
<211> 1332
<212> DNA
<213> Myceliophthora thermophila
<400> 170
atggctccaa agattaacgg cactggcgtc atgcccgagg acacgcgcat ctgcgttgtc 60
atggtcggcc ttccggctcg gggcaagagt tacatagccc aaagagcgca acgctatctc 120
aagtggctgt caatcccggc gaagaccttc aacgtgggca actaccgccg gaaagatgcg 180
ccacatccat cagccgactt cttcgacacc aacaacgccg aaggcgagag acagcgccgc 240
gcagctgcca acgccgccgt gacagacatg atccagtggt tcaaaaccgg cggtgttgtc 300
ggaatccttg acgctacaaa cagcaccaaa gaacggcgga aatgggtact cgagcgactc 360
tctaaagagg ggatcgaggt cctctttgtc gagtccaagt gcgacgacga ggaactgata 420
atggccaaca tccgcgatgt caaaaccact tccccggatt accagggtca ggaccctgag 480
caggctgccc ttgacttccg cgaacgcatc cgccattacg agaaggtcta caagtcgatc 540
aacgacgata atgatgaaga ccacttgacg tacctcaaga taatgaatgt gggcaagaag 600
gtcttcatca accgcattca ggactaccta cagagccgcg tcgtctactt cctcatgaac 660
ctgcatatcc ggccccgctc ggtttggctg tctcgcggcg gcgagtctga atacaatttg 720
gaaggcggga ttggcggtga ttcattgctc tctcaccgcg gcgaagagta cgcccgcaaa 780
ttgcccgaat tggttcgcca gtctgttggt agcgaccgac cattaacggt ctggacgtcg 840
acgctgaaac ggaccatcgc cacagcccgg cacttgccaa aacactacaa ccaactgcag 900
tggaaggcgc tggatggcct agacgctggt gtctgcgacg ggttgacgta ccaggagatt 960
gcagaccagt atccggagga ctttcaggcg cgcgacgaag ataaatacaa ctaccgatat 1020
cgcggcggcg agagctaccg cgacgtcgtc attcgcctcg aacccatcat catggaactc 1080
gagcgcagcg aaaacatcct catcatctcg ggccaggccg tcatccgctg catctacgcc 1140
tacttcatgc aaaagcctca agaggaaagc ccgtgggtgc ctgtaccgct gcacacccta 1200
atcaagctca cgccccgcgc ctacgacacc ctggtggaga agtacgatgc gaaaatcaag 1260
gccgtgagca cctggcgcgg caaggggagc accgccaaac acgaagatcc aaccccagaa 1320
ggcggcattt ga 1332

<210> 171
<211> 443

<212>  PRT
<213>  Myceliophthora thermophila
<400>  171
Met Ala Pro Lys Ile Asn Gly Thr Gly Val Met Pro Glu Asp Thr Arg
1               5                   10                  15
Ile Cys Val Val Met Val Gly Leu Pro Ala Arg Gly Lys Ser Tyr Ile
            20                  25                  30
Ala Gln Arg Ala Gln Arg Tyr Leu Lys Trp Leu Ser Ile Pro Ala Lys
        35                  40                  45
Thr Phe Asn Val Gly Asn Tyr Arg Arg Lys Asp Ala Pro His Pro Ser
    50                  55                  60
Ala Asp Phe Phe Asp Thr Asn Asn Ala Glu Gly Glu Arg Gln Arg Arg
65                  70                  75                  80
Ala Ala Ala Asn Ala Ala Val Thr Asp Met Ile Gln Trp Phe Lys Thr
                85                  90                  95
Gly Gly Val Val Gly Ile Leu Asp Ala Thr Asn Ser Thr Lys Glu Arg
            100                 105                 110
Arg Lys Trp Val Leu Glu Arg Leu Ser Lys Glu Gly Ile Glu Val Leu
        115                 120                 125
Phe Val Glu Ser Lys Cys Asp Asp Glu Glu Leu Ile Met Ala Asn Ile
    130                 135                 140
Arg Asp Val Lys Thr Thr Ser Pro Asp Tyr Gln Gly Gln Asp Pro Glu
145                 150                 155                 160
Gln Ala Ala Leu Asp Phe Arg Glu Arg Ile Arg His Tyr Glu Lys Val
                165                 170                 175
Tyr Lys Ser Ile Asn Asp Asp Asn Asp Glu Asp His Leu Thr Tyr Leu
            180                 185                 190
Lys Ile Met Asn Val Gly Lys Lys Val Phe Ile Asn Arg Ile Gln Asp
        195                 200                 205
Tyr Leu Gln Ser Arg Val Val Tyr Phe Leu Met Asn Leu His Ile Arg
    210                 215                 220
Pro Arg Ser Val Trp Leu Ser Arg Gly Gly Glu Ser Glu Tyr Asn Leu
225                 230                 235                 240
Glu Gly Arg Ile Gly Gly Asp Ser Leu Leu Ser His Arg Gly Glu Glu
                245                 250                 255
Tyr Ala Arg Lys Leu Pro Glu Leu Val Arg Gln Ser Val Gly Ser Asp
            260                 265                 270
Arg Pro Leu Thr Val Trp Thr Ser Thr Leu Lys Arg Thr Ile Ala Thr
        275                 280                 285
Ala Arg His Leu Pro Lys His Tyr Asn Gln Leu Gln Trp Lys Ala Leu
    290                 295                 300
Asp Gly Leu Asp Ala Gly Val Cys Asp Gly Leu Thr Tyr Gln Glu Ile
305                 310                 315                 320
Ala Asp Gln Tyr Pro Glu Asp Phe Gln Ala Arg Asp Glu Asp Lys Tyr
                325                 330                 335
Asn Tyr Arg Tyr Arg Gly Gly Glu Ser Tyr Arg Asp Val Val Ile Arg
            340                 345                 350
Leu Glu Pro Ile Ile Met Glu Leu Glu Arg Ser Glu Asn Ile Leu Ile
        355                 360                 365
Ile Ser Gly Gln Ala Val Ile Arg Cys Ile Tyr Ala Tyr Phe Met Gln
    370                 375                 380
Lys Pro Gln Glu Glu Ser Pro Trp Val Pro Val Pro Leu His Thr Leu
385                 390                 395                 400
Ile Lys Leu Thr Pro Arg Ala Tyr Asp Thr Leu Val Glu Lys Tyr Asp
                405                 410                 415
Ala Lys Ile Lys Ala Val Ser Thr Trp Arg Gly Lys Gly Ser Thr Ala
            420                 425                 430
Lys His Glu Asp Pro Thr Pro Glu Gly Gly Ile
        435                 440

<210>  172
<211>  28
<212>  DNA

<213>  Primer
<400>  172
ctgtcggcag cgcgagcctt ggtctccg                                          28

<210>  173
<211>  22
<212>  DNA
<213>  Primer
<400>  173
ATGGCTCCAA AGATTAACGG CA                                                22

<210>  174
<211>  24
<212>  DNA
<213>  Primer
<400>  174
TCAGACTCGC CGCCGCGAGA CAGC                                              24

<210>  175
<211>  24
<212>  DNA
<213>  Primer
<400>  175
GCTGTCTCGC GGCGGCGAGT CTGA                                              24

<210>  176
<211>  24
<212>  DNA
<213>  Primer
<400>  176
CAGCGTCTAG GCCATCCAGC GCCT                                              24

<210>  177
<211>  24
<212>  DNA
<213>  Primer
<400>  177
AGGCGCTGG ATGGCCTAGAC GCTG                                              24

<210>  178
<211>  24
<212>  DNA
<213>  Primer
<400>  178
ATGACGGCC TGGCCCGAGAT GATG                                             24

<210>  179
<211>  24
<212>  DNA
<213>  Primer
<400>  179
CATCATCTCG GGCCAGGCCG TCAT                                             24

<210>  180
<211>  23
<212>  DNA
<213>  Primer
<400>  180
TCAAATGCC GCCTTCTGGGG TTG                                              23

<210>  181
<211>  45
<212>  DNA

<213>  Primer
<400>  181
gccagtttcg ttcttcagaa ctagtatggc tccaaagatt aacgg                    45


<210>  182
<211>  43
<212>  DNA
<213>  Primer
<400>  182
gatttcagta acgttaagtg gatcctcaaa tgccgccttc tgg                      43

<210>  183
<211>  22
<212>  DNA
<213>  Primer
<400>  183
aggatcggtg gagtgaagtt cg                                             22

<210>  184
<211>  40
<212>  DNA
<213>  Primer
<400>  184
atactggtct gcaatctccg aggaaagaaa gaaaagaaga                          40

<210>  185
<211>  42
<212>  DNA
<213>  Primer
<400>  185
agattgcaga ccagtatcgt tttagagcta gaaatagcaa gt                       42

<210>  186
<211>  23
<212>  DNA
<213>  Primer
<400>  186
aaaaaaagca ccgactcggt gcc                                            23


<210>  187
<211>  43
<212>  DNA
<213>  Primer
<400>  187
tacacagtac acgaggactt ctagaggctc gggtcggtta atg                      43


<210>  188
<211>  28
<212>  DNA
<213>  Primer
<400>  188
ccttcaatat ctttgctgag cgacagcc                                       28

<210>  189
<211>  33
<212>  DNA
<213>  Primer
<400>  189
ctcagcaaag atattgaagg agcacttttt ggg                                 33

<210>  190
<211>  28
<212>  DNA
<213>  Primer
<400>  190
gaggcagatg tcagatctcg gtgacggg                                          28

<210>  191
<211>  28
<212>  DNA
<213>  Primer
<400>  191
cgagatctga catctgcctc acggggtg                                          28

<210>  192
<211>  50
<212>  DNA
<213>  Primer
<400>  192
aatatcatct tctgtcgagg aattctcaat ggcgtaaagt tcaacatcag                   50

<210>  193
<211>  27
<212>  DNA
<213>  Primer
<400>  193
catcatcgat acactggttc ttgacaa                                           27

<210>  194
<211>  25
<212>  DNA
<213>  Primer
<400>  194
ctcagggtcc tgaccctggt aatcc                                             25

<210>  195
<211>  860
<212>  PRT
<213>  Thermoanaerobacterium saccharolyticum
<400>  195
Met Ala Thr Thr Lys Thr Glu Leu Asp Val Gln Lys Gln Ile Asp Leu
1               5                   10                  15
Leu Val Ser Arg Ala Gln Glu Ala Gln Lys Lys Phe MET Ser Tyr Thr
            20                  25                  30
Gln Glu Gln Ile Asp Ala Ile Val Lys Ala MET Ala Leu Ala Gly Val
        35                  40                  45
Asp Lys His Val Glu Leu Ala Lys MET Ala Tyr Glu Glu Thr Lys MET
    50                  55                  60
Gly Val Tyr Glu Asp Lys Ile Thr Lys Asn Leu Phe Ala Thr Glu Tyr
65                  70                  75                  80
Val Tyr His Asp Ile Lys Asn Glu Lys Thr Val Gly Ile Ile Asn Glu
                85                  90                  95
Asn Ile Glu Glu Asn Tyr MET Glu Val Ala Glu Pro Ile Gly Val Ile
            100                 105                 110
Ala Gly Val Thr Pro Val Thr Asn Pro Thr Ser Thr Thr MET Phe Lys
        115                 120                 125
Cys Leu Ile Ser Ile Lys Thr Arg Asn Pro Ile Ile Phe Ser Phe His
    130                 135                 140
Pro Lys Ala Ile Lys Cys Ser Ile Ala Ala Ala Lys Val MET Tyr Glu
145                 150                 155                 160

Ala Ala Leu Lys Ala Gly Ala Pro Glu Gly Cys Ile Gly Trp Ile Glu
                165                 170                 175
Thr Pro Ser Ile Glu Ala Thr Gln Leu Leu MET Thr His Pro Gly Val
            180                 185                 190
Ser Leu Ile Leu Ala Thr Gly Gly Ala Gly MET Val Lys Ala Ala Tyr
        195                 200                 205
Ser Ser Gly Lys Pro Ala Leu Gly Val Gly Pro Gly Asn Val Pro Cys
    210                 215                 220
Tyr Ile Glu Lys Ser Ala Asn Ile Lys Arg Ala Val Ser Asp Leu Ile
225                 230                 235                 240
Leu Ser Lys Thr Phe Asp Asn Gly Val Ile Cys Ala Ser Glu Gln Ala
                245                 250                 255
Val Ile Ile Asp Glu Glu Ile Ala Asp Glu Val Lys Lys Leu MET Lys
            260                 265                 270
Glu Tyr Gly Cys Tyr Phe Leu Asn Lys Asp Glu Ile Lys Lys Leu Glu
        275                 280                 285
Lys Phe Ala Ile Asp Glu Gln Ser Cys Ala MET Ser Pro Ala Val Val
    290                 295                 300
Gly Gln Pro Ala Ala Lys Ile Ala Glu MET Ala Gly Phe Lys Val Pro
305                 310                 315                 320
Glu Gly Thr Lys Ile Leu Val Ala Glu Tyr Glu Gly Val Gly Pro Lys
                325                 330                 335
Tyr Pro Leu Ser Arg Glu Lys Leu Ser Pro Ile Leu Ala Cys Tyr Thr
            340                 345                 350
Val Lys Asp Tyr Asn Glu Gly Ile Lys Lys Cys Glu Glu MET Thr Glu
        355                 360                 365
Phe Gly Gly Leu Gly His Ser Ala Val Ile His Ser Glu Asn Gln Asn
    370                 375                 380
Val Ile Asn Glu Phe Ala Arg Arg Val Arg Thr Gly Arg Leu Ile Val
385                 390                 395                 400
Asn Ser Pro Ser Ser Gln Gly Ala Ile Gly Asp Ile Tyr Asn Thr Asn
                405                 410                 415
Thr Pro Ser Leu Thr Leu Gly Cys Gly Ser MET Gly Arg Asn Ser Thr
            420                 425                 430
Thr Asp Asn Val Ser Val Lys Asn Leu Leu Asn Ile Lys Arg Val Val
        435                 440                 445
Ile Arg Lys Asp Arg MET Lys Trp Phe Lys Ile Pro Pro Lys Ile Tyr
    450                 455                 460
Phe Glu Ser Gly Ser Leu Gln Tyr Leu Cys Lys Val Lys Arg Lys Lys
465                 470                 475                 480
Ala Phe Ile Val Thr Asp Pro Phe MET Val Lys Leu Gly Phe Val Asp
                485                 490                 495
Lys Val Thr Tyr Gln Leu Asp Lys Ala Asn Ile Glu Tyr Glu Ile Phe
            500                 505                 510
Ser Glu Val Glu Pro Asp Pro Ser Val Asp Thr Val MET Asn Gly Val
        515                 520                 525
Lys Ile MET Asn Ser Tyr Asn Pro Asp Leu Ile Ile Ala Val Gly Gly
    530                 535                 540
Gly Ser Ala Ile Asp Ala Ala Lys Gly MET Trp Leu Phe Tyr Glu Tyr
545                 550                 555                 560
Pro Asp Thr Glu Phe Glu Thr Leu Arg Leu Lys Phe Ala Asp Ile Arg
                565                 570                 575
Lys Arg Ala Phe Lys Phe Pro Glu Leu Gly Lys Lys Ala Leu Phe Ile
            580                 585                 590
Ala Ile Pro Thr Thr Ser Gly Thr Gly Ser Glu Val Thr Ala Phe Ala
        595                 600                 605
Val Ile Thr Asp Lys Lys Arg Asn Ile Lys Tyr Pro Leu Ala Asp Tyr
    610                 615                 620
Glu Leu Thr Pro Asp Ile Ala Ile Ile Asp Pro Asp Leu Thr Lys Thr
625                 630                 635                 640
Val Pro Pro Ser Val Thr Ala Asp Thr Gly MET Asp Val Leu Thr His
                645                 650                 655
Ala Ile Glu Ala Tyr Val Ser Val MET Ala Ser Asp Tyr Thr Asp Ala

            660                 665                 670
Leu Ala Glu Lys Ala Ile Lys Ile Val Phe Glu Tyr Leu Pro Arg Ala
        675                 680                 685
Tyr Lys Asn Gly Asn Asp Glu Glu Ala Arg Glu Lys MET His Asn Ala
    690                 695                 700
Ser Cys MET Ala Gly MET Ala Phe Thr Asn Ala Phe Leu Gly Ile Asn
705                 710                 715                 720
His Ser MET Ala His Ile Leu Gly Gly Lys Phe His Ile Pro His Gly
                725                 730                 735
Arg Ala Asn Ala Ile Leu Leu Pro Tyr Val Ile Arg Tyr Asn Ala Glu
            740                 745                 750
Lys Pro Thr Lys Phe Val Ala Phe Pro Gln Tyr Glu Tyr Pro Lys Ala
        755                 760                 765
Ala Glu Arg Tyr Ala Glu Ile Ala Lys Phe Leu Gly Leu Pro Ala Ser
    770                 775                 780
Thr Val Glu Glu Gly Val Glu Ser Leu Ile Glu Ala Ile Lys Asn Leu
785                 790                 795                 800
Met Lys Glu Leu Asn Ile Pro Leu Thr Leu Lys Asp Ala Gly Ile Asn
                805                 810                 815
Lys Glu Gln Phe Glu Lys Glu Ile Glu Glu MET Ser Asp Ile Ala Phe
            820                 825                 830
Asn Asp Gln Cys Thr Gly Thr Asn Pro Arg MET Pro Leu Thr Lys Glu
        835                 840                 845
Ile Ala Glu Ile Tyr Arg Lys Ala Tyr Gly Ala
ã\200\200ã\200\200850 855

ã\200\200ã\200\200
<210> 196
<211> 2580
<212> DNA
<213> Thermoanaerobacterium saccharolyticum
<400> 196
atggccacca ccaagaccga gctcgacgtc cagaagcaga tcgacctcct cgtcagccgc 60
gcccaggagg cccagaagaa gttcatgagc tacacccagg agcagatcga cgccatcgtc 120
aaggccatgg ccctcgccgg cgtcgacaag cacgtcgagc tcgccaagat ggcctacgag 180
gagaccaaga tgggcgtcta cgaggacaag atcaccaaga acctcttcgc caccgagtac 240
gtctaccacg acatcaagaa cgagaagacc gtcggcatca tcaacgagaa catcgaggag 300
aactacatgg aggtcgccga gccgatcggc gtcatcgccg gcgtcactcc cgtaaccaac 360
ccgaccagca caacaatgtt taaatgtctg atatctatta aaactcgaaa ccctattata 420
ttctctttcc acccgaaggc gattaaatgt tcgattgccg cagcgaaggt catgtatgaa 480
gctgctctga aagcaggcgc accggaaggc tgcatcggct ggatcgagac tccctcaatc 540
gaggccaccc agctcttgat gacacatccc ggagtttcgt tgattctggc tactggagga 600
gcaggaatgg taaaggcagc gtattcttcc ggaaagccgg cgctgggcgt ggggccggga 660
aatgtcccgt gctacattga aaagtcggcc aacatcaaac gggcagtgag cgacctcatc 720
ctcagcaaga ccttcgacaa cggcgtcatc tgcgcaagtg aacaggccgt catcatcgac 780
gaagaaattg ccgatgaagt caagaagctt atgaaggagt acgggtgcta ctttctgaat 840
aaggacgaga tcaagaagtt agagaaattc gcgatcgatg agcagtcatg tgcaatgtct 900
ccagcagtag tgggacagcc ggccgccaag atagcagaaa tggctggctt caaggtcccg 960
gagggcacca agatcctcgt cgccgagtac gagggagttg gacccaagta cccacttagt 1020
cgtgagaagc tcagcccgat cctcgcctgc tacaccgtca aggactacaa cgagggcatc 1080
aagaagtgcg aggagatgac cgagttcggc ggattgggtc actccgcagt tatacactcc 1140
gaaaatcaga acgtaattaa cgagtttgct cggcgagtta ggacgggccg cttaattgtc 1200
aacagcccga gcagccaggg cgccatcggc gacatctaca acaccaacac gccttcgctc 1260
accctcggct gcgggtccat ggggcgtaat tcaaccacgg acaatgtttc agttaagaat 1320
cttttgaata taaagcgcgt ggtgatccgc aaggaccgca tgaagtggtt caagatcccg 1380
ccgaagatct acttcgagag cggcagcctc cagtacctct gcaaggtcaa gcgcaagaag 1440
gccttcatcg tcaccgaccc gttcatggtc aagctcggct tcgtcgacaa ggtcacctac 1500
cagctcgaca aggccaacat cgagtacgag atcttcagcg aggtcgagcc ggacccgagc 1560
gtcgacaccg tcatgaacgg cgtcaagatc atgaacagct acaatcctga tctgattata 1620
gccgtcggcg gcggcagcgc catcgacgcc gccaagggca tgtggctctt ctatgaatat 1680
cccgatactg agttcgagac cctccgcctc aagttcgccg acatccgcaa gcgcgccttc 1740
aagttcccgg agctcggcaa gaaggccctc ttcatcgcca tcccgaccac cagcggcacc 1800
ggcagcgagg tcaccgcctt cgccgtcatc accgacaaga agcgcaacat caaatatccg 1860

cttgctgatt acgagctcac acccgatatc gccatcatcg atcccgattt gacgaagaca 1920
gtgccgccga gcgtcaccgc cgacaccggc atggacgtcc tcacccacgc catcgaggcc 1980
tatgtgagtg taatggcaag cgactacacc gacgccctcg ccgagaaggc tattaagatc 2040
gtctttgaat acctcccgcg cgcctacaag aacggcaacg acgaggaggc ccgcgagaag 2100
atgcacaacg ccagctgcat ggccggcatg gccttcacca acgccttcct cggcatcaac 2160
cacagcatgg cccacatcct cggcggcaag ttccacatcc cgcacggccg cgccaacgcc 2220
atcctcctcc cgtacgtcat ccgctacaac gccgagaagc cgaccaagtt cgtcgccttc 2280
ccgcagtatg aatatccaaa ggccgcggag cgctacgccg agatcgccaa gttcctcggc 2340
ctcccggcca gcactgtaga ggagggcgtc gagagcttaa ttgaagctat taagaatttg 2400
atgaaagagt taaacatccc gctcaccctc aaggacgccg gcatcaacaa ggagcagttc 2460
gagaaggaga tcgaggagat gagcgacatc gccttcaacg accagtgcac cggcaccaac 2520
ccgcgcatgc cgctcaccaa agagatagct gagatttatc gcaaggccta cggcgcctga 2580

<210> 197
<211> 78
<212> DNA
<213> Thermoanaerobacterium saccharolyticum
<400> 197
atgttggctt ctcgtcagct cctgggcctc gcccggagcc gcgctgtcgg cagcgcgagc 60
cttggtctcc ggcgcatg 78

<210> 198
<211> 26
<212> PRT
<213> Thermoanaerobacterium saccharolyticum
<400> 198
MET Leu Ala Ser Arg Gln Leu Leu Gly Leu Ala Arg Ser Arg Ala Val
1               5                   10                  15
Gly Ser Ala Ser Leu Gly Leu Arg Arg MET
            20                  25
<210> 199
<211> 27
<212> DNA
<213> Primer
<400> 199
gaaggcgtgt tggtgttgta gatgtcg 27

<210> 200
<211> 78
<212> DNA
<213> Thermoanaerobacterium saccharolyticum
<400> 200
atgttcctgg gcagccgcat ccagacccgc gccttctcgg cgtcggcccg aaacctctcc 60
aaggtcgccg ttctcggt 78

ã\200\200ã\200\200ã\200\200